# EUROPEAN PATENT APPLICATION

(11) **EP 4 672 949 A1**
(43) Date of publication of application: **31.12.2025**
(21) Application number: 25185321.4
(22) Date of filing: 25.06.2025
(51) Int. Cl.: H10K 85/60, H10K 50/11

(54) **LIGHT-EMITTING DEVICE INCLUDING HETEROCYCLIC COMPOUND, ELECTRONIC APPARATUS AND ELECTRONIC DEVICE INCLUDING THE LIGHT-EMITTING DEVICE, AND THE HETEROCYCLIC COMPOUND**

(30) Priority: 26.06.2024 KR 20240083969; 24.06.2025 KR 20250083852
(71) Applicant: Samsung Display Co., Ltd., Yongin-si, Gyeonggi-do 17113 (KR)
(72) Inventor: KIM, Hyojeong, 17113 Yongin-si (KR); UM, Hyunah, 17113 Yongin-si (KR); LEE, Hyunwoo, 17113 Yongin-si (KR); CHO, Seowon, 17113 Yongin-si (KR)
(74) Representative: Marks & Clerk LLP

(57) **Abstract**

Embodiments provide a heterocyclic compound, a light-emitting device including the heterocyclic compound, an electronic apparatus including the light-emitting device, and an electronic device including the light-emitting device. The light-emitting device includes a first electrode (110), a second electrode (150) facing the first electrode, an interlayer (130) between the first electrode and the second electrode and including an emission layer, and the heterocyclic compound. The heterocyclic compound is represented by Formula 1, which is explained in the specification:

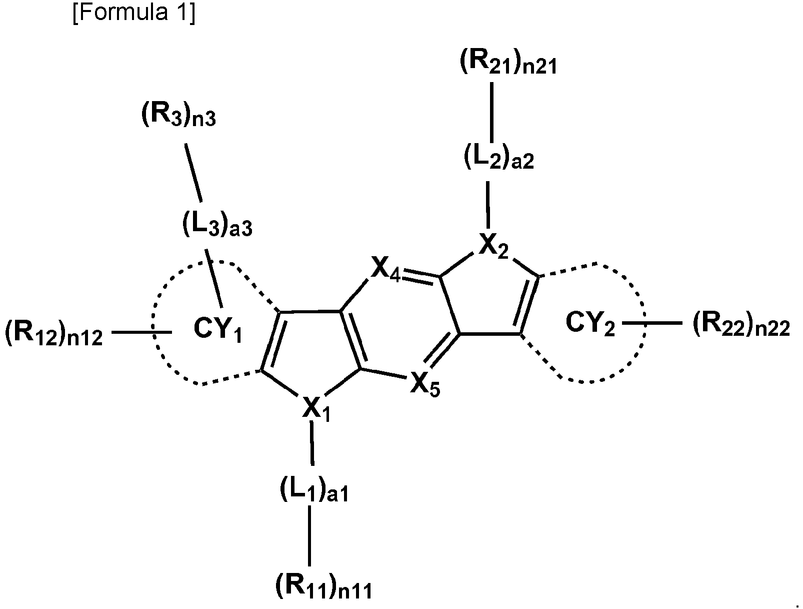

## Description

### BACKGROUND

### 1. Technical Field

Embodiments relate to a light-emitting device including a heterocyclic compound, an electronic apparatus including the light-emitting device, an electronic device including the light-emitting device, and the heterocyclic compound.

### 2. Description of the Related Art

Light-emitting devices are self-emissive devices that have wide viewing angles, high contrast ratios, short response times, and excellent characteristics in terms of luminance, driving voltage, and response speed.

In a light-emitting device, a first electrode is arranged on a substrate, and a hole transport region, an emission layer, an electron transport region, and a second electrode are sequentially arranged on the first electrode. Holes provided from the first electrode move toward the emission layer through the hole transport region, and electrons provided from the second electrode move toward the emission layer through the electron transport region. Carriers, such as holes and electrons, recombine in the emission layer to produce excitons. The excitons may transition from an excited state to a ground state, thereby generating light.

It is to be understood that this background of the technology section is, in part, intended to provide useful background for understanding the technology. However, this background of the technology section may also include ideas, concepts, or recognitions that were not part of what was known or appreciated by those skilled in the pertinent art prior to a corresponding effective filing date of the subject matter disclosed herein.

### SUMMARY

Embodiments include a light-emitting device, an electronic apparatus including the light-emitting device, an electronic device including the light-emitting device, and the heterocyclic compound.

Additional aspects will be set forth in part in the description which follows and, in part, will be apparent from the description, or may be learned by practice of the embodiments of the disclosure.

According to embodiments, a light-emitting device includes
a first electrode,
a second electrode facing the first electrode,
an interlayer between the first electrode and the second electrode and including an emission layer, and
a heterocyclic compound represented by Formula 1:

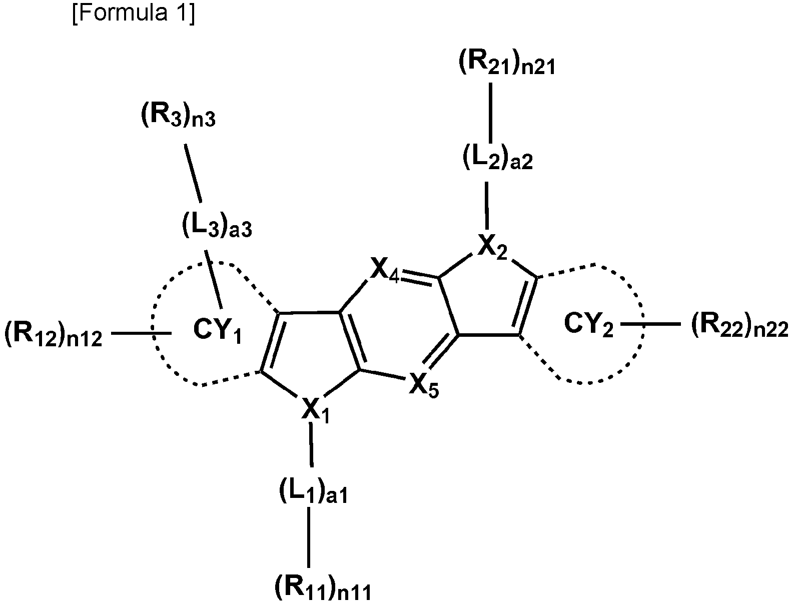

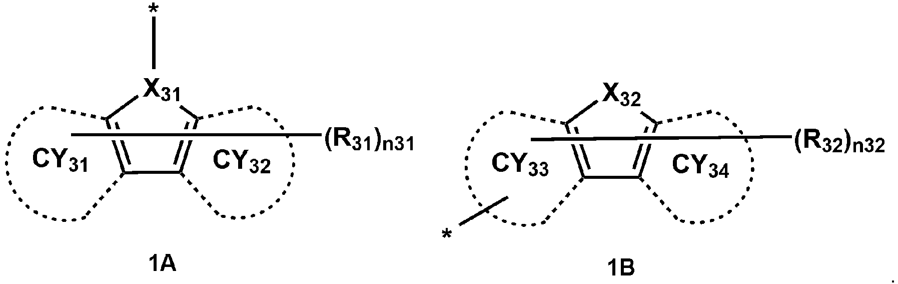

In Formulae 1, 1A, and 1B,
X₁ is C(R₁) or N,
X₂ is C(R₂) or N,
X₃₁ is C(R₃ₐ) or N,
X₃₂ is N(R₃ₐ) or C(R₃ₐ)(R_{3b}),
X₄ is C(R₄) or N,
X₅ is C(R₅) or N,
L₁ to L₃ are each independently a single bond, a C₅-C₆₀ carbocyclic group unsubstituted or substituted with at least one R₁₀ₐ, or a C₁-C₆₀ heterocyclic group unsubstituted or substituted with at least one R₁₀ₐ,
a1 to a3 are each independently an integer from 1 to 3,
ring CY₁, ring CY₂, and ring CY₃₁ to ring CY₃₄ are each independently a C₅-C₆₀ carbocyclic group or a C₁-C₆₀ heterocyclic group,
R₁, R₂, R₃ₐ, R_{3b}, R₄, R₅, R₁₁, R₁₂, R₂₁, R₂₂, R₃, R₃₁, and R₃₂ are each independently a group represented by Formula 1A, a group represented by Formula 1B, hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, a C₆-C₆₀ arylseleno group unsubstituted or substituted with at least one R₁₀ₐ, a C₇-C₆₀ aryl alkyl group unsubstituted or substituted with at least one R₁₀ₐ, a C₂-C₆₀ heteroaryl alkyl group unsubstituted or substituted with at least one R₁₀ₐ, - Si(Q₁)(Q₂)(Q₃), -Ge(Q₁)(Q₂)(Q₃), -N(Q₁)(Q₂), -B(Q₁)(Q₂), -C(=O)(Q₁), -S(=O)₂(Q₁), or - P(=O)(Q₁)(Q₂),
at least one of R₃ in the number of n3 is a group represented by Formula 1A or Formula 1B,
n11 is an integer from 0 to 20,
n12 is an integer from 0 to 30,
n21 is an integer from 0 to 20,
n22 is an integer from 0 to 30,
n3 is an integer from 0 to 30,
n31 is an integer from 0 to 20,
n32 is an integer from 0 to 20,
* indicates a binding site to a neighboring atom,
R₁₀ₐ is:
   deuterium, -F, -Cl, -Br, -I, a hydroxyl group, a cyano group, or a nitro group;
   a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
   a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
   -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
   Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, -F, a cyano group, a C₁-C₆₀ alkyl group, a C₁-C₆₀ alkoxy group, a phenyl group, a biphenyl group, a C₃-C₆₀ carbocyclic group, a C₁-C₆₀ heterocyclic group, or any combination thereof.

In an embodiment, the emission layer may include: the heterocyclic compound; and a third compound that includes a transition metal, a fourth compound that includes a cyclic group including boron (B) and nitrogen (N) as ring-forming atoms, or any combination thereof, and
the heterocyclic compound, the third compound, and the fourth compound may be different from each other.

In an embodiment, the emission layer may include: the heterocyclic compound; and a second compound that includes at least one π electron-deficient nitrogen-containing C₁-C₆₀ heterocyclic group, and
the second compound may be different from the heterocyclic compound.

In an embodiment, the emission layer may include a host and a dopant, and the host may include the heterocyclic compound.

In an embodiment, the emission layer may emit blue light.

According to embodiments, an electronic apparatus includes the light-emitting device.

In an embodiment, the electronic apparatus may further include a thin-film transistor, wherein the thin-film transistor may include a source electrode and a drain electrode, and the first electrode of the light-emitting device may be electrically connected to at least one of the source electrode and the drain electrode.

In an embodiment, the electronic apparatus may further include a color filter, a color conversion layer, a touch screen layer, a polarizing layer, or any combination thereof.

In an embodiment, the color conversion layer may include quantum dots.

According to embodiments, an electronic device includes the light-emitting device.

According to embodiments, a heterocyclic compound is represented by Formula 1, which is explained herein.

In an embodiment, ring CY₁, ring CY₂, and ring CY₃₁ to ring CY₃₄ may each independently be a benzene group, a naphthalene group, an anthracene group, a phenanthrene group, a triphenylene group, a pyrene group, a chrysene group, a cyclopentadiene group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an azafluorene group, an azacarbazole group, an azadibenzofuran group, an azadibenzothiophene group, an azadibenzosilole group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a dibenzoxasiline group, a dibenzothiasiline group, a dibenzodihydroazasiline group, a dibenzodihydrodisiline group, a dibenzodihydrosiline group, a dibenzodioxine group, a dibenzoxathiine group, a dibenzoxazine group, a dibenzopyran group, a dibenzodithiine group, a dibenzothiazine group, a dibenzothiopyran group, a dibenzocyclohexadiene group, a dibenzodihydropyridine group, or a dibenzodihydropyrazine group.

In an embodiment, ring CY₁, and ring CY₃₁ to ring CY₃₄ may each be a benzene group, and ring CY₂ may be a furan group or a thiophene group.

In an embodiment, L₁ to L₃ may each independently be: a single bond; or a benzene group, a naphthalene group, a phenanthrene group, a fluorene group, a carbazole group, a dibenzofuran group, a dibenzothiophene group, an azacarbazole group, an azadibenzofuran group, or an azadibenzothiophene group, each unsubstituted or substituted with at least one R₁₀ₐ.

In an embodiment, L₁ to L₃ may each independently be a single bond, or a group represented by one of Formula 2-1 to Formula 2-5, which are explained below.

In an embodiment, R₁, R₂, R₃ₐ, R_{3b}, R₄, R₅, R₁₁, R₁₂, R₂₁, R₂₂, R₃, R₃₁, and R₃₂ may each independently be:

a group represented by Formula 1A, a group represented by Formula 1B, hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a phenyl group, a biphenyl group, or a terphenyl group;

a C₁-C₆₀ alkyl group, a C₃-C₆₀ cycloalkyl group, a C₁-C₆₀ heterocycloalkyl group, a phenyl group, a biphenyl group, a terphenyl group, a naphthyl group, an anthracenyl group, a phenanthrenyl group, a phenalenyl group, a triphenylenyl group, a pyrenyl group, a chrysenyl group, a pyrrolyl group, a furanyl group, a thiophenyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a phenanthrolinyl group, a benzoimidazolyl group, a benzofuranyl group, a dibenzofuranyl group, a benzothiophenyl group, a dibenzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a carbazolyl group, a benzocarbazolyl group, a fluorenyl group, a benzoisothiazolyl group, a benzoxazolyl group, a benzoisoxazolyl group, a phenoxazinyl group, an acridinyl group, or a xanthenyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a phenyl group, a biphenyl group, a terphenyl group, or a combination thereof; or
-Si(Q₁)(Q₂)(Q₃), -Ge(Q₁)(Q₂)(Q₃), or -N(Q₁)(Q₂).

In an embodiment, the heterocyclic compound may include:
i) at least one carbazole group; or
ii) at least one deuterium; or
iii) at least one -Si(Q₁)(Q₂)(Q₃); or
iv) at least one cyano group; or
v) any combination selected from i) to iv).

In an embodiment, the group represented by Formula 1A may be represented by one of Formulae 1A-1 to 1A-6, which are explained below; and the group represented by Formula 1B may be represented by one of Formulae 1B-1 to 1B-12, which are explained below.

In an embodiment, the heterocyclic compound may be represented by one of Formula 1-1 to Formula 1-4, which are explained below.

In an embodiment, the heterocyclic compound may be one of Compounds 1 to 21, which are explained below.

At least some of the above and other features of the invention are set out in the claims.

It is to be understood that the embodiments above are described in a generic and explanatory sense only and not for the purposes of limitation, and the disclosure is not limited to the embodiments described above.

### BRIEF DESCRIPTION OF THE DRAWINGS

The accompanying drawings are included to provide a further understanding of the embodiments, and are incorporated in and constitute a part of this specification. The drawings illustrate embodiments of the disclosure and principles thereof. The above and other aspects and features of the disclosure will become more apparent by describing in detail embodiments thereof with reference to the accompanying drawings, in which:
FIG. 1 is a schematic cross-sectional view of a light-emitting device according to an embodiment;
FIG. 2 is a schematic cross-sectional view of an electronic apparatus according to an embodiment;
FIG. 3 is a schematic cross-sectional view of an electronic apparatus according to another embodiment;
FIG. 4 is a schematic perspective view of an electronic device including a light-emitting device according to an embodiment;
FIG. 5 is a schematic perspective view of an exterior of a vehicle according to an embodiment; and
FIGS. 6A to 6C are each a schematic diagram of an interior of a vehicle according to embodiments.

### DETAILED DESCRIPTION OF THE EMBODIMENTS

The disclosure will now be described more fully hereinafter with reference to the accompanying drawings, in which embodiments are shown. This disclosure may, however, be embodied in different forms and should not be construed as limited to the embodiments set forth herein. Rather, these embodiments are provided so that this disclosure will be thorough and complete, and will fully convey the scope of the disclosure to those skilled in the art.

In the drawings, the sizes, thicknesses, ratios, and dimensions of the elements may be exaggerated for ease of description and for clarity. Like reference numbers and reference characters refer to like elements throughout.

In the specification, it will be understood that when an element (or region, layer, part, etc.) is referred to as being "on", "connected to", or "coupled to" another element, it can be directly on, connected to, or coupled to the other element, or one or more intervening elements may be present therebetween. In a similar sense, when an element (or region, layer, part, etc.) is described as "covering" another element, it can directly cover the other element, or one or more intervening elements may be present therebetween.

In the specification, when an element is "directly on", "directly connected to", or "directly coupled to" another element, there are no intervening elements present. For example, "directly on" may mean that two layers or two elements are disposed without an additional element such as an adhesion element therebetween.

In the specification, the expressions used in the singular such as "a", "an", and "the" are intended to include the plural forms as well, unless the context clearly indicates otherwise.

In the specification, the term "and/or" includes any and all combinations of one or more of the associated listed items. For example, "A and/or B" may be understood to mean "A, B, or A and B". The terms "and" and "or" may be used in the conjunctive or disjunctive sense and may be understood to be equivalent to "and/or".

In the specification and the claims, the term "at least one of" is intended to include the meaning of "at least one selected from the group consisting of" for the purpose of its meaning and interpretation. For example, "at least one of A, B, and C" may be understood to mean A only, B only, C only, or any combination of two or more of A, B, and C, such as ABC, ACC, BC, or CC. When preceding a list of elements, the term, "at least one of", modifies the entire list of elements and does not modify the individual elements of the list.

It will be understood that, although the terms first, second, etc. may be used herein to describe various elements, these elements should not be limited by these terms. These terms are only used to distinguish one element from another element. Thus, a first element could be termed a second element without departing from the teachings of the disclosure. Similarly, a second element could be termed a first element, without departing from the scope of the disclosure.

The spatially relative terms "below", "beneath", "lower", "above", "upper", or the like, may be used herein for ease of description to describe the relations between one element or component and another element or component as illustrated in the drawings. It will be understood that the spatially relative terms are intended to encompass different orientations of the device in use or operation, in addition to the orientation depicted in the drawings. For example, in the case where a device illustrated in the drawing is turned over, the device positioned "below" or "beneath" another device may be placed "above" another device. Accordingly, the illustrative term "below" may include both the lower and upper positions. The device may also be oriented in other directions and thus the spatially relative terms may be interpreted differently depending on the orientations.

The terms "about" or "approximately" as used herein is inclusive of the stated value and means within an acceptable range of deviation for the recited value as determined by one of ordinary skill in the art, considering the measurement in question and the error associated with measurement of the recited quantity (i.e., the limitations of the measurement system). For example, "about" may mean within one or more standard deviations, or within ±20%, ±10%, or ±5% of the stated value.

It should be understood that the terms "comprises", "comprising", "includes", "including", "have", "having", "contains", "containing", and the like are intended to specify the presence of stated features, integers, steps, operations, elements, components, or combinations thereof in the disclosure, but do not preclude the presence or addition of one or more other features, integers, steps, operations, elements, components, or combinations thereof.

In the specification, the term "interlayer" may refer to a single layer and/or multiple layers between a first electrode and a second electrode of a light-emitting device.

Unless otherwise defined or implied herein, all terms (including technical and scientific terms) used have the same meaning as commonly understood by those skilled in the art to which this disclosure pertains. It will be further understood that terms, such as those defined in commonly used dictionaries, should be interpreted as having a meaning that is consistent with their meaning in the context of the relevant art and should not be interpreted in an ideal or excessively formal sense unless clearly defined in the specification.

According to embodiments, a light-emitting device includes: a first electrode; a second electrode facing the first electrode; an interlayer between the first electrode and the second electrode and including an emission layer; and a heterocyclic compound represented by Formula 1:

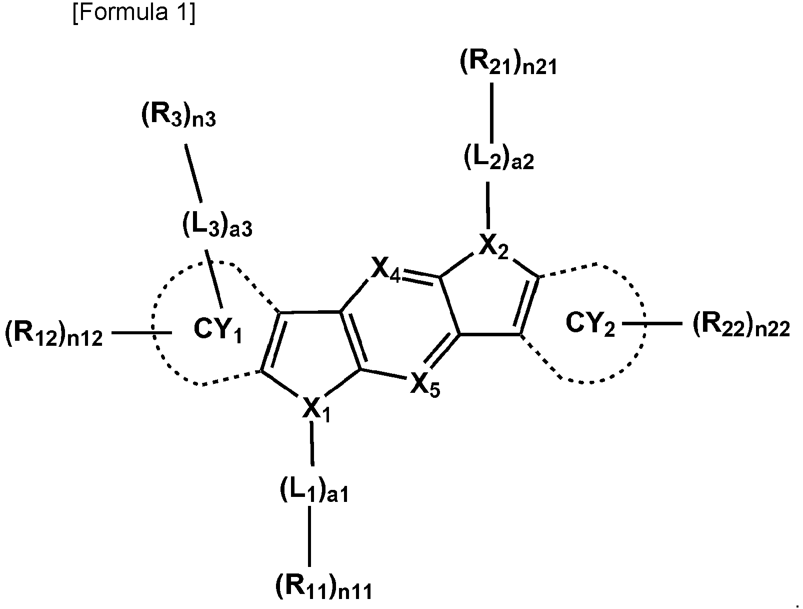

A complete description of Formula 1 will be provided below.

According to an embodiment,
the first electrode may be an anode,
the second electrode may be a cathode,
the interlayer may further include a hole transport region between the first electrode and the emission layer, and an electron transport region between the emission layer and the second electrode,
the hole transport region may include at least one of a hole injection layer, a hole transport layer, a buffer layer, an emission auxiliary layer, and an electron-blocking layer, and
the electron transport region may include at least one of a hole blocking layer, an electron transport layer, and an electron injection layer.

According to an embodiment, the heterocyclic compound may be included between the first electrode and the second electrode of the light-emitting device. For example, the interlayer may include the heterocyclic compound represented by Formula 1. For example, the emission layer may include the heterocyclic compound represented by Formula 1.

According to an embodiment, the emission layer may include a dopant and a host, and the host may include the heterocyclic compound represented by Formula 1. For example, the heterocyclic compound may serve as a host. The emission layer may emit red light, green light, blue light, and/or white light. In an embodiment, the emission layer may emit blue light. The blue light may have a maximum emission wavelength in a range of, for example, about 400 nm to about 490 nm. For example, the blue light may have a wavelength in a range of about 430 nm to about 480 nm.

According to an embodiment, the light-emitting device may include a capping layer located outside the first electrode or outside the second electrode.

In an embodiment, the light-emitting device may further include at least one of a first capping layer located outside the first electrode and a second capping layer located outside the second electrode, and at least one of the first capping layer and the second capping layer may include the heterocyclic compound represented by Formula 1. Further details on the first capping layer and the second capping layer may be the same as described herein.

In the specification, the expression "(an interlayer and/or a capping layer) includes at least one heterocyclic compound" may include a case in which "(an interlayer and/or a capping layer) includes identical heterocyclic compounds represented by Formula 1" and may include a case in which "(an interlayer and/or a capping layer) includes two or more different heterocyclic compounds, each independently represented by Formula 1."

For example, the interlayer and/or capping layer may include Compound 1 only as the heterocyclic compound. In this regard, Compound 1 may be present in an emission layer of the light-emitting device. According to an embodiment, the interlayer may include, as the heterocyclic compound, Compound 1 and Compound 2. In this regard, Compound 1 and Compound 2 may be present in a same layer (for example, both Compound 1 and Compound 2 may be present in the emission layer), or may be present in different layers (for example, Compound 1 may be present in the emission layer, and Compound 2 may be present in the electron transport region).

According to an embodiment,
the emission layer in the light-emitting device may include:
a first compound that includes the heterocyclic compound represented by Formula 1; and
a second compound that includes at least one π electron-deficient nitrogen-containing C₁-C₆₀ cyclic group, a third compound that includes a transition metal, a fourth compound that includes a cyclic group including boron (B) and nitrogen (N) as ring-forming atoms, or any combination thereof, and
the first compound, the second compound, the third compound, and the fourth compound may be different from each other.
In an embodiment, the emission layer may include: the first compound; and at least one of the second compound, the third compound, and the fourth compound. In an embodiment, the emission layer may include the first compound and the second compound. The first compound and the second compound may be different from each other.

In an embodiment, the emission layer may include the first compound and the fourth compound.

In an embodiment, the emission layer may include the first compound and the third compound.

In an embodiment, the emission layer may include the first compound and the second compound.

In an embodiment, the emission layer may include the first compound, the second compound, and the fourth compound. In an embodiment, the emission layer may include the first compound, third compound and the fourth compound. The first compound, third compound and the fourth compound may be different from each other.

In an embodiment, the emission layer may include the first compound, the third compound, and the fourth compound.

In an embodiment, the emission layer may include the first compound, the second compound, and the third compound.

In an embodiment, the emission layer may include the first compound, the second compound, the third compound, and the fourth compound.

In an embodiment, when the emission layer includes at least one of the first compound, the second compound, the third compound, and the fourth compound, on a basis of 100% (wt%) of the total amount of the emission layer,
a combined amount of the first compound and the second compound (a sum of an amount of the first compound and an amount of the second compound) may be in a range of about 50% to about 99%, about 55% to about 98%, or about 60% to about 97%,
an amount of the third compound may be in a range of about 10% to about 30%, about 11% to about 28%, about 12% to about 26%, or about 13% to about 24%, and
an amount of the fourth compound may be in a range of about 0.1% to about 5%, about 0.5% to about 4%, or about 1% to about 3%.

In an embodiment, the second compound may include a compound represented by Formula 20:

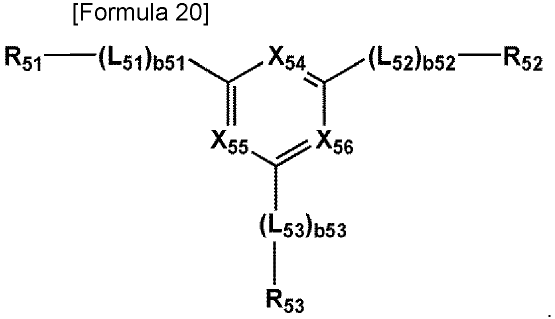

In Formula 20,
L₅₁ to L₅₃ may each independently be a single bond, a C₃-C₆₀ carbocyclic group unsubstituted or substituted with at least one R₁₀ₐ, or a C₁-C₆₀ heterocyclic group unsubstituted or substituted with at least one R₁₀ₐ,
b51 to b53 may each independently be an integer from 1 to 5,
X₅₄ may be N or C(R₅₄), X₅₅ may be N or C(R₅₅), X₅₆ may be N or C(R₅₆), and at least one of X₅₄ to X₅₆ may each be N,
R₅₁ to R₅₆ are respectively the same as described in the specification, and
R₁₀ₐ and Q₁ to Q₃ are respectively the same as described herein.

In an embodiment, the third compound may include a compound represented by Formula 31:

[Formula 31] M(L₄₀₁)_{xc1}(L402)xc2

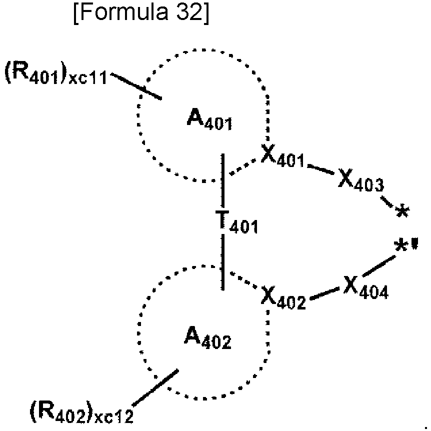

In Formulae 31 and 32,
M may be a transition metal (for example, iridium (Ir), platinum (Pt), palladium (Pd), osmium (Os), titanium (Ti), gold (Au), hafnium (Hf), europium (Eu), terbium (Tb), rhodium (Rh), rhenium (Re), or thulium (Tm)),
L₄₀₁ may be a ligand represented by Formula 32, and xc1 may be 1, 2, or 3, wherein when xc1 is two or more, two or more of L₄₀₁ may be identical to or different from each other,
L₄₀₂ may be an organic ligand, and xc2 may be 0, 1, 2, 3, or 4, wherein when xc2 is 2 or more, two or more of L₄₀₂ may be identical to or different from each other,
X₄₀₁ and X₄₀₂ may each independently be nitrogen or carbon,
ring A₄₀₁ and ring A₄₀₂ may each independently a C₃-C₆₀ carbocyclic group or a C₁-C₆₀ heterocyclic group,
T₄₀₁ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*',
X₄₀₃ and X₄₀₄ may each independently be a chemical bond, O, S, N(Q₄₁₃), B(Q₄₁₃), P(Q₄₁₃), C(Q₄₁₃)(Q₄₁₄), or Si(Q₄₁₃)(Q₄₁₄),
R₄₀₁ and R₄₀₂ may each independently be hydrogen, deuterium, -F, -Cl, -Br, -I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), or -P(=O)(Q₄₀₁)(Q₄₀₂),
xc11 and xc12 may each independently be an integer from 0 to 10,
Q₄₁₁ to Q₄₁₄ and Q₄₀₁ to Q₄₀₃ may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, -F, a cyano group, a C₁-C₆₀ alkyl group, a C₁-C₆₀ alkoxy group, a phenyl group, a biphenyl group, or any combination thereof,
* and *' in Formula 32 each indicate a binding site to M in Formula 31, and
R₁₀ₐ may be the same as described herein.

In an embodiment, the fourth compound may be a C₈-C₆₀ polycyclic group-containing compound that includes two or more cyclic groups that are condensed while sharing a boron (B) atom.

In an embodiment, the fourth compound may include a condensed ring in which at least one third ring is condensed with at least one fourth ring, wherein

the third ring may be a cyclopentane group, a cyclohexane group, a cycloheptane group, a cyclooctane group, a cyclopentene group, a cyclohexene group, a cycloheptene group, a cyclooctene group, an adamantane group, a norbornene group, a norbornane group, a bicyclo[1.1.1]pentane group, a bicyclo[2.1.1]hexane group, a bicyclo[2.2.2]octane group, a benzene group, a pyridine group, a pyrimidine group, a pyridazine group, a pyrazine group, or a triazine group, and

the fourth ring may be a 1,2-azaborinine group, a 1,3-azaborinine group, a 1,4-azaborinine group, a 1,2-dihydro-1,2-azaborinine group, a 1,4-oxaborinine group, a 1,4-thiaborinine group, or a 1,4-dihydroborinine group.

In an embodiment, the fourth compound may include a compound represented by Formula 42, a compound represented by Formula 43, or any combination thereof:

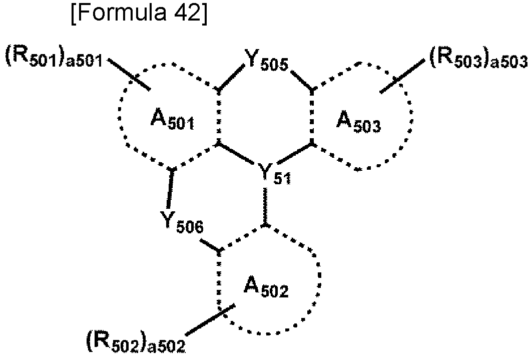

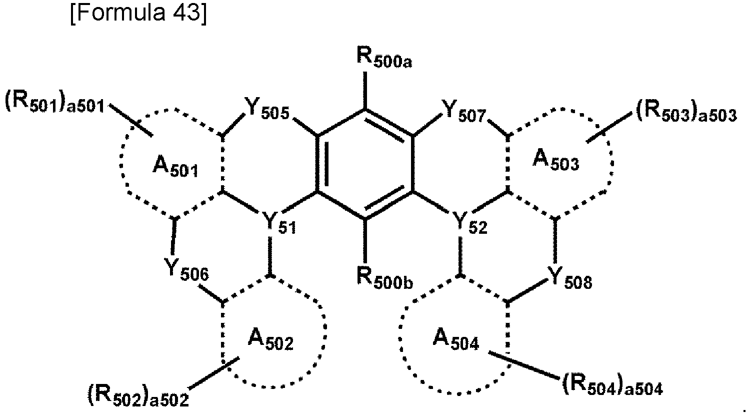

In Formulae 42 and 43,
ring A₅₀₁ to ring A₅₀₄ may each independently be a C₃-C₆₀ carbocyclic group or a C₁-C₆₀ heterocyclic group,
Y₅₀₅ may be O, S, N(R₅₀₅), B(R₅₀₅), C(R₅₀₅ₐ)(R_{505b}), or Si(R₅₀₅ₐ)(R_{505b}),
Y₅₀₆ may be O, S, N(R₅₀₆), B(R₅₀₆), C(R₅₀₆ₐ)(R_{506b}), or Si(R₅₀₆ₐ)(R_{506b}),
Y₅₀₇ may be O, S, N(R₅₀₇), B(R₅₀₇), C(R₅₀₇ₐ)(R_{507b}), or Si(R₅₀₇ₐ)(R_{507b}),
Y₅₀₈ may be O, S, N(R₅₀₈), B(R₅₀₈), C(R₅₀₈ₐ)(R_{508b}), or Si(R₅₀₈ₐ)(R_{508b}),
Y₅₁ and Y₅₂ may each independently be B, P(=O), or S(=O),
R₅₀₀ₐ, R_{500b}, R₅₀₁ to R₅₀₈, R₅₀₅ₐ, R_{505b}, R₅₀₆ₐ, R_{506b}, R₅₀₇ₐ, R_{507b}, R₅₀₈ₐ, and R_{508b} may respectively be the same as described herein, and
a501 to a504 may each independently be an integer from 0 to 20.

### [Description of Formulae 20, 31, 32, 42, and 43]

In Formula 20, b51 to b53 respectively indicate numbers of L₅₁ to L₅₃, and b51 to b53 may each independently be an integer from 1 to 5. In Formula 20, when b51 is 2 or more, two or more of L₅₁ may be identical to or different from each other, when b52 is 2 or more, two or more of L₅₂ may be identical to or different from each other, and when b53 is 2 or more, two or more of L₅₃ may be identical to or different from each other. In an embodiment, b51 to b53 may each independently be 1 or 2.

In an embodiment, in Formula 20, L₅₁ to L₅₃ may each independently be:
a single bond; or
a benzene group, a naphthalene group, an anthracene group, a phenanthrene group, a triphenylene group, a pyrene group, a chrysene group, a cyclopentadiene group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an azafluorene group, an azacarbazole group, an azadibenzofuran group, an azadibenzothiophene group, an azadibenzosilole group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a dibenzoxasiline group, a dibenzothiasiline group, a dibenzodihydroazasiline group, a dibenzodihydrodisiline group, a dibenzodihydrosiline group, a dibenzodioxane group, a dibenzoxathiene group, a dibenzoxazine group, a dibenzopyran group, a dibenzodithiine group, a dibenzothiazine group, a dibenzothiopyran group, a dibenzocyclohexadiene group, a dibenzodihydropyridine group, or a dibenzodihydropyrazine group, each unsubstituted or substituted with deuterium, -F, -Cl, -Br, -I, a hydroxyl group, a cyano group, a nitro group, a C₁-C₂₀ alkyl group, a C₁-C₂₀ alkoxy group, a phenyl group, a naphthyl group, a pyridinyl group, a pyrimidinyl group, a triazinyl group, a fluorenyl group, a dimethylfluorenyl group, a diphenylfluorenyl group, a carbazolyl group, a phenylcarbazolyl group, a dibenzofuranyl group, a dibenzothiophenyl group, a dibenzosilolyl group, a dimethyldibenzosilolyl group, a diphenyldibenzosilolyl group, -O(Q₃₁), -S(Q₃₁), - Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), -P(=O)(Q₃₁)(Q₃₂), or any combination thereof, and
Q₃₁ to Q₃₃ may each independently be hydrogen, deuterium, a C₁-C₂₀ alkyl group, a C₁-C₂₀ alkoxy group, a phenyl group, a biphenyl group, a terphenyl group, a pyridinyl group, a pyrimidinyl group, a pyridazinyl group, a pyrazinyl group, or a triazinyl group.

In an embodiment, in Formula 20, a bond between L₅₁ and R₅₁, a bond between L₅₂ and R₅₂, a bond between L₅₃ and R₅₃, a bond between two or more of L₅₁, a bond between two or more of L₅₂, a bond between two or more of L₅₃, a bond between L₅₁ and carbon between X₅₄ and X₅₅ in Formula 20, a bond between L₅₂ and carbon between X₅₄ and X₅₆ in Formula 20, and a bond between L₅₃ and carbon between X₅₅ and X₅₆ in Formula 20 may each be a "carbon-carbon single bond".

In Formula 20, X₅₄ may be N or C(R₅₄), X₅₅ may be N or C(R₅₅), X₅₆ may be N or C(R₅₆), and at least one of X₅₄ to X₅₆ may each be N. R₅₄ to R₅₆ may respectively be the same as described herein. According to an embodiment, two or three of X₅₄ to X₅₆ may each be N.

In the specification, R₅₁ to R₅₆ in Formula 20, and R₅₀₀ₐ, R_{500b}, R₅₀₁ to R₅₀₈, R₅₀₅ₐ, R_{505b}, R₅₀₆ₐ, R_{506b}, R₅₀₇ₐ, R_{507b}, R₅₀₈ₐ, and R_{508b} in Formulae 42 and 43 may each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -C(Q₁)(Q₂)(Q₃), -Si(Q₁)(Q₂)(Q₃), -N(Q₁)(Q₂), - B(Q₁)(Q₂), -C(=O)(Q₁), -S(=O)₂(Q₁), or -P(=O)(Q₁)(Q₂). Q₁ to Q₃ may respectively be the same as described herein.

In embodiments, R₅₁ to R₅₆ in Formula 20, R₄₀₁ and R₄₀₂ in Formulae 31 and 32, R₅₀₀ₐ, R_{500b}, R₅₀₁ to R₅₀₈, R₅₀₅ₐ, R_{505b}, R₅₀₆ₐ, R_{506b}, R₅₀₇ₐ, R_{507b}, R₅₀₈ₐ and R_{508b} in Formulae 42 and 43, and R₁₀ₐ may each independently be:
hydrogen, deuterium, -F, -Cl, -Br, -I, a hydroxyl group, a cyano group, a nitro group, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, an azadibenzosilolyl group, or a group represented by Formula 91, each unsubstituted or substituted with deuterium, -F, -Cl, -Br, -I, -CD₃, - CD₂H, -CDH₂, -CF₃, -CF₂H, -CFH₂, a hydroxyl group, a cyano group, a nitro group, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, -O(Q₃₁), -S(Q₃₁), -Si(Q₃₁)(Q₃₂)(Q₃₃), -N(Q₃₁)(Q₃₂), -B(Q₃₁)(Q₃₂), -P(Q₃₁)(Q₃₂), - C(=O)(Q₃₁), -S(=O)₂(Q₃₁), -P(=O)(Q₃₁)(Q₃₂), or any combination thereof; or
-C(Q₁)(Q₂)(Q₃), -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂), and
Q₁ to Q₃ and Q₃₁ to Q₃₃ may each independently be:
-CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂; or
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, a pyridinyl group, a pyrimidinyl group, a pyridazinyl group, a pyrazinyl group, or a triazinyl group, each unsubstituted or substituted with deuterium, a C₁-C₁₀ alkyl group, a phenyl group, a biphenyl group, a pyridinyl group, a pyrimidinyl group, a pyridazinyl group, a pyrazinyl group, a triazinyl group, or any combination thereof.

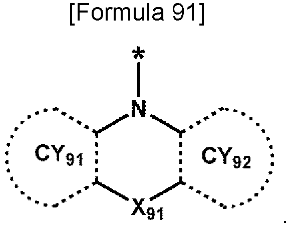

In Formula 91,
ring CY₉₁ and ring CY₉₂ may each independently be a C₅-C₃₀ carbocyclic group unsubstituted or substituted with at least one R₁₀ₐ or a C₁-C₃₀ heterocyclic group unsubstituted or substituted with at least one R₁₀ₐ,
X₉₁ may be a single bond, O, S, N(R₉₁), B(R₉₁), C(R₉₁ₐ)(R_{91b}), or Si(R₉₁ₐ)(R_{91b}),
R₉₁, R₉₁ₐ and R_{91b} may each independently be the same as described in connection with R₅₁,
R₁₀ₐ may be the same as described herein, and
* indicates a binding site to a neighboring atom.

In an embodiment, in Formula 91,
ring CY₉₁ and ring CY₉₂ may each independently be a benzene group, a pyridine group, a pyrimidine group, a pyrazine group, a pyridazine group, or a triazine group, each unsubstituted or substituted with at least one R₁₀ₐ, and
R₉₁, R₉₁ₐ, and R_{91b} may each independently be:
hydrogen or a C₁-C₁₀ alkyl group; or
a phenyl group, a pyridinyl group, a pyrimidinyl group, a pyridazinyl group, a pyrazinyl group, or a triazinyl group, each unsubstituted or substituted with deuterium, a C₁-C₁₀ alkyl group, a phenyl group, a biphenyl group, a pyridinyl group, a pyrimidinyl group, a pyridazinyl group, a pyrazinyl group, a triazinyl group, or any combination thereof.

In an embodiment, R₅₁ to R₅₆ in Formula 20, R₄₀₁ and R₄₀₂ in Formulae 31 and 32, R₅₀₀ₐ, R_{500b}, R₅₀₁ to R₅₀₈, R₅₀₅ₐ, R_{505b}, R₅₀₆ₐ, R_{506b}, R₅₀₇ₐ, R_{507b}, R₅₀₈ₐ and R_{508b} in Formulae 42 and 43, and R₁₀ₐ may each independently be hydrogen, deuterium, -F, a cyano group, a nitro group, -CH₃, -CD₃, -CD₂H, -CDH₂, -CF₃, -CF₂H, -CFH₂, a group represented by one of Formulae 9-1 to 9-67, a group represented by one of Formulae 10-1 to 10-154 and 10-201 to 10-368, -C(Q₁)(Q₂)(Q₃), -Si(Q₁)(Q₂)(Q₃), or - P(=O)(Q₁)(Q₂), wherein Q₁ to Q₃ may respectively be the same as described herein:

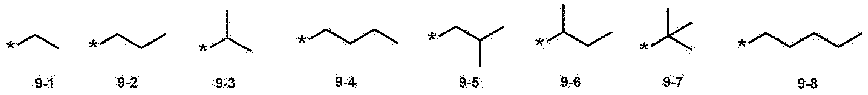

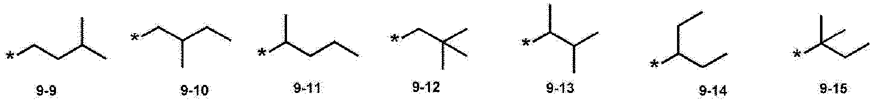

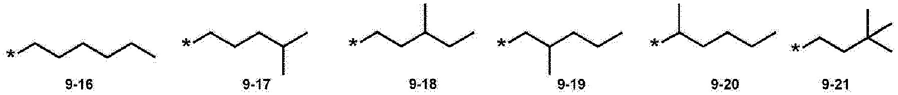

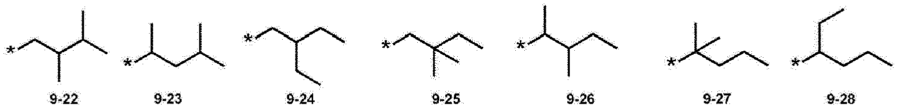

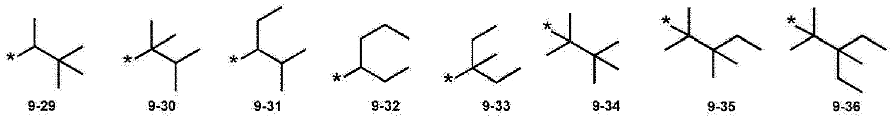

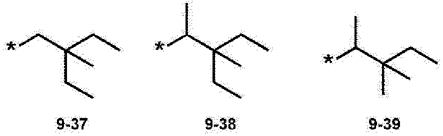

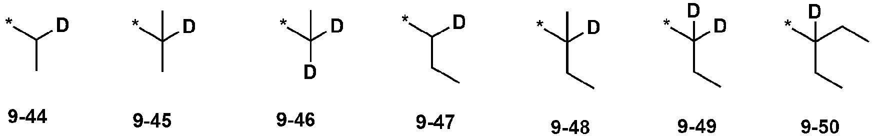

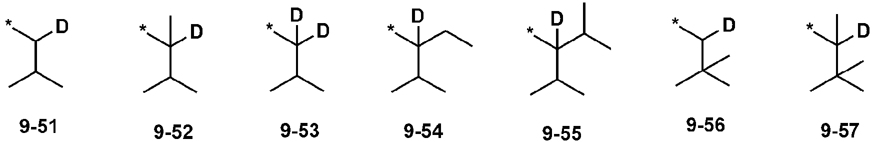

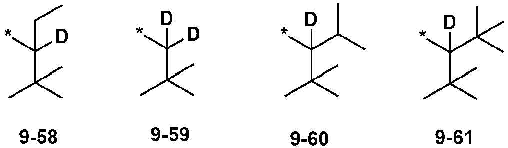

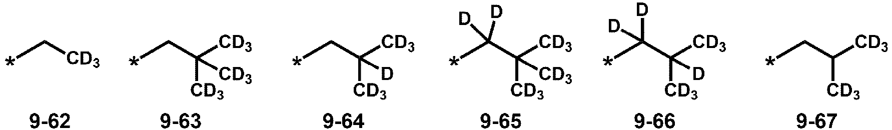

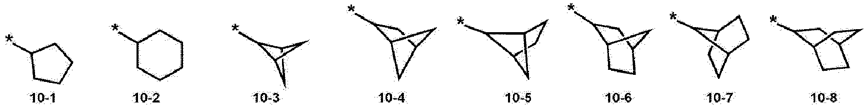

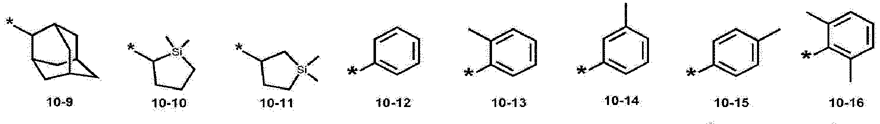

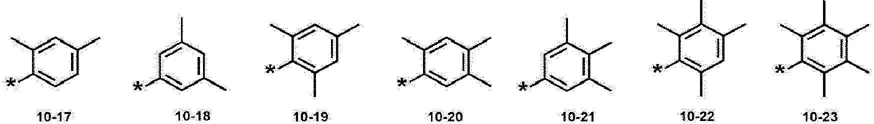

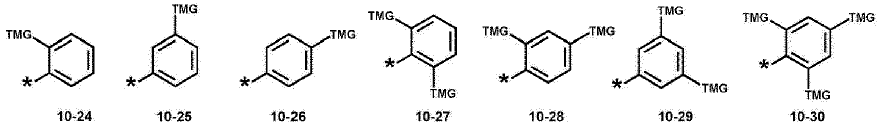

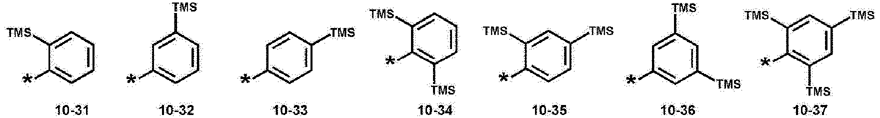

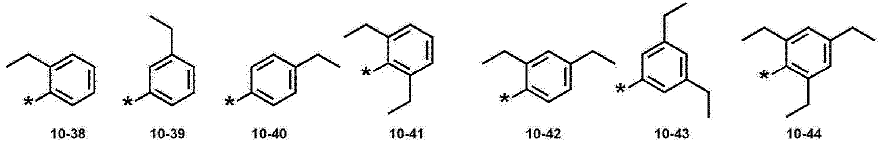

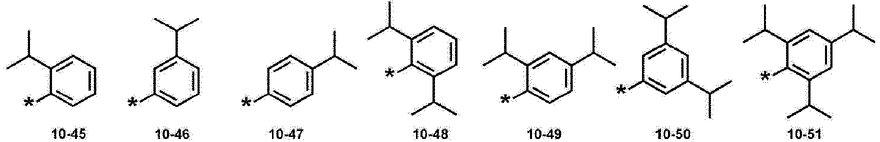

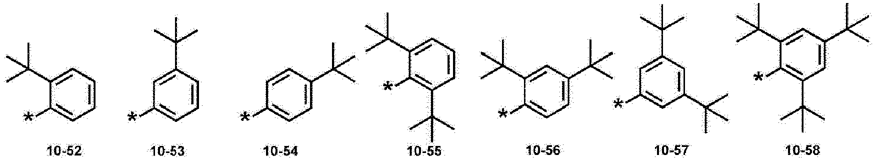

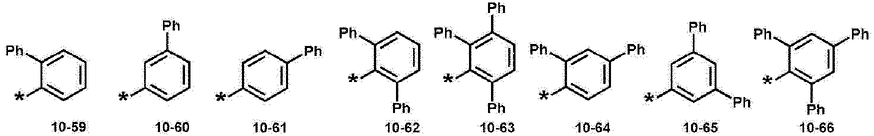

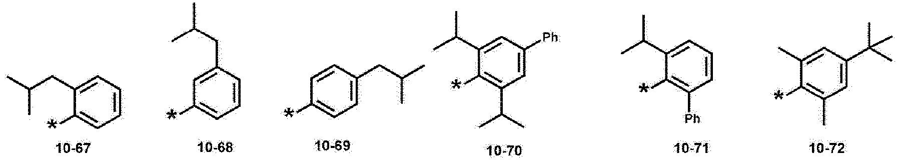

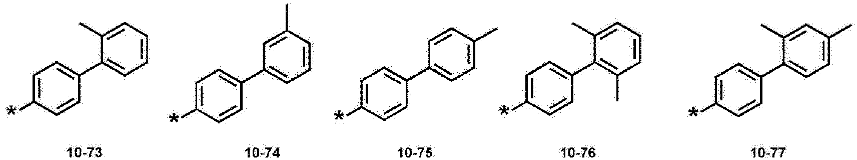

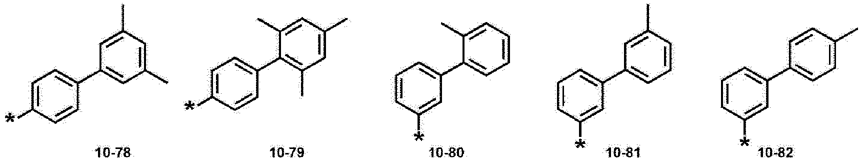

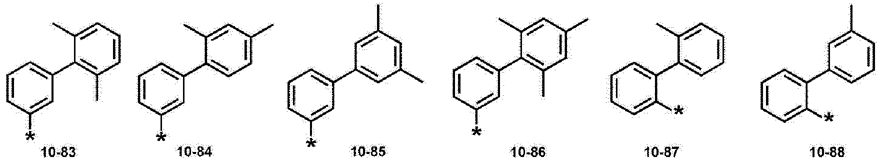

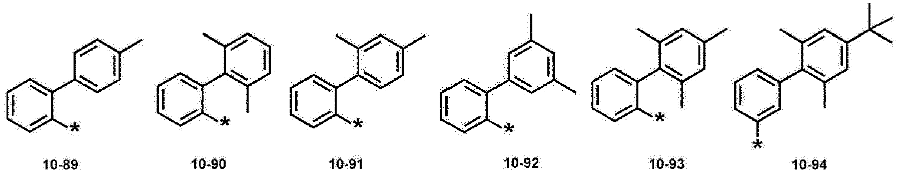

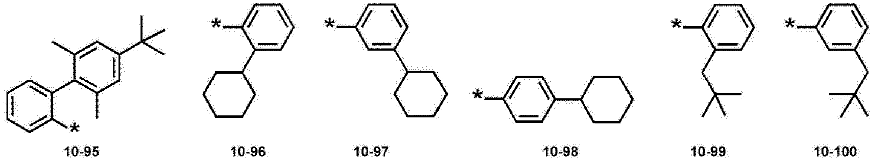

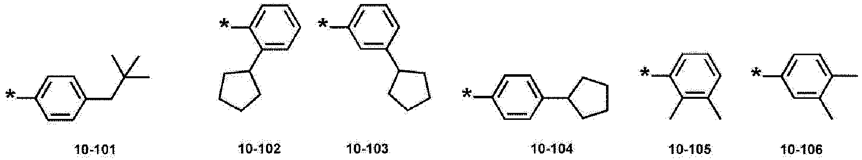

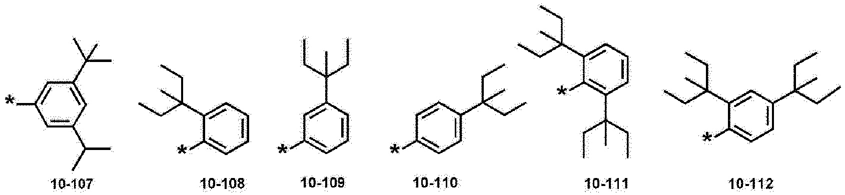

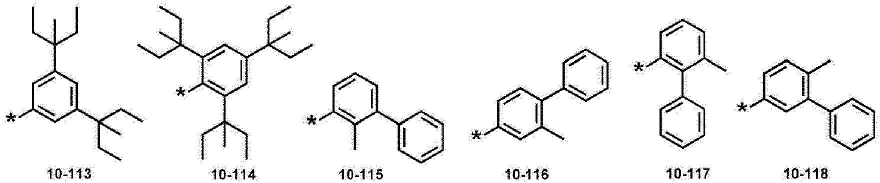

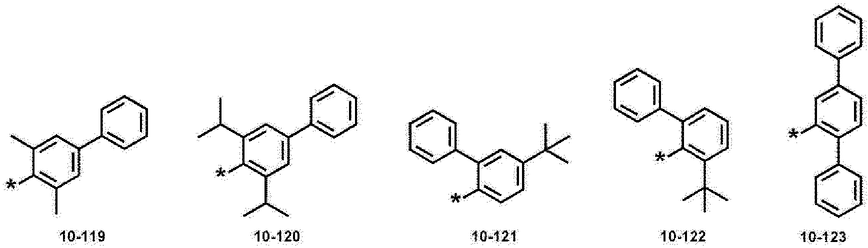

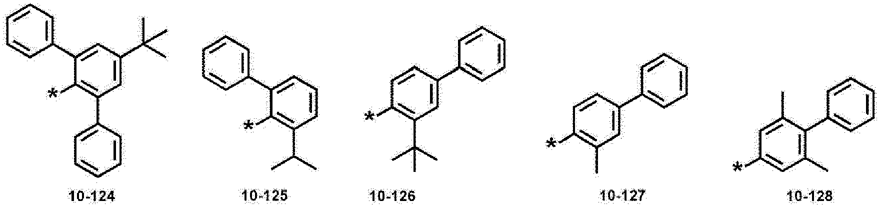

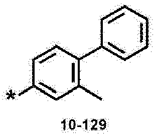

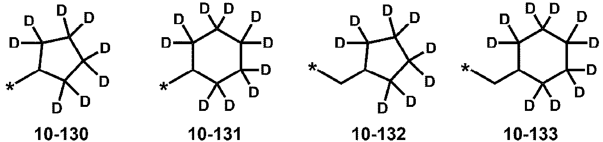

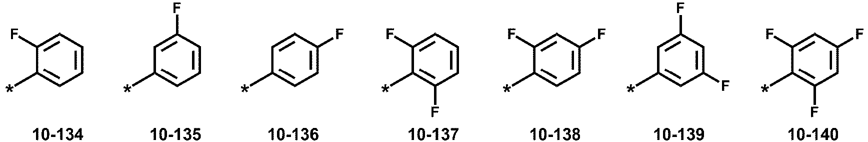

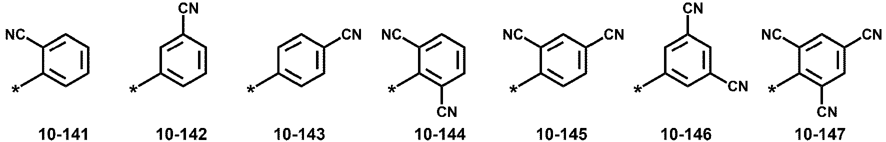

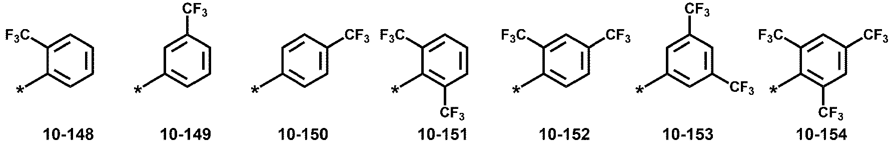

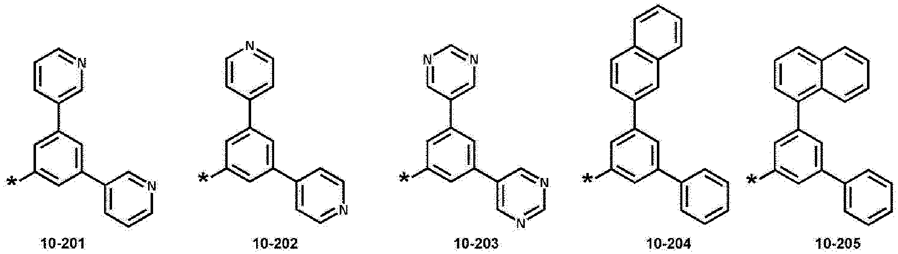

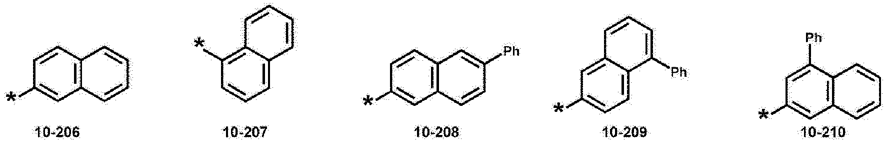

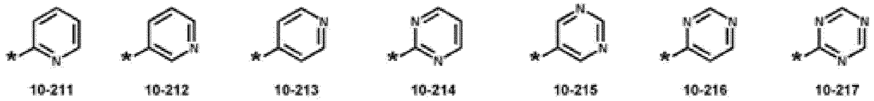

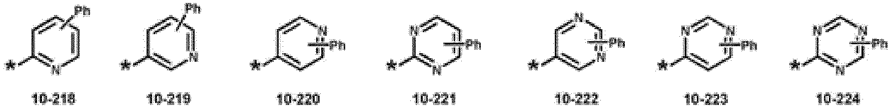

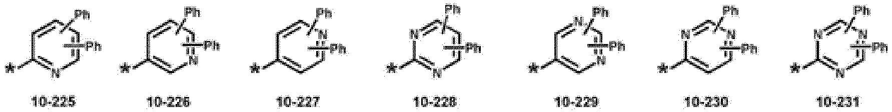

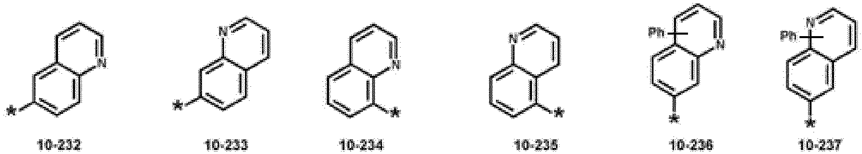

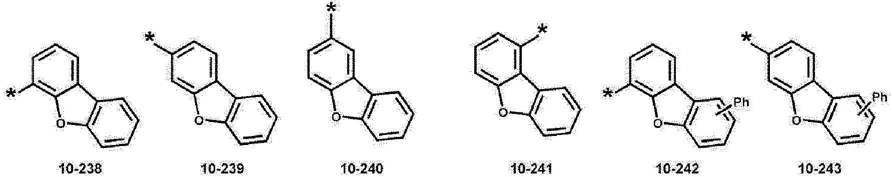

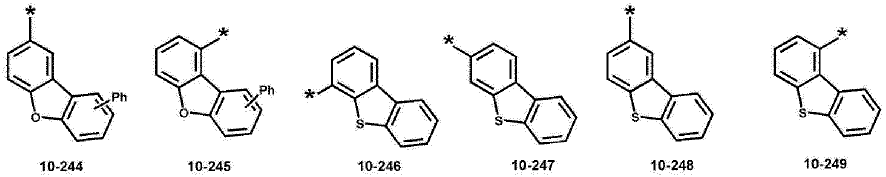

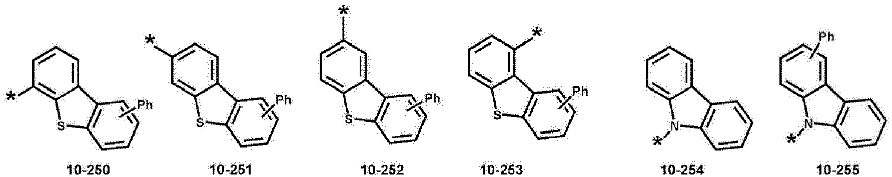

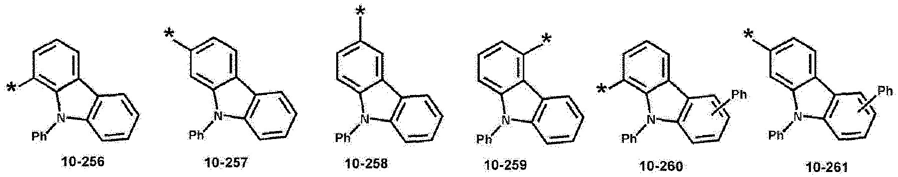

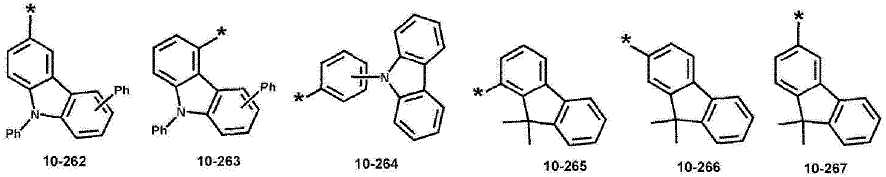

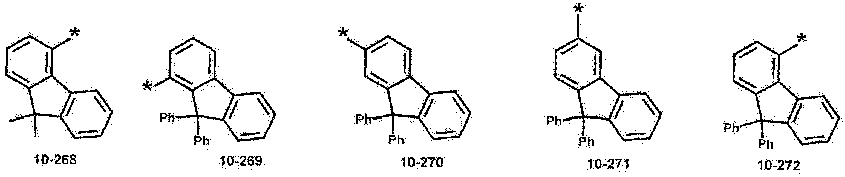

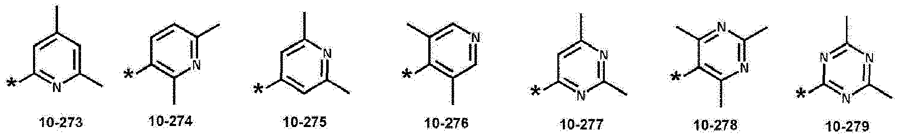

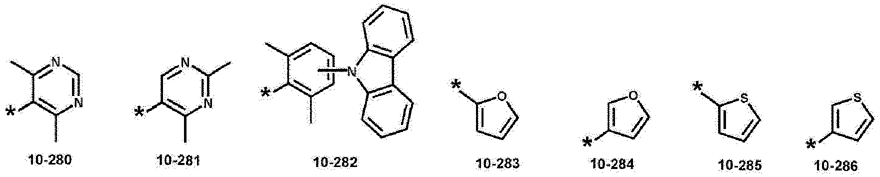

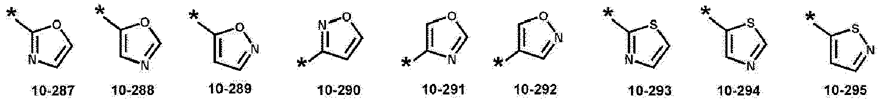

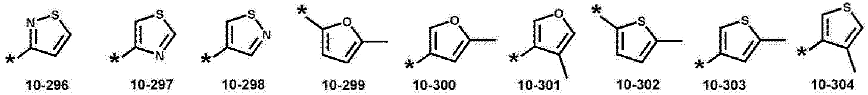

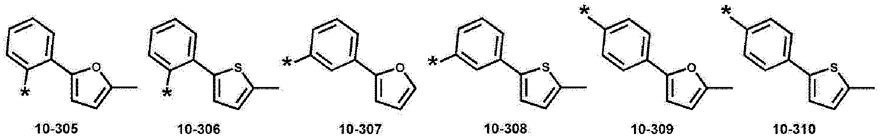

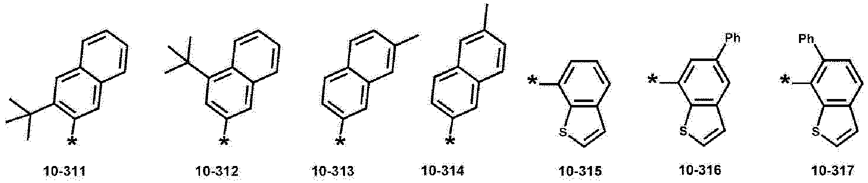

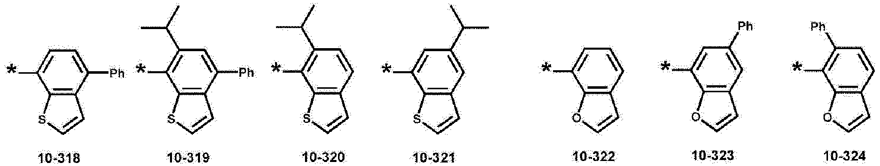

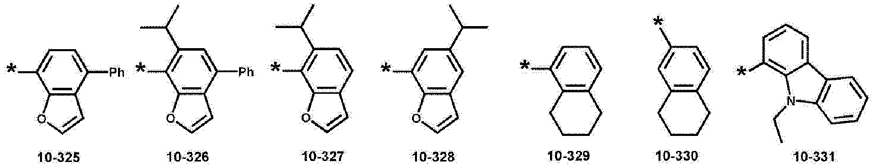

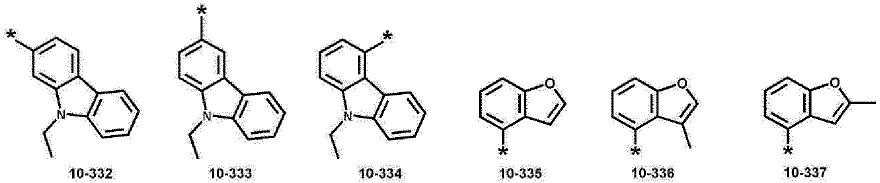

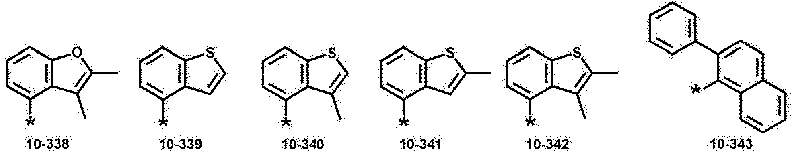

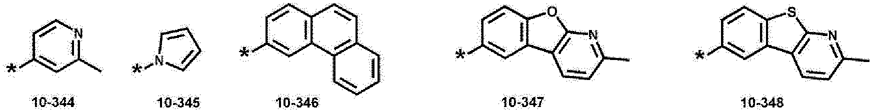

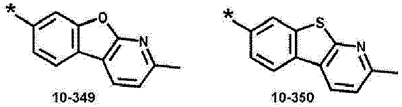

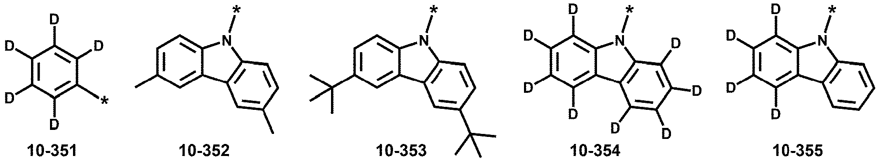

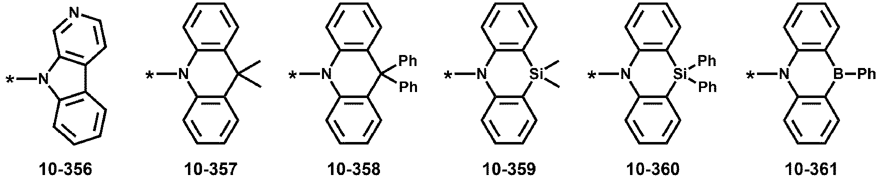

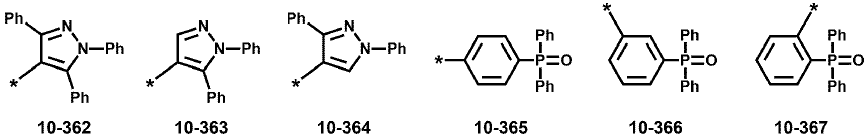

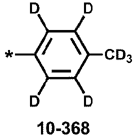

In Formulae 9-1 to 9-61, 10-1 to 10-154, and 10-201 to 10-368, * indicates a binding site to an adjacent atom, D represents a deuterium atom, Ph represents a phenyl group, TMS represents a trimethylsilyl group, and TMG represents a trimethylgermyl group.

In an embodiment, in Formula 20, a group represented by *-(L₅₁)_{b51}-R₅₁ and a group represented by *-(L₅₂)_{b52}-R₅₂ may each not be a phenyl group.

In an embodiment, in Formula 20, a group represented by *-(L₅₁)_{b51}-R₅₁ and a group represented by *-(L₅₂)_{b52}-R₅₂ may be identical to each other.

In an embodiment, in Formula 20, a group represented by *-(L₅₁)_{b51}-R₅₁ and a group represented by *-(L₅₂)_{b52}-R₅₂ in Formula 2 may be different from each other.

In an embodiment, in Formula 20, b51 and b52 may each be 1, 2, or 3; and L₅₁ and L₅₂ may each independently be a benzene group, a pyridine group, a pyrimidine group, a pyridazine group, a pyrazine group, or a triazine group, each unsubstituted or substituted with at least one R₁₀ₐ.

In an embodiment, in Formula 20, R₅₁ and R₅₂ may each independently be a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -C(Q₁)(Q₂)(Q₃), or -Si(Q₁)(Q₂)(Q₃), and

Q₁ to Q₃ may each independently be a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

In an embodiment, in Formula 20,
a group represented by *-(L₅₁)_{b51}-R₅₁ may be a group represented by one of Formulae CY51-1 to CY51-26, and/or
a group represented by *-(L₅₂)_{b52}-R₅₂ may be a group represented by one of Formulae CY52-1 to CY52-26, and/or
a group represented by *-(L₅₃)_{b53}-R₅₃ may be a group represented by one of Formulae CY53-1 to CY53-27, -C(Q₁)(Q₂)(Q₃), or -Si(Q₁)(Q₂)(Q₃):

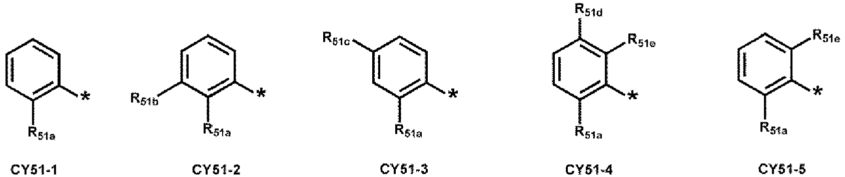

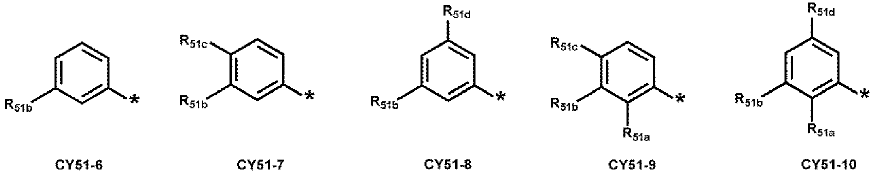

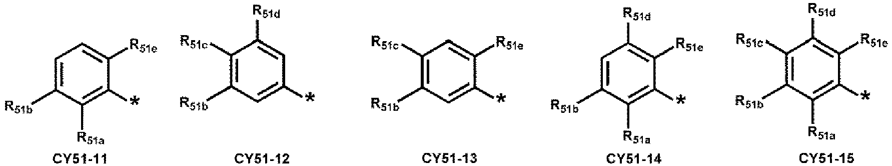

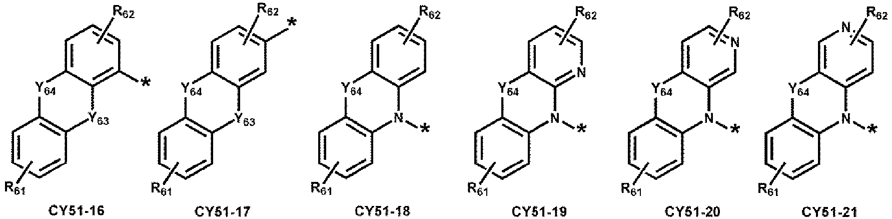

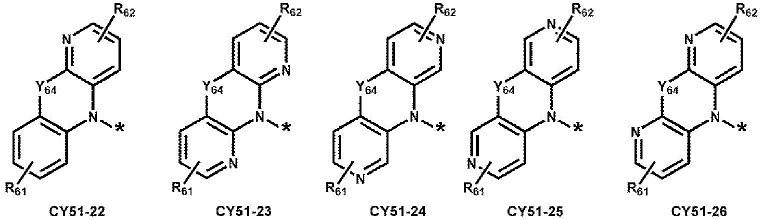

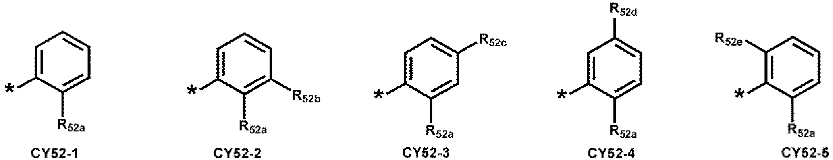

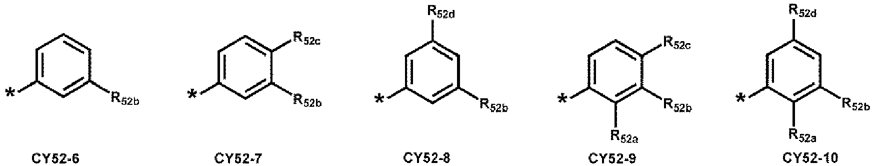

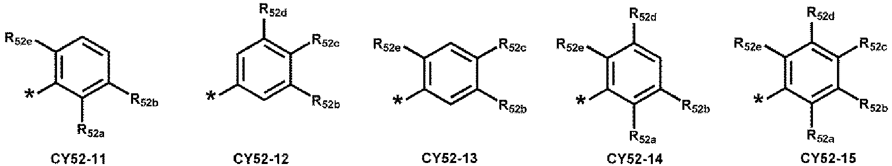

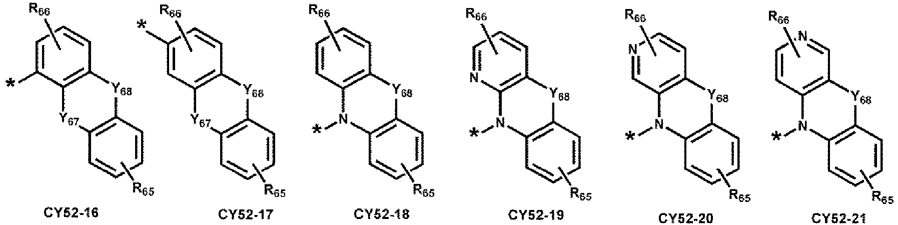

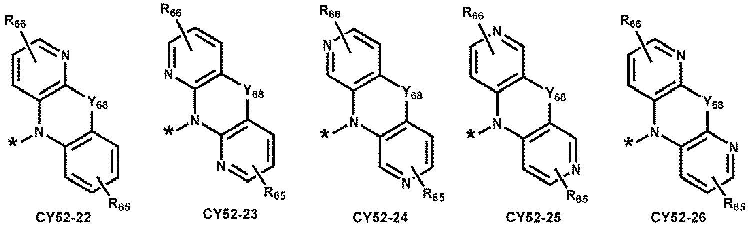

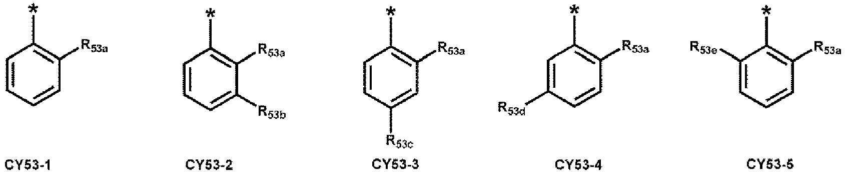

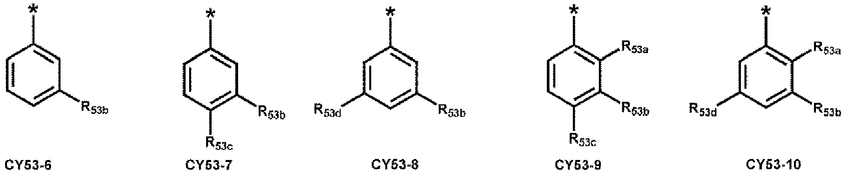

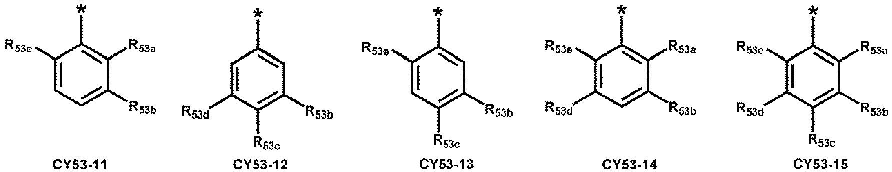

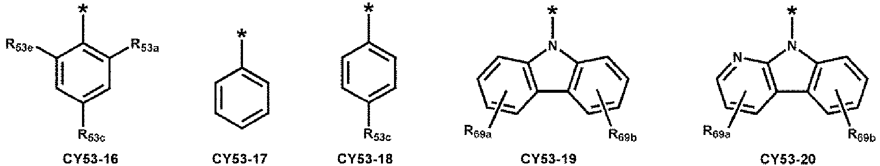

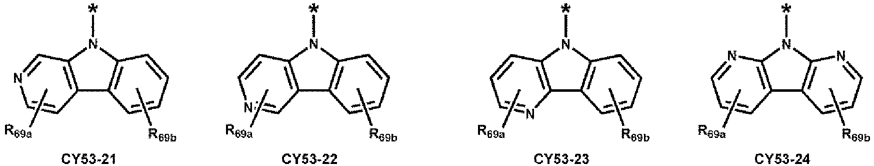

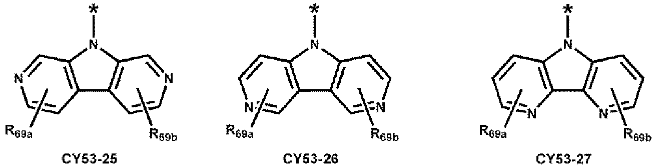

In Formulae CY51-1 to CY51-26, CY52-1 to CY52-26, and CY53-1 to CY53-27,
Y₆₃ may be a single bond, O, S, N(R₆₃), B(R₆₃), C(R₆₃ₐ)(R_{63b}), or Si(R₆₃ₐ)(R_{63b}),
Y₆₄ may be a single bond, O, S, N(R₆₄), B(R₆₄), C(R₆₄ₐ)(R_{64b}), or Si(R₆₄ₐ)(R_{64b}),
Y₆₇ may be a single bond, O, S, N(R₆₇), B(R₆₇), C(R₆₇ₐ)(R_{67b}), or Si(R₆₇ₐ)(R_{67b}),
Y₆₈ may be a single bond, O, S, N(R₆₈), B(R₆₈), C(R₆₈ₐ)(R_{68b}), or Si(R₆₈ₐ)(R_{68b}),
Y₆₃ and Y₆₄ in Formulae CY51-16 and CY51-17 may each not be a single bond at the same time,
Y₆₇ and Y₆₈ in Formulae CY52-16 and CY52-17 may each not be a single bond at the same time,
R₅₁ₐ to R₅₁ₑ, R₆₁ to R₆₄, R₆₃ₐ, R_{63b}, R₆₄ₐ, and R_{64b} may each independently be the same as described in connection with R₅₁, except that R₅₁ₐ to R₅₁ₑ may each not be hydrogen,
R₅₂ₐ to R₅₂ₑ, R₆₅ to R₆₈, R₆₇ₐ, R_{67b}, R₆₈ₐ, and R_{68b} may each independently be the same as described in connection with R₅₂, except that R₅₂ₐ to R₅₂ₑ may each not be hydrogen,
R₅₃ₐ to R₅₃ₑ, R₆₉ₐ, and R_{69b} may each independently be the same as described in connection with R₅₃, except that R₅₃ₐ to R₅₃ₑ may each not be hydrogen, and
* indicates a binding site to a neighboring atom.

In an embodiment, in Formulae CY51-1 to CY51-26 and CY52-1 to 52-26, R₅₁ₐ to R₅₁ₑ and R₅₂ₐ to R₅₂ₑ may each independently be:
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, an azafluorenyl group, an azadibenzosilolyl group, or a group represented by Formula 91, each unsubstituted or substituted with deuterium, -F, -Cl, -Br, -I, -CD₃, - CD₂H, -CDH₂, -CF₃, -CF₂H, -CFH₂, a hydroxyl group, a cyano group, a nitro group, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a C₁-C₁₀ alkylphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof; or
-C(Q₁)(Q₂)(Q₃) or -Si(Q₁)(Q₂)(Q₃),
Q₁ to Q₃ may each independently be a phenyl group, a naphthyl group, a pyridinyl group, a pyrimidinyl group, a pyridazinyl group, a pyrazinyl group, or a triazinyl group, each unsubstituted or substituted with deuterium, a C₁-C₁₀ alkyl group, a phenyl group, a biphenyl group, a pyridinyl group, a pyrimidinyl group, a pyridazinyl group, a pyrazinyl group, a triazinyl group, or any combination thereof,
in Formulae CY51-16 and CY51-17, Y₆₃ may be O or S, and Y₆₄ may be Si(R₆₄ₐ)(R_{64b}); or ii) Y₆₃ may be Si(R₆₃ₐ)(R_{63b}), and Y₆₄ may be O or S, and
in Formulae CY52-16 and CY52-17, Y₆₇ may be O or S, and Y₆₈ may be Si(R₆₈ₐ)(R_{68b}); or Y₆₇ may be Si(R₆₇ₐ)(R_{67b}), and Y₆₈ may be O or S.
In an embodiment, in Formula 32, X₄₀₁ may be nitrogen and X₄₀₂ may be carbon, or X₄₀₁ and X₄₀₂ may each be nitrogen.

In an embodiment, in Formula 31, when xc1 is 2 or more, two ring A₄₀₁(s) in two or more of L₄₀₁ may be optionally linked to each other via T₄₀₂, which is a linking group, or two ring A₄₀₂(s) may be optionally linked to each other via T₄₀₃, which is a linking group (see Compounds PD1 to PD4 and PD7). T₄₀₂ and T₄₀₃ may each independently be the same as described in connection with T₄₀₁.

In Formula 31, L₄₀₂ may be an organic ligand. In an embodiment, in Formula 31, L₄₀₂ may include a halogen group, a diketone group (for example, an acetylacetonate group), a carboxylic acid group (for example, a picolinate group), - C(=O), an isonitrile group, a -CN group, a phosphorus group (for example, a phosphine group, a phosphite group, etc.), or any combination thereof.

### [Examples of second compound, third compound, and fourth compound]

In an embodiment, the second compound may include at least one of Compounds ETH1 to ETH96:

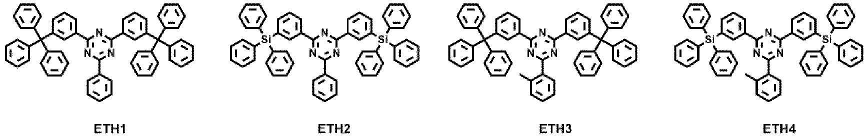

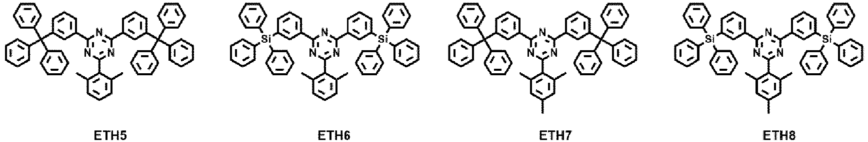

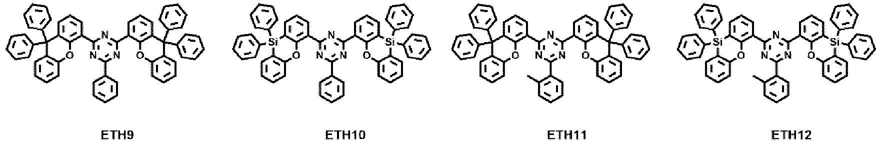

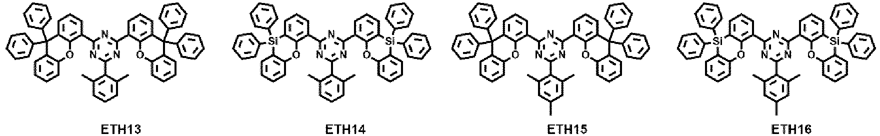

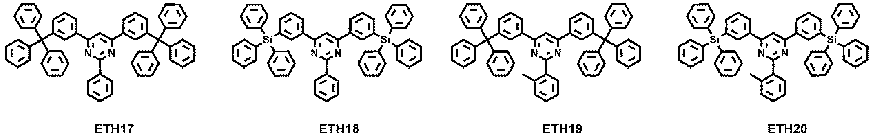

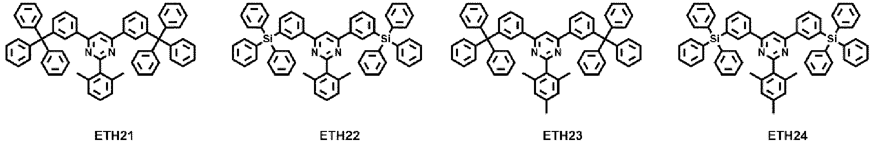

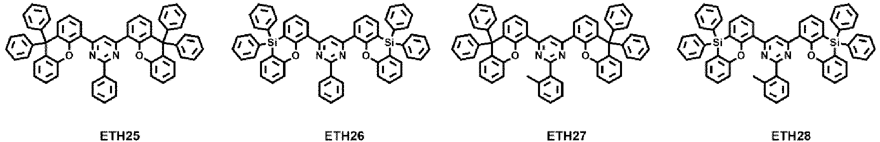

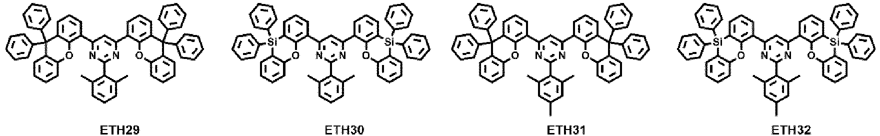

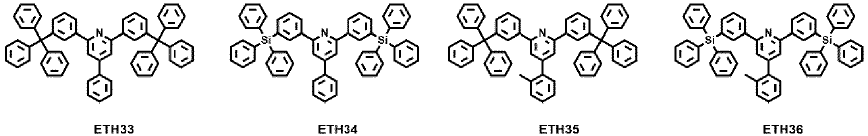

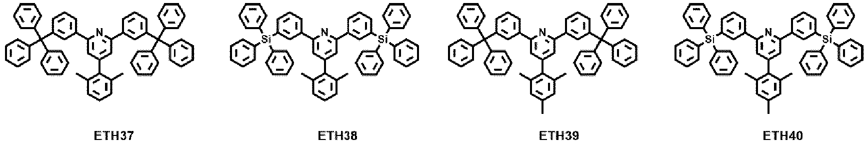

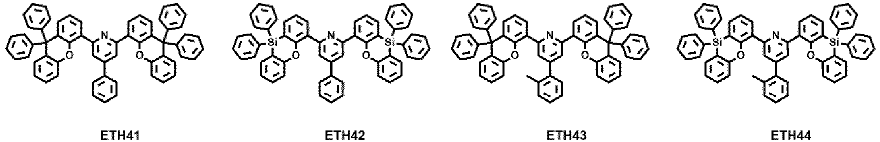

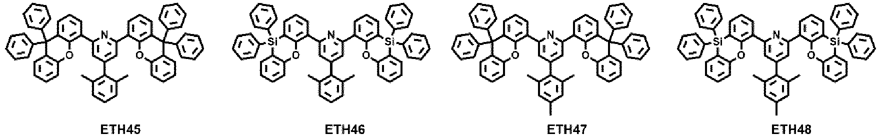

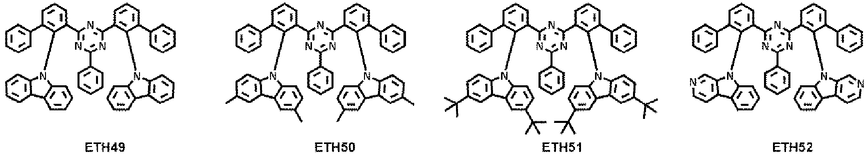

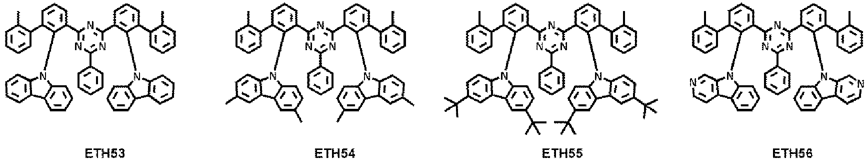

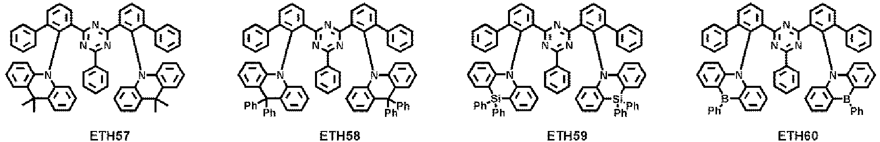

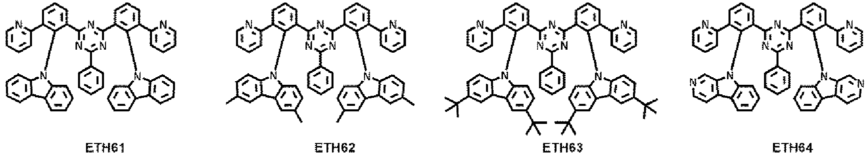

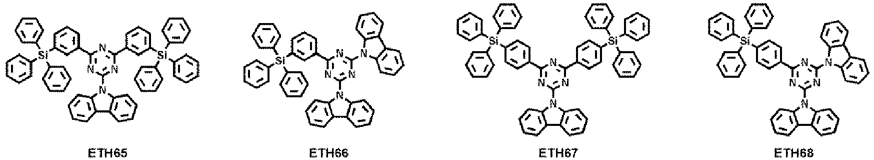

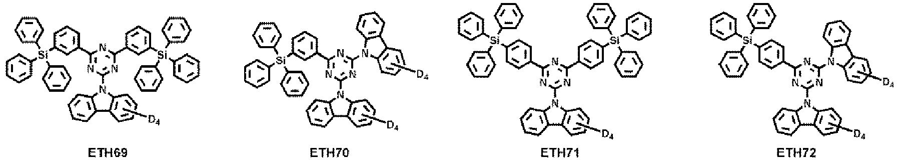

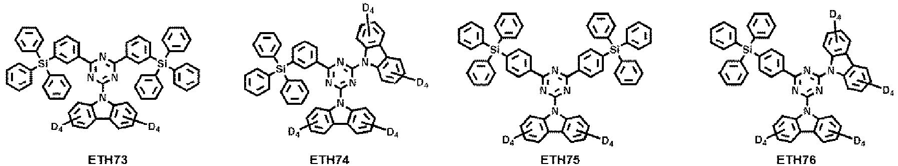

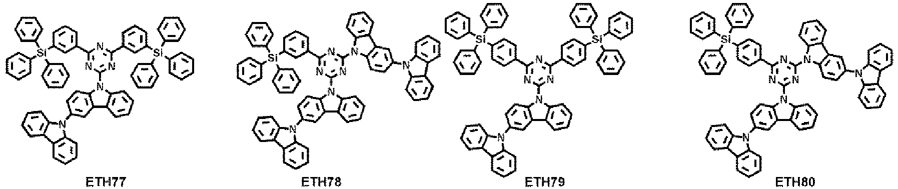

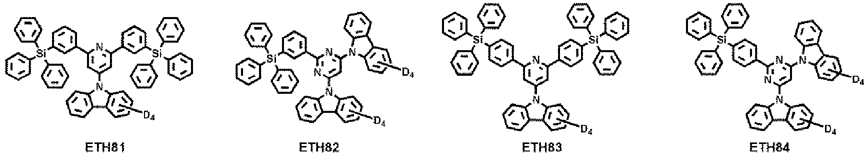

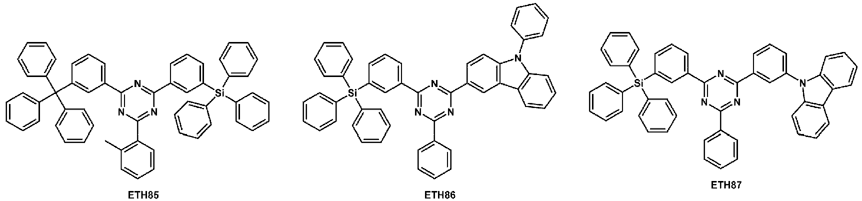

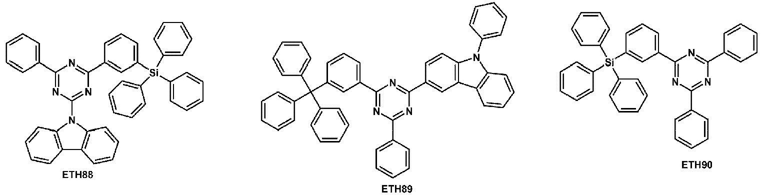

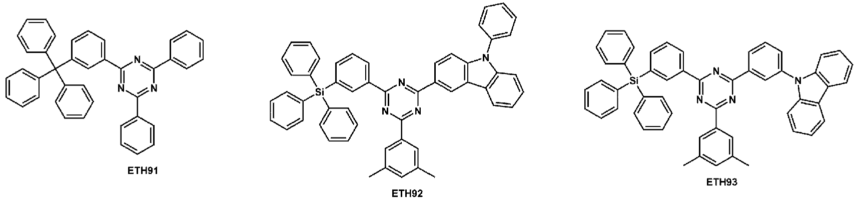

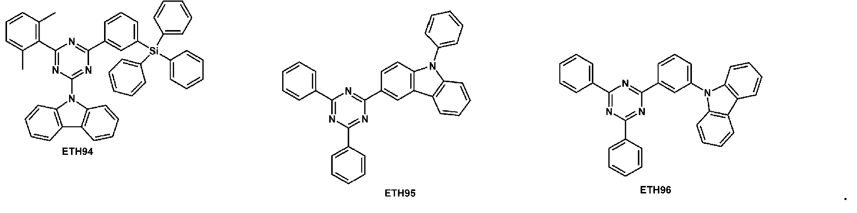

In an embodiment, the third compound may include at least one of Compounds PD1 to PD40:

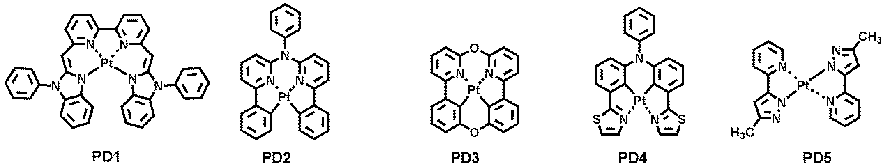

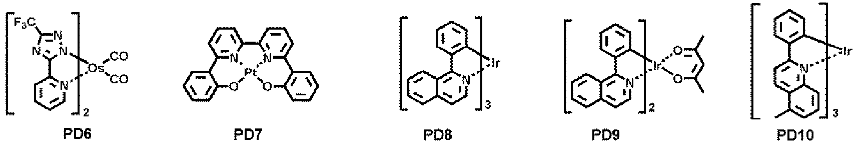

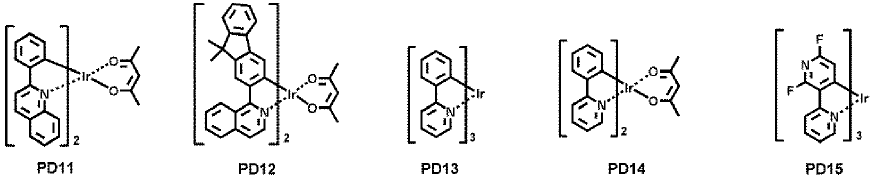

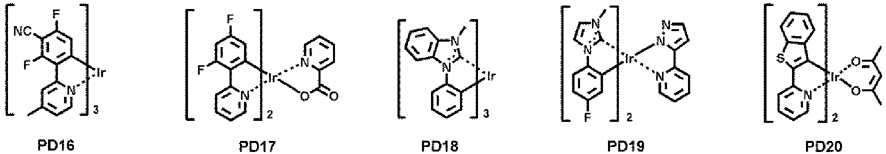

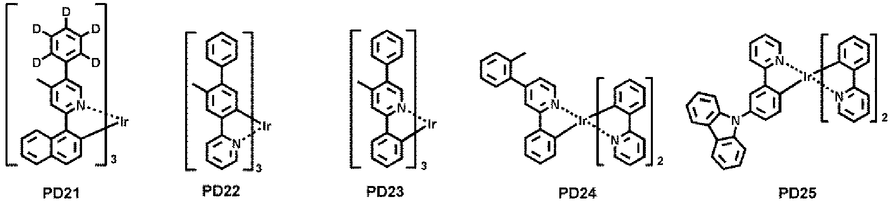

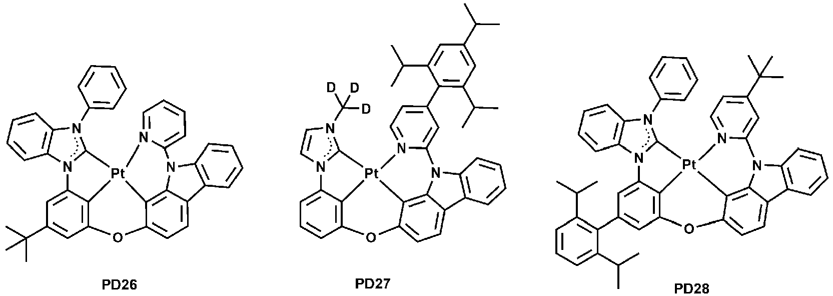

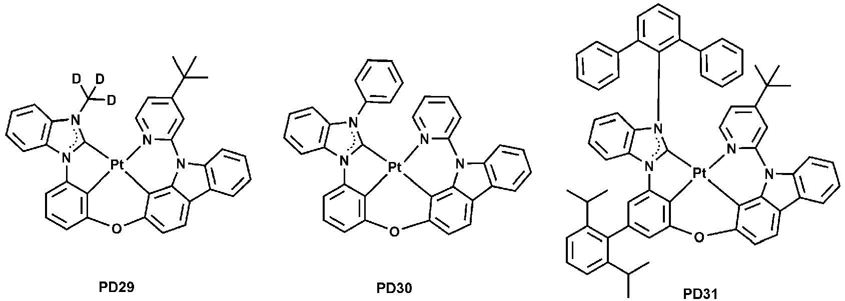

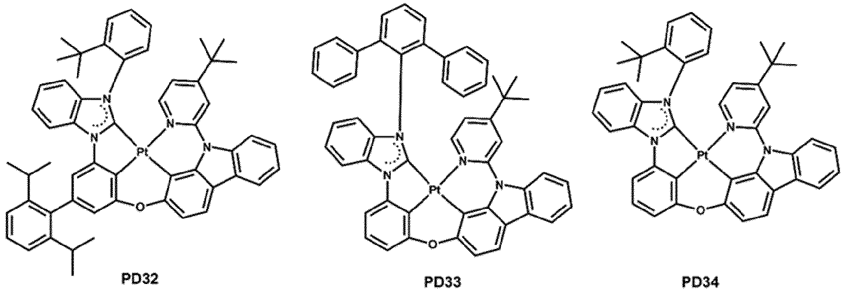

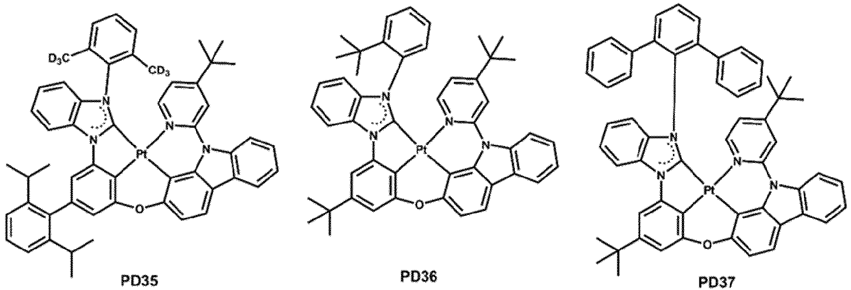

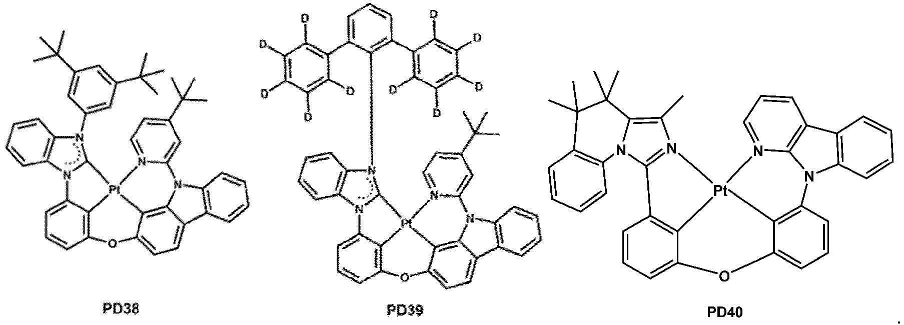

In an embodiment, the fourth compound may include at least one of Compounds DF8 to DF15:

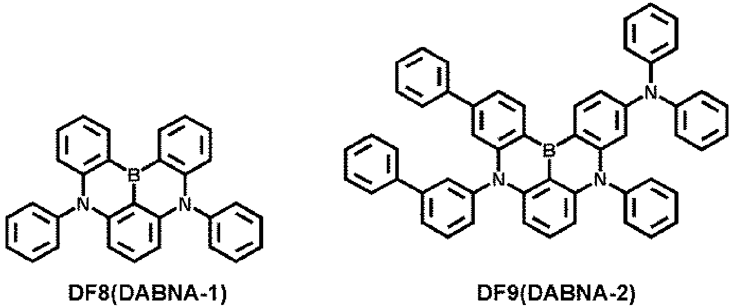

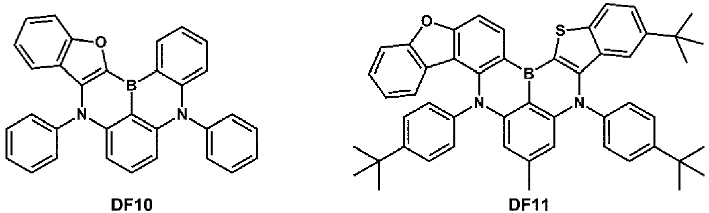

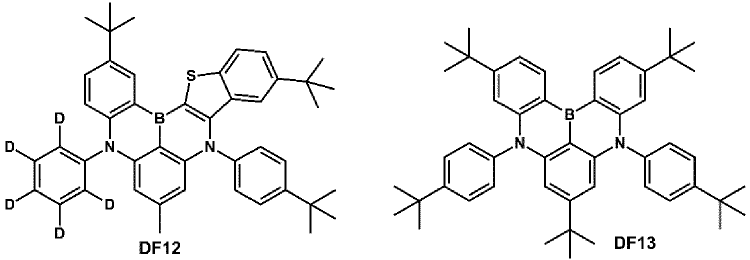

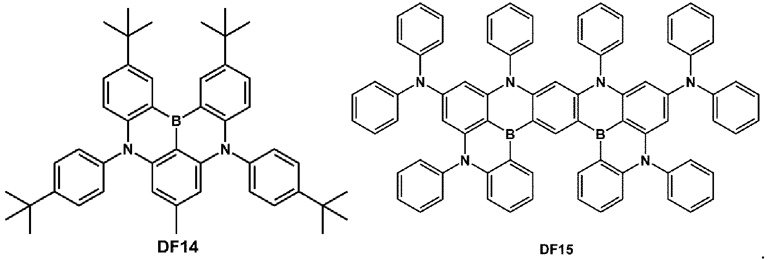

In Compounds ETH1 to ETH96, PD1 to PD40, and DF8 to DF15, Ph represents a phenyl group, D₅ represents substitution with five deuterium atoms, and D₄ represents substitution with four deuterium atoms. For example, a group represented by 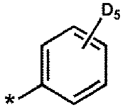 may be identical to a group represented by

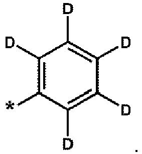

In an embodiment, the light-emitting device may satisfy at least one of Conditions 1 to 4:
[Condition 1]
   Lowest unoccupied molecular orbital (LUMO) energy level (eV) of heterocyclic compound > LUMO energy level (eV) of third compound;
[Condition 2]
   LUMO energy level (eV) of third compound > LUMO energy level (eV) of second compound;
[Condition 3]
   Highest occupied molecular orbital (HOMO) energy level (eV) of third compound > HOMO energy level (eV) of heterocyclic compound;
[Condition 4]
   HOMO energy level (eV) of heterocyclic compound> HOMO energy level (eV) of second compound.

The HOMO energy level and the LUMO energy level of each of the heterocyclic compound, the second compound, and the third compound may each be a negative value, and may be measured according to a method of the related art.

In an embodiment, an absolute value of a difference between a LUMO energy level of the third compound and a LUMO energy level of the second compound may be at least 0.1 eV but not more than 1.0 eV; or an absolute value of a difference between a LUMO energy level of the third compound and a LUMO energy level of the heterocyclic compound may be at least 0.1 eV but not more than 1.0 eV; or an absolute value of a difference between a HOMO energy level of the third compound and a HOMO energy level of the second compound may be 1.25 eV or less (e.g., at least 0.2 eV but not more than 1.25 eV); or an absolute value of a difference between a HOMO energy level of the third compound and a HOMO energy level of the heterocyclic compound may be 1.25 eV or less (e.g., at least 0.2 eV but not more than 1.25 eV).

When the relationships between the LUMO energy level and the HOMO energy level satisfy the aforementioned conditions, a balance between holes and electrons injected into the emission layer may be achieved.

The light-emitting device may have a structure according to a first embodiment or according to a second embodiment.

### [First embodiment]

According to a first embodiment, the heterocyclic compound may be included in an emission layer of the interlayer of a light-emitting device, the emission layer may further include a third compound, and the emission layer may emit phosphorescent light or fluorescent light from the third compound. For example, according to the first embodiment, the heterocyclic compound may be a host, and the third compound may be a dopant or an emitter. In an embodiment, the third compound may be a phosphorescent dopant or a phosphorescent emitter.

The phosphorescent light or the fluorescent light emitted from the third compound may be blue light.

The emission layer may further include an auxiliary dopant. The auxiliary dopant may improve luminescence efficiency from the third compound by effectively transferring energy to the third compound as a dopant or an emitter.

The auxiliary dopant may be different from each of the third compound and the heterocyclic compound.

In an embodiment, the auxiliary dopant may be a compound that emits delayed fluorescence.

In an embodiment, the auxiliary dopant may be a compound including at least one cyclic group that includes boron (B) and nitrogen (N) as ring-forming atoms.

The emission layer may further include one or more hosts that are different from the heterocyclic compound, the third compound, and the auxiliary dopant. For example, the emission layer may further include the second compound as a host.

### [Second embodiment]

According to a second embodiment, the heterocyclic compound may be included in an emission layer of the interlayer of a light-emitting device, the emission layer may further include a third compound and a dopant, the heterocyclic compound, the third compound, and the dopant are different from each other, and the emission layer may emit phosphorescent light or fluorescent light (e.g., delayed fluorescence) from the dopant. For example, according to the second embodiment, the heterocyclic compound may be a host, and the third compound may serve as an auxiliary dopant that transfers energy to the dopant (or emitter), rather than serve as a dopant.

In an embodiment, the heterocyclic compound may be a host, the third compound may serve as an emitter, and may also serve as an auxiliary dopant that transfers energy to the dopant (or emitter).

For example, phosphorescence or fluorescence emitted from the dopant (or the emitter) in the second embodiment may be blue phosphorescence or blue fluorescence (e.g., blue delayed fluorescence).

In the second embodiment, the dopant (or emitter) may be a phosphorescent dopant material (e.g., a third compound or a fluorescent dopant material as described in the specification, such as a compound represented by Formula 42, a compound represented by Formula 43, or any combination thereof, as described in the specification).

In the second embodiment, the emission layer may further include one or more hosts that are different from the heterocyclic compound, the third compound, and the dopant (or emitter). For example, the emission layer may further include the second compound as a host.

In the first and second embodiments, the blue light may have a maximum emission wavelength in a range of about 400 nm to about 500 nm. For example, the blue light may have a maximum emission wavelength in a range of about 410 nm to about 490 nm. For example, the blue light may have a maximum emission wavelength in a range of about 420 nm to about 480 nm. For example, the blue light may have a maximum emission wavelength in a range of about 430 nm to about 475 nm. For example, the blue light may have a maximum emission wavelength in a range of about 440 nm to about 475 nm. For example, the blue light may have a maximum emission wavelength in a range of about 450 nm to about 475 nm. For example, the blue light may have a maximum emission wavelength in a range of about 430 nm to about 470 nm. For example, the blue light may have a maximum emission wavelength in a range of about 440 nm to about 470 nm. For example, the blue light may have a maximum emission wavelength in a range of about 450 nm to about 470 nm. For example, the blue light may have a maximum emission wavelength in a range of about 430 nm to about 465 nm. For example, the blue light may have a maximum emission wavelength in a range of about 440 nm to about 465 nm. For example, the blue light may have a maximum emission wavelength in a range of about 450 nm to about 465 nm. For example, the blue light may have a maximum emission wavelength in a range of about 430 nm to about 460 nm. For example, the blue light may have a maximum emission wavelength in a range of about 440 nm to about 460 nm. For example, the blue light may have a maximum emission wavelength in a range of about 450 nm to about 460 nm.

In the first embodiment, the auxiliary dopant may include, for example, a delayed fluorescent compound represented by Formula 42 or Formula 43, as described in the specification.

In the first and second embodiments, the host may further include any host material (e.g., a compound represented by Formula 301, a compound represented by 301-1, a compound represented by Formula 301-2, or any combination thereof).

In an embodiment, the light-emitting device may further include a capping layer arranged outside the first electrode and/or outside the second electrode.

In an embodiment, the light-emitting device may further include at least one of a first capping layer arranged outside the first electrode and a second capping layer arranged outside the second electrode, and at least one of the first capping layer and the second capping layer may include the heterocyclic compound. Further details on the first capping layer and/or the second capping layer may be the same as described herein.

In an embodiment, the light-emitting device may include:
a first capping layer located outside the first electrode and including the heterocyclic compound represented by Formula 1;
a second capping layer located outside the second electrode and including the heterocyclic compound represented by Formula 1; or
the first capping layer and the second capping layer.

In the specification, the expression "(an interlayer and/or a capping layer) includes a heterocyclic compound represented by Formula 1" may include a case in which "(an interlayer and/or a capping layer) includes a same heterocyclic compound represented by Formula 1" and a case in which "(an interlayer and/or a capping layer) includes two or more different heterocyclic compounds, each independently represented by Formula 1."

For example, the interlayer and/or the capping layer may include, as the heterocyclic compound, Compound 1 only. In this regard, Compound 1 may be present in an emission layer of the light-emitting device. In an embodiment, the interlayer may include, as the heterocyclic compound, Compounds 1 and 2. In this regard, Compound 1 and Compound 2 may be present in a same layer (e.g., both Compound 1 and Compound 2 may be present in an emission layer), or may be present in different layers (e.g., Compound 1 may be present in the emission layer, and Compound 2 may be present in the electron transport region).

In the specification, the term "interlayer" may refer to a single layer and/or multiple layers between the first electrode and the second electrode of the light-emitting device.

According to an embodiment, an electronic apparatus includes the light-emitting device. In an embodiment, the electronic apparatus may further include a thin-film transistor. In an embodiment, the electronic apparatus may further include a thin-film transistor including a source electrode and a drain electrode, wherein the first electrode of the light-emitting device may be electrically connected to the source electrode or the drain electrode. In an embodiment, the electronic apparatus may further include a color filter, a color conversion layer, a touch screen layer, a polarizing layer, or any combination thereof. Further details on the electronic apparatus may be the same as described herein.

According to another embodiment, an electronic device includes the light-emitting device.

For example, the electronic device may be a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater screen, a stadium screen, a phototherapy device, or a signboard.

According to embodiments, a heterocyclic compound is represented by Formula 1. The heterocyclic compound represented Formula 1 will be described in further detail below.

Synthesis methods of the heterocyclic compound may be recognizable by one of ordinary skill in the art by referring to the Synthesis Examples and/or the Examples provided below.

[Description of Formula 1]

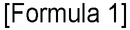

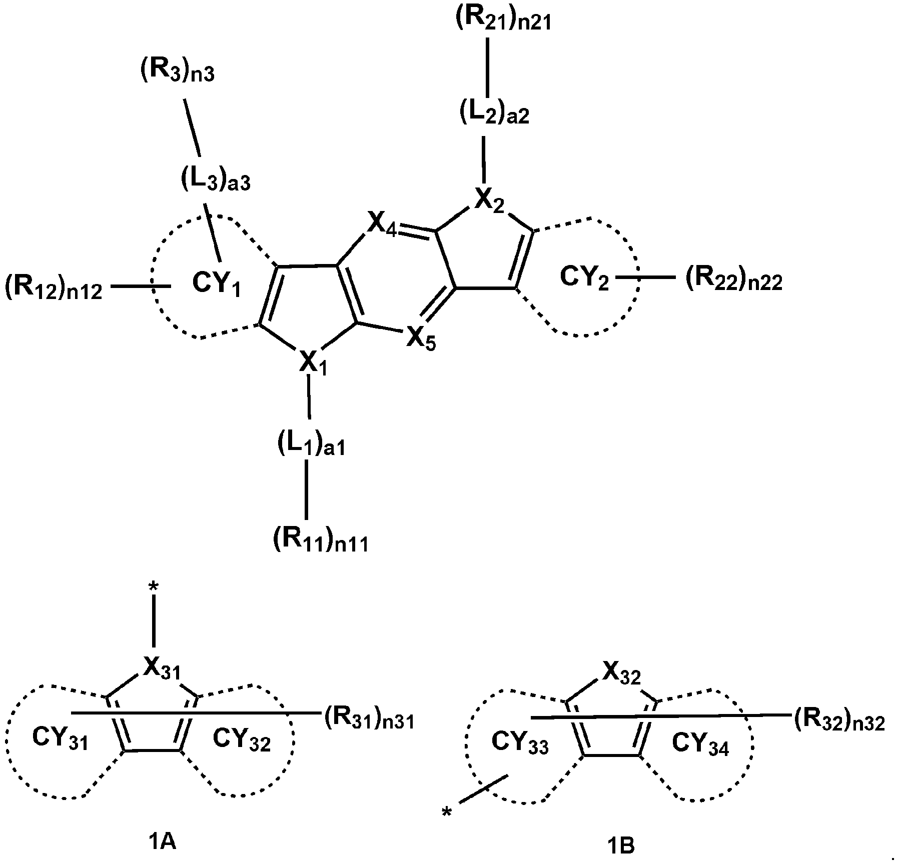

According to embodiments, in Formulae 1, 1A, and 1B,
X₁ is C(R₁) or N, X₂ is C(R₂) or N, X₃₁ is C(R₃ₐ) or N, X₃₂ is N(R₃ₐ) or C(R₃ₐ)(R_{3b}), X₄ is C(R₄) or N, and X₅ is C(R₅) or N.

In an embodiment, X₁ may be N.

In an embodiment, X₂ may be N.

In an embodiment, X₄ may be C(R₄).

In an embodiment, X₃₁ may be N.

In an embodiment, X₃₂ may be N(R₃ₐ).

In an embodiment, X₅ may be C(R₅).

In Formula 1, L₁ to L₃ are each independently a single bond, a C₅-C₆₀ (e.g. C₅-C₃₀) carbocyclic group unsubstituted or substituted with at least one R₁₀ₐ, or a C₁-C₆₀ (e.g. C₁-C₂₀) heterocyclic group unsubstituted or substituted with at least one R₁₀ₐ.

In an embodiment, L₁ to L₃ may each independently be:
a single bond; or
a benzene group, a naphthalene group, a phenanthrene group, a fluorene group, a carbazole group, a dibenzofuran group, a dibenzothiophene group, an azacarbazole group, an azadibenzofuran group, or azadibenzothiophene group, each unsubstituted or substituted with at least one R₁₀ₐ.

In an embodiment, L₁ to L₃ may each independently be a single bond, or a group represented by one of Formula 2-1 to Formula 2-5:

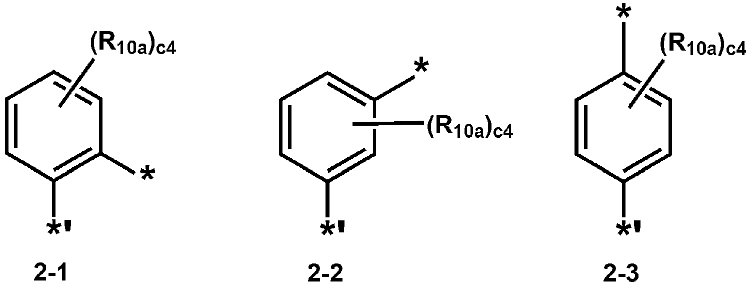

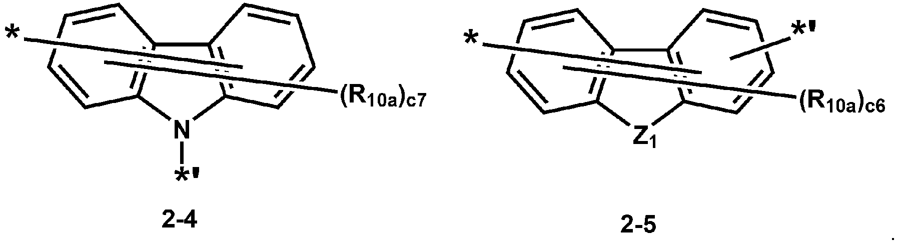

In Formulae 2-1 to 2-5,
Z₁ is O, S, N(R₁'), or C(R₁')(R₂'),
R₁' and R₂' are each independently the same as described in connection with R₁,
R₁₀ₐ is the same as described herein,
c4 is an integer from 0 to 4,
c6 is an integer from 0 to 6,
c7 is an integer from 0 to 7, and
* and *' each indicate a binding site to a neighboring atom.

In Formula 1, a1 to a3 are each independently an integer from 1 to 3. For example, when a1 is 1 and L₁ is a single bond, X₁ and R₁₁ may directly be connected to each other.

In Formulae 1, 1A, and 1B, ring CY₁, ring CY₂, and ring CY₃₁ to ring CY₃₄ are each independently a C₅-C₆₀ (e.g. C₅-C₃₀) carbocyclic group or a C₁-C₆₀ (e.g. C₁-C₂₀) heterocyclic group.

In an embodiment, ring CY₁, ring CY₂, and ring CY₃₁ to ring CY₃₄ may each independently be a benzene group, a naphthalene group, an anthracene group, a phenanthrene group, a triphenylene group, a pyrene group, a chrysene group, a cyclopentadiene group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an azafluorene group, an azacarbazole group, an azadibenzofuran group, an azadibenzothiophene group, an azadibenzosilole group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a dibenzoxasiline group, a dibenzothiasiline group, a dibenzodihydroazasiline group, a dibenzodihydrodisiline group, a dibenzodihydrosiline group, a dibenzodioxine group, a dibenzoxathiine group, a dibenzoxazine group, a dibenzopyran group, a dibenzodithiine group, a dibenzothiazine group, a dibenzothiopyran group, a dibenzocyclohexadiene group, a dibenzodihydropyridine group, or a dibenzodihydropyrazine group.

In an embodiment, ring CY₁, and ring CY₃₁ to CY₃₄ may each be a benzene group, and ring CY₂ may be a furan group or a thiophene group.

In Formulae 1, 1A, and 1B, R₁, R₂, R₃ₐ, R_{3b}, R₄, R₅, R₁₁, R₁₂, R₂₁, R₂₂, R₃, R₃₁, and R₃₂ are each independently a group represented by Formula 1A, a group represented by Formula 1B, hydrogen, deuterium, -F, -Cl, -Br, -I, a hydroxyl group, a cyano group, a nitro group, a C₁-C₆₀ (e.g. C₁-C₂₀) alkyl group unsubstituted or substituted with at least one R₁₀ₐ, a C₂-C₆₀ (e.g. C₂-C₂₀) alkenyl group unsubstituted or substituted with at least one R₁₀ₐ, a C₂-C₆₀ (e.g. C₂-C₂₀) alkynyl group unsubstituted or substituted with at least one R₁₀ₐ, a C₁-C₆₀ (e.g. C₁-C₂₀) alkoxy group unsubstituted or substituted with at least one R₁₀ₐ, a C₃-C₆₀ (e.g. C₃-C₃₀) carbocyclic group unsubstituted or substituted with at least one R₁₀ₐ, a C₁-C₆₀ (e.g. C₁-C₂₀) heterocyclic group unsubstituted or substituted with at least one R₁₀ₐ, a C₆-C₆₀ (e.g. C₆-C₃₀) aryloxy group unsubstituted or substituted with at least one R₁₀ₐ, a C₆-C₆₀ (e.g. C₆-C₃₀) arylthio group unsubstituted or substituted with at least one R₁₀ₐ, a C₆-C₆₀ arylseleno group unsubstituted or substituted with at least one R₁₀ₐ, a C₇-C₆₀ (e.g. C₇-C₃₀) aryl alkyl group unsubstituted or substituted with at least one R₁₀ₐ, a C₂-C₆₀ (e.g. C₂-C₃₀) heteroaryl alkyl group unsubstituted or substituted with at least one R₁₀ₐ, - Si(Q₁)(Q₂)(Q₃), -Ge(Q₁)(Q₂)(Q₃), -N(Q₁)(Q₂), -B(Q₁)(Q₂), -C(=O)(Q₁), -S(=O)₂(Q₁), or - P(=O)(Q₁)(Q₂).

In an embodiment, R₁, R₂, R₃ₐ, R_{3b}, R₄, R₅, R₁₁, R₁₂, R₂₁, R₂₂, R₃, R₃₁, and R₃₂ may each independently be:
a group represented by Formula 1A, a group represented by Formula 1B, hydrogen, deuterium, -F, -Cl, -Br, -I, a hydroxyl group, a cyano group, a nitro group, a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, a C₁-C₆₀ (e.g. C₁-C₂₀) alkoxy group, a phenyl group, a biphenyl group, or a terphenyl group;
a C₁-C₆₀ (e.g. C₁-C₂₀) alkyl group, a C₃-C₆₀ (e.g. C₃-C₂₀) cycloalkyl group, a C₁-C₆₀ (e.g. C₁-C₂₀) heterocycloalkyl group, a phenyl group, a biphenyl group, a terphenyl group, a naphthyl group, an anthracenyl group, a phenanthrenyl group, a phenalenyl group, a triphenylenyl group, a pyrenyl group, a chrysenyl group, a pyrrolyl group, a furanyl group, a thiophenyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a phenanthrolinyl group, a benzoimidazolyl group, a benzofuranyl group, a dibenzofuranyl group, a benzothiophenyl group, a dibenzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a carbazolyl group, a benzocarbazolyl group, a fluorenyl group, a benzoisothiazolyl group, a benzoxazolyl group, a benzoisoxazolyl group, a phenoxazinyl group, an acridinyl group, or a xanthenyl group, each unsubstituted or substituted with deuterium, -F, -Cl, -Br, -I, a hydroxyl group, a cyano group, a nitro group, a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, a C₁-C₆₀ (e.g. C₁-C₂₀) alkoxy group, a phenyl group, a biphenyl group, a terphenyl group, or any combination thereof; or
-Si(Q₁)(Q₂)(Q₃), -Ge(Q₁)(Q₂)(Q₃), or -N(Q₁)(Q₂).

In Formula 1, at least one of R₃ in the number of n3 is a group represented by Formula 1A or Formula 1B. Thus, the heterocyclic compound represented by Formula 1 includes at least one group represented by Formula 1A or Formula 1B.

In Formulae 1, 1A, and 1B,
n11 is an integer from 0 to 20,
n12 is an integer from 0 to 30,
n21 is an integer from 0 to 20,
n22 is an integer from 0 to 30,
n3 is an integer from 0 to 30,
n31 is an integer from 0 to 20,
n32 is an integer from 0 to 20, and
* indicates a binding site to a neighboring atom.

In Formulae 1, 1A, and 1B, R₁₀ₐ is:
deuterium, -F, -Cl, -Br, -I, a hydroxyl group, a cyano group, or a nitro group;
a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, or a C₁-C₆₀ (e.g. C₁-C₂₀) alkoxy group, each unsubstituted or substituted with deuterium, -F, -Cl, -Br, -I, a hydroxyl group, a cyano group, a nitro group, a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ arylalkyl group, a C₂-C₆₀ heteroarylalkyl group, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), -P(=O)(Q₁₁)(Q₁₂), or any combination thereof;
a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, a C₁-C₆₀ (e.g. C₁-C₂₀) alkoxy group, a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; a C₁-C₆₀ (e.g. C₁-C₂₀) alkyl group; a C₂-C₆₀ (e.g. C₂-C₂₀) alkenyl group; a C₂-C₆₀ (e.g. C₂-C₂₀) alkynyl group; a C₁-C₆₀ (e.g. C₁-C₂₀) alkoxy group; or a C₃-C₆₀ (e.g. C₃-C₃₀) carbocyclic group, a C₁-C₆₀ (e.g. C₁-C₂₀) heterocyclic group, a C₇-C₆₀ arylalkyl group, or a C₂-C₆₀ heteroarylalkyl group, each unsubstituted or substituted with deuterium, - F, a cyano group, a C₁-C₆₀ alkyl group, a C₁-C₆₀ (e.g. C₁-C₂₀) alkoxy group, a phenyl group, a biphenyl group, a C₃-C₆₀ (e.g. C₃-C₃₀) carbocyclic group, a C₁-C₆₀ (e.g. C₁-C₂₀) heterocyclic group, or any combination thereof.

In an embodiment, the heterocyclic compound represented may include:
i) at least one carbazole group; or
ii) at least one deuterium; or
iii) at least one -Si(Q₁)(Q₂)(Q₃); or
iv) at least one cyano group; or
v) any combination selected from i) to iv).

For example, the heterocyclic compound may include at least one carbazole group, at least one deuterium, at least one silicon, or at least one cyano group.

In an embodiment, the group represented by Formula 1A may be represented by one of Formula 1A-1 to Formula 1A-6, and the group represented by Formula 1B may be represented by one of Formula 1B-1 to Formula 1B-12:

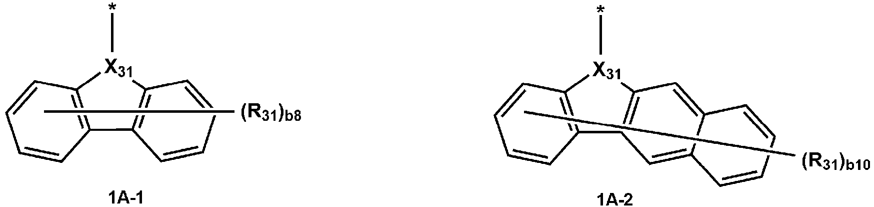

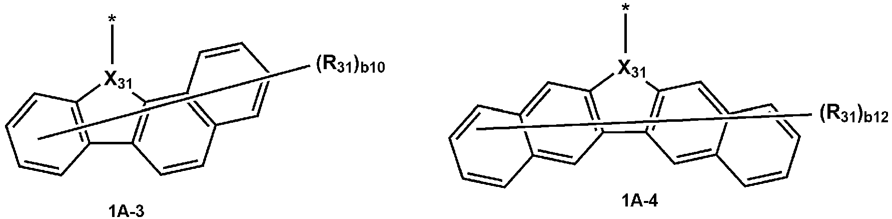

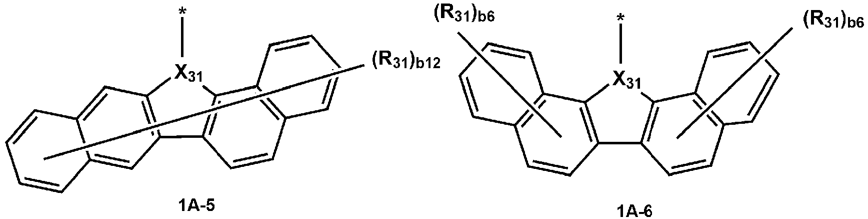

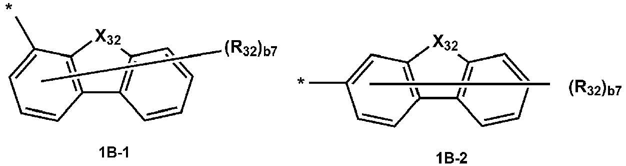

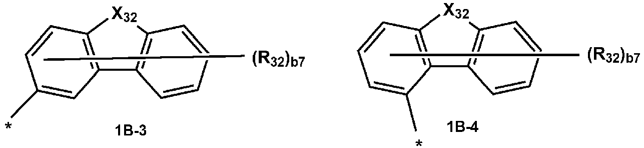

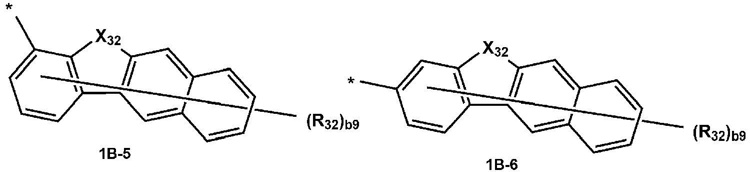

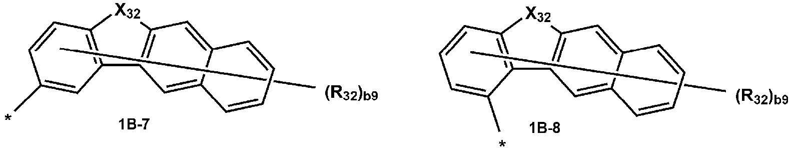

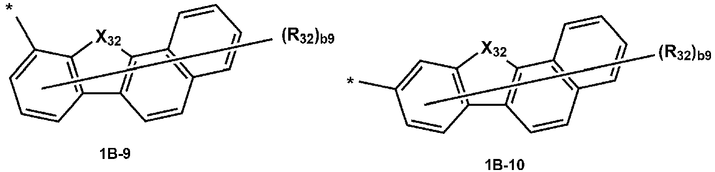

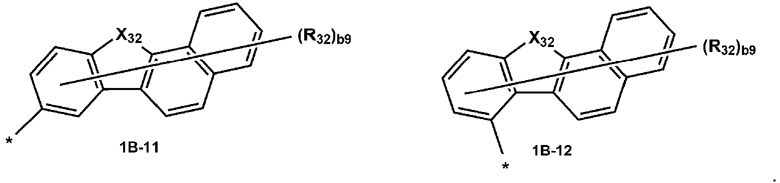

In Formulae 1A-1 to 1A-6 and 1B-1 to 1B-12,
X₃₁, X₃₂, R₃₁, and R₃₂ are the same as described herein,
b6 is an integer from 0 to 6,
b7 is an integer from 0 to 7,
b8 is an integer from 0 to 8,
b9 is an integer from 0 to 9,
b10 is an integer from 0 to 10,
b12 is an integer from 0 to 12, and
* indicates a binding site to a neighboring atom.

In an embodiment, the heterocyclic compound represented by Formula 1 may be represented by any one of Formula 1-1 to Formula 1-4:

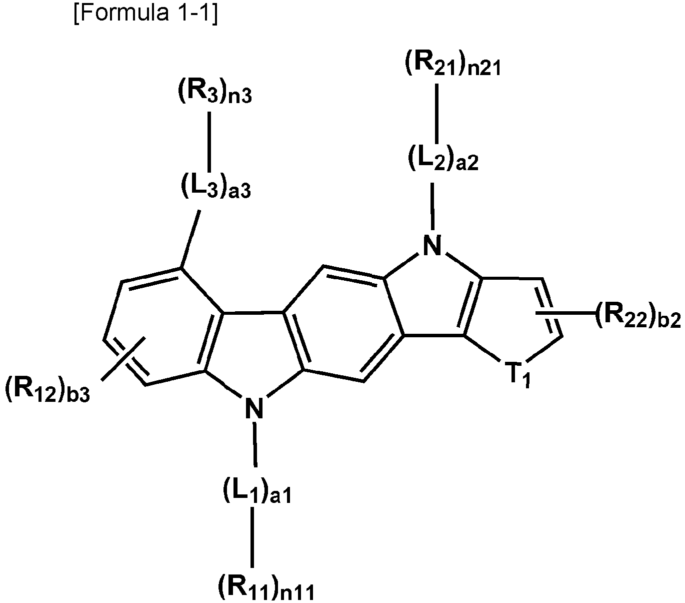

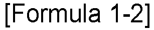

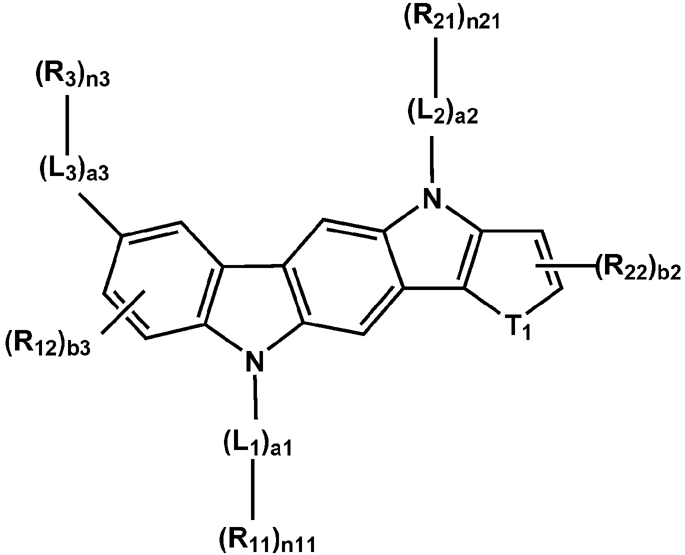

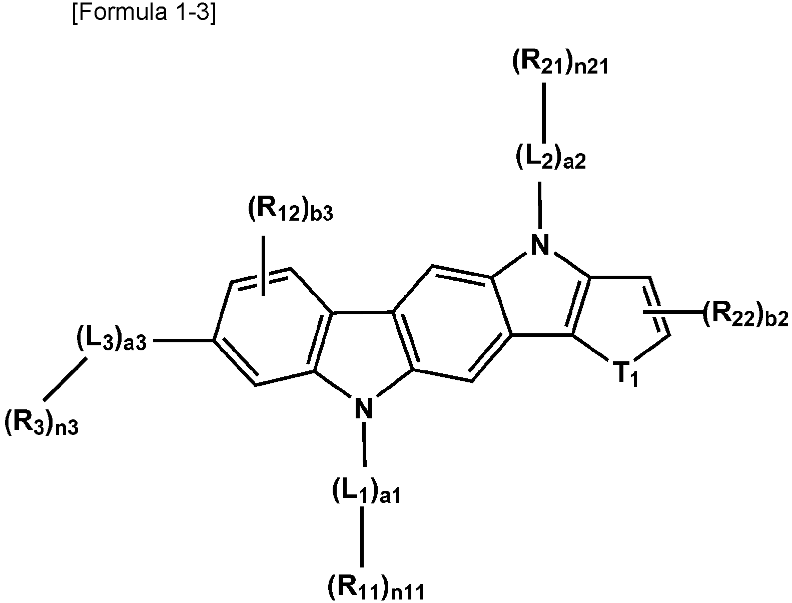

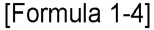

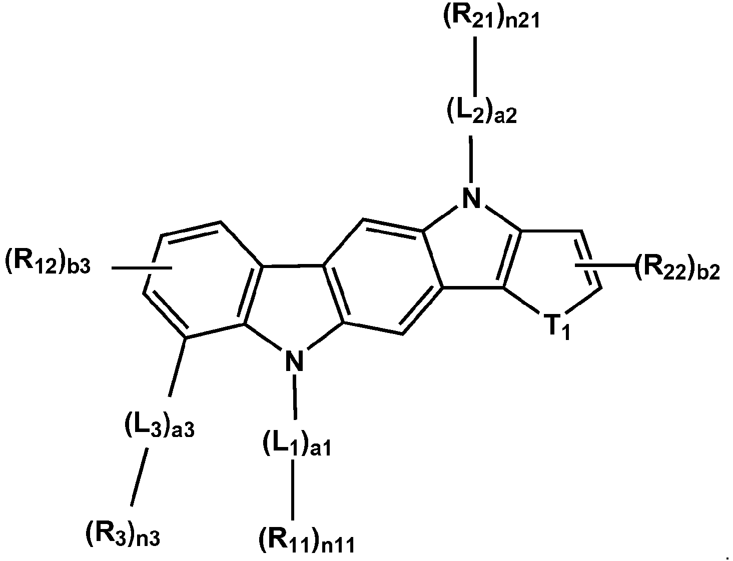

In Formulae 1-1 to 1-4,
L₁ to L₃, a1 to a3, R₁₁, R₁₂, R₂₁, R₂₂, R₃, n11, n21, and n3 are respectively the same as described herein,
T₁ is O or S,
b2 is an integer from 0 to 2, and
b3 is an integer from 0 to 3.

### [Examples of heterocyclic compound]

In an embodiment, the heterocyclic compound represented by Formula 1 (e.g., the first compound) may be one of Compounds 1 to 21. In an embodiment, in the light-emitting device, the first compound may include at least one of Compounds 1 to 21:

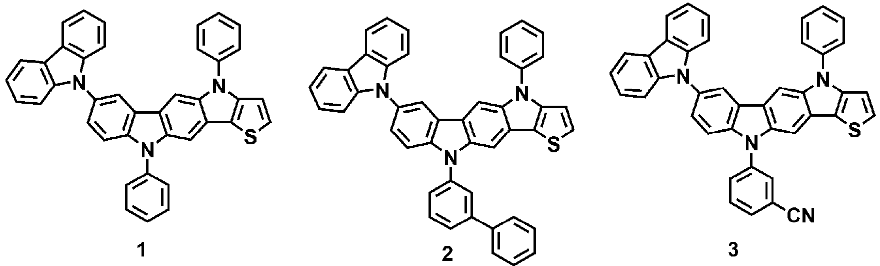

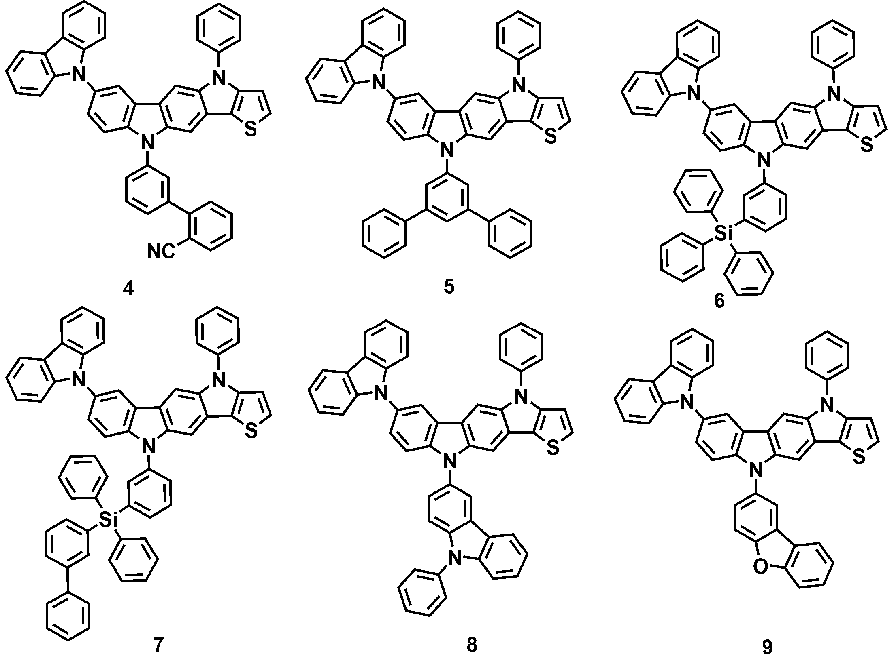

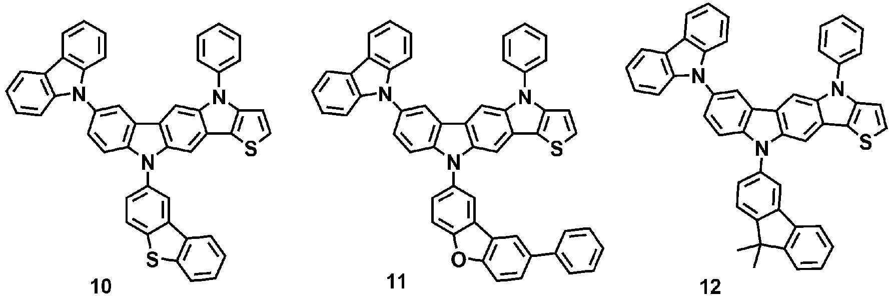

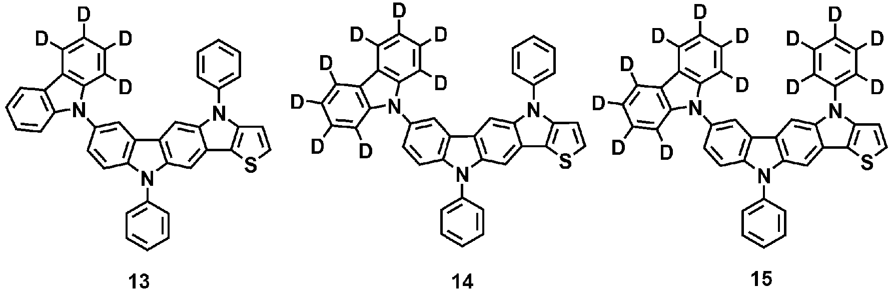

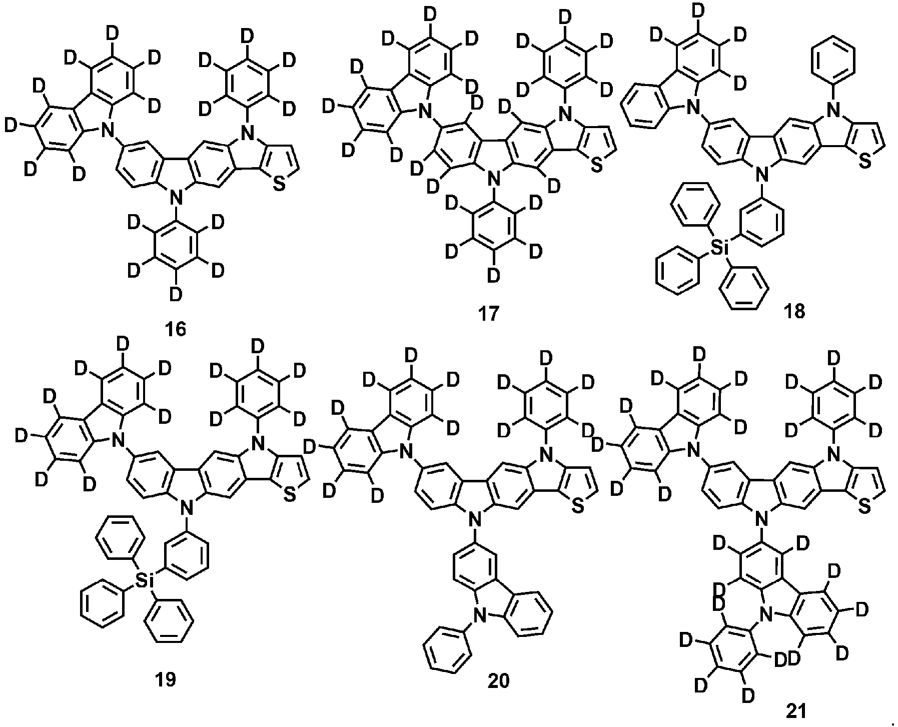

The heterocyclic compound represented by Formula 1 contains a C-C bond and has a core structure with enhanced planarity. As a result of this structure, the thermal stability of the heterocyclic compound may be improved.

The heterocyclic compound has enhanced electron-donating properties and thus can improve hole injection and hole transport capabilities so that when the heterocyclic compound is used in an emission layer, luminescence efficiency and color purity can be significantly improved.

The heterocyclic compound represented by Formula 1 may have a high T₁ energy level due to the inclusion of a group represented by Formula 1A or Formula 1B at the position of R₃.

The heterocyclic compound represented by Formula 1 may have improved lifetime characteristics due to the inclusion of at least one deuterium.

Therefore, a light-emitting device including the heterocyclic compound can have excellent low driving voltage, luminescence efficiency, lifespan characteristics, and color purity characteristics.

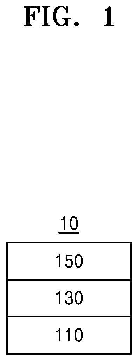

### [Description of FIG. 1]

FIG. 1 is a schematic cross-sectional view of a light-emitting device 10 according to an embodiment. The light-emitting device 10 includes a first electrode 110, an interlayer 130, and a second electrode 150.

Hereinafter, a structure of the light-emitting device 10 according to an embodiment and a method of manufacturing the light-emitting device 10 are described with reference to FIG. 1.

### [First electrode 110]

In FIG. 1, a substrate may be further included under the first electrode 110 or on the second electrode 150. The substrate may be a glass substrate or a plastic substrate. In an embodiment, the substrate may be a flexible substrate and may include plastics with excellent heat resistance and durability, such as polyimide, polyethylene terephthalate (PET), polycarbonate, polyethylene naphthalate, polyarylate (PAR), polyetherimide, or any combination thereof.

The first electrode 110 may be formed by, for example, depositing or sputtering a material for forming the first electrode 110 on the substrate. When the first electrode 110 is an anode, a material for forming the first electrode 110 may be a high-work function material that facilitates the injection of holes.

The first electrode 110 may be a reflective electrode, a transflective electrode, or a transmissive electrode. When the first electrode 110 is a transmissive electrode, a material for forming the first electrode 110 may include indium tin oxide (ITO), indium zinc oxide (IZO), tin oxide (SnO₂), zinc oxide (ZnO), or any combination thereof. In an embodiment, when the first electrode 110 is a transflective electrode or a reflective electrode, a material for forming the first electrode 110 may include magnesium (Mg), silver (Ag), aluminium (Al), aluminium-lithium (Al-Li), calcium (Ca), magnesium-indium (Mg-In), magnesium-silver (Mg-Ag), or any combination thereof.

The first electrode 110 may have a structure consisting of a single layer or a structure including multiple layers. In an embodiment, the first electrode 110 may have a three-layered structure of ITO/Ag/ITO.

### [Interlayer 130]

The interlayer 130 may be disposed on the first electrode 110. The interlayer 130 includes an emission layer.

The interlayer 130 may further include a hole transport region between the first electrode 110 and the emission layer, and an electron transport region between the emission layer and the second electrode 150.

The interlayer 130 may further include, in addition to various organic materials, an inorganic material such as a metal-containing compound, quantum dots, or the like.

In an embodiment, the interlayer 130 may include two or more emitting units stacked between the first electrode 110 and the second electrode 150, and at least one charge generation layer, each between adjacent units among the two or more emitting units. When the interlayer 130 includes the two or more emitting units and the at least one charge generation layer as described above, the light-emitting device 10 may be a tandem light-emitting device.

### [Hole transport region in interlayer 130]

The hole transport region may have a structure consisting of a layer consisting of a single material, a structure consisting of a layer including different materials, or a structure including multiple layers including different materials.

The hole transport region may include a hole injection layer, a hole transport layer, an emission auxiliary layer, an electron blocking layer, or any combination thereof.

In embodiments, the hole transport region may have a multilayered structure, for example, a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein the layers of each structure may be stacked from the first electrode 110 in its respective stated order, but the structure of the hole transport region is not limited thereto.

In embodiments, the hole transport region may include a compound represented by Formula 201, a compound represented by Formula 202, or any combination thereof:

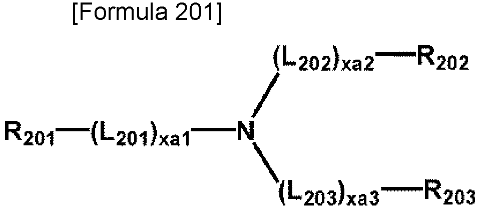

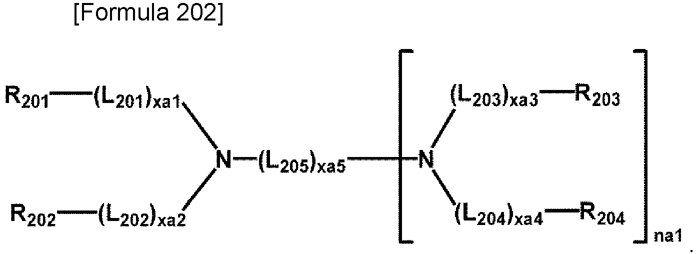

In Formulae 201 and 202,
L₂₀₁ to L₂₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
L₂₀₅ may be *-O-*', *-S-*', *-N(Q₂₀₁)-*', a C₁-C₂₀ alkylene group unsubstituted or substituted with at least one R₁₀ₐ, a C₂-C₂₀ alkenylene group unsubstituted or substituted with at least one R₁₀ₐ, a C₃-C₆₀ carbocyclic group unsubstituted or substituted with at least one R₁₀ₐ, or a C₁-C₆₀ heterocyclic group unsubstituted or substituted with at least one R₁₀ₐ,
xa1 to xa4 may each independently be an integer from 0 to 5,
xa5 may be an integer from 1 to 10,
R₂₀₁ to R₂₀₄ and Q₂₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
R₂₀₁ and R₂₀₂ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ to form a C₈-C₆₀ polycyclic group (e.g., a carbazole group or the like) unsubstituted or substituted with at least one R₁₀ₐ (e.g., see Compound HT16),
R₂₀₃ and R₂₀₄ may optionally be linked to each other via a single bond, a C₁-C₅ alkylene group that is unsubstituted or substituted with at least one R₁₀ₐ, or a C₂-C₅ alkenylene group that is unsubstituted or substituted with at least one R₁₀ₐ to form a C₈-C₆₀ polycyclic group that is unsubstituted or substituted with at least one R₁₀ₐ, and
na1 may be an integer from 1 to 4.

In an embodiment, the compound represented by Formula 201 and the compound represented by Formula 202 may each independently include at least one of groups represented by Formulae CY201 to CY217:

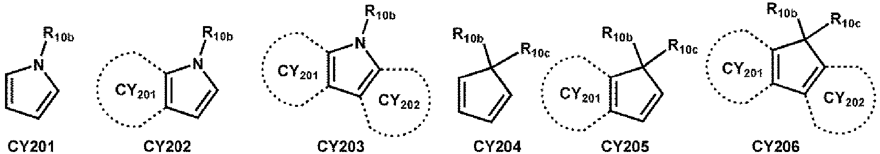

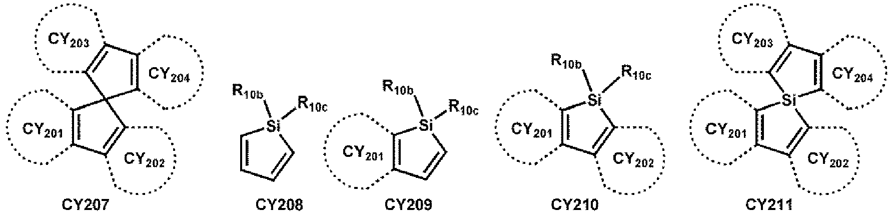

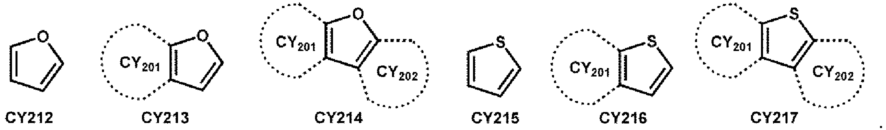

In Formulae CY201 to CY217, R_{10b} and R_{10c} may each independently be the same as described in connection with R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ.

In an embodiment, in Formulae CY201 to CY217, ring CY₂₀₁ to ring CY₂₀₄ may each independently be a benzene group, a naphthalene group, a phenanthrene group, or an anthracene group.

In an embodiment, the compound represented by Formula 201 and the compound represented by Formula 202 may each independently include at least one of groups represented by Formulae CY201 to CY203.

In an embodiment, the compound represented by Formula 201 may include at least one of groups represented by Formulae CY201 to CY203 and at least one of groups represented by Formulae CY204 to CY217.

In an embodiment, in Formula 201, xa1 may be 1, R₂₀₁ may be a group represented by one of Formulae CY201 to CY203, xa2 may be 0, and R₂₀₂ may be a group represented by one of Formulae CY204 to CY207.

In an embodiment, the compound represented by Formula 201 and the compound represented by Formula 202 may each not include a group represented by one of Formulae CY201 to CY203.

In an embodiment, the compound represented by Formula 201 and the compound represented by Formula 202 may each not include a group represented by one of Formulae CY201 to CY203, and may each independently include at least one group represented by one of Formulae CY204 to CY217.

In an embodiment, the compound represented by Formula 201 and the compound represented by Formula 202 may each not include a group represented by one of Formulae CY201 to CY217.

In an embodiment, the hole transport region may include one of Compounds HT1 to HT46, m-MTDATA, TDATA, 2-TNATA, NPB (NPD), β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzene sulfonic acid (PANI/DBSA), poly(3,4-ethylene dioxythiophene)/poly(4-styrene sulfonate) (PEDOT/PSS), polyaniline/camphor sulfonic acid (PANI/CSA), polyaniline/poly(4-styrene sulfonate) (PANI/PSS), or any combination thereof:

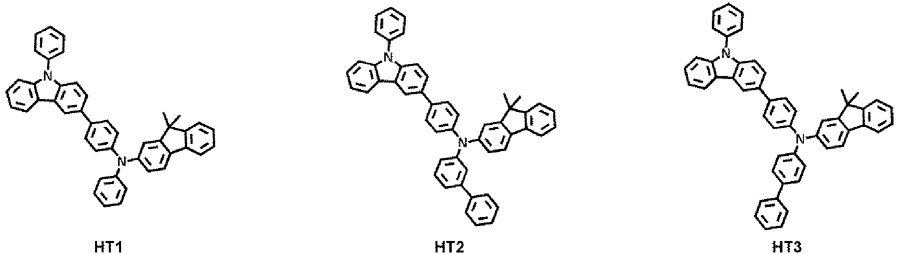

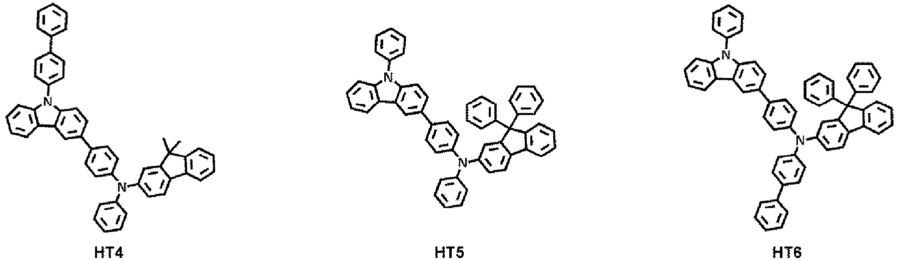

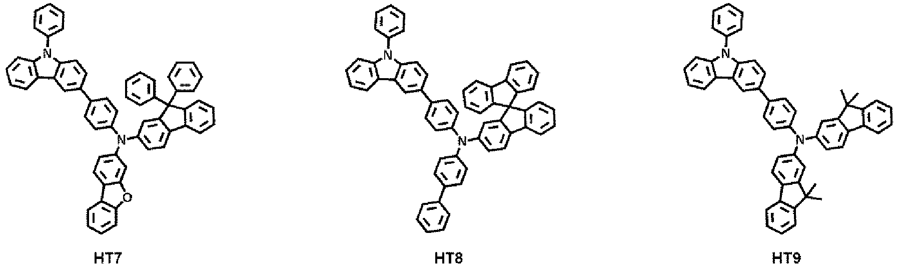

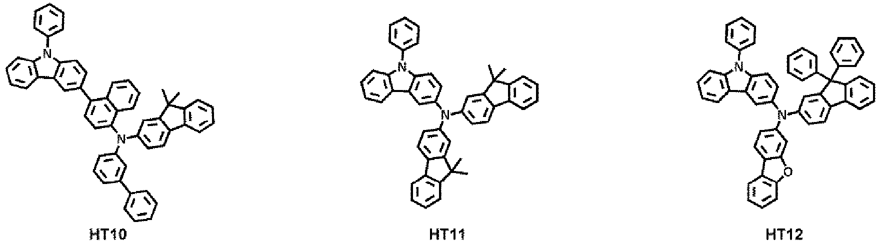

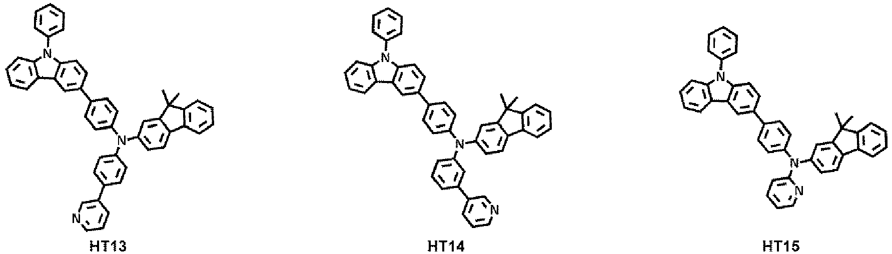

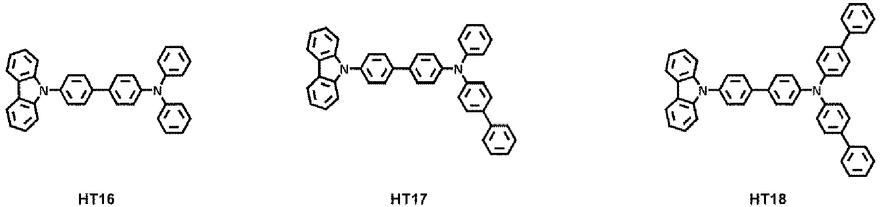

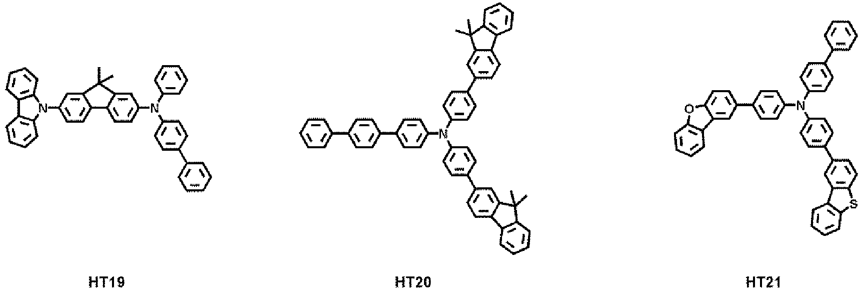

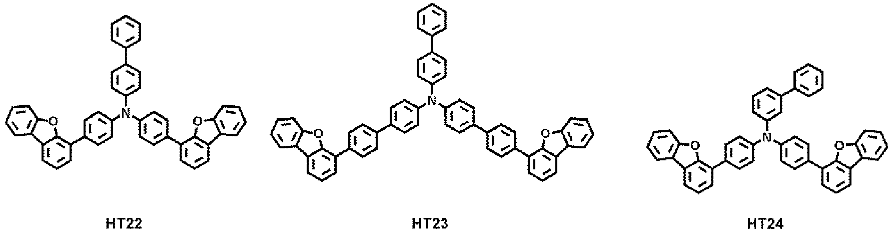

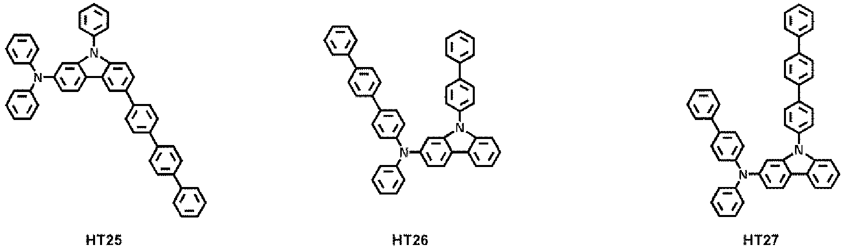

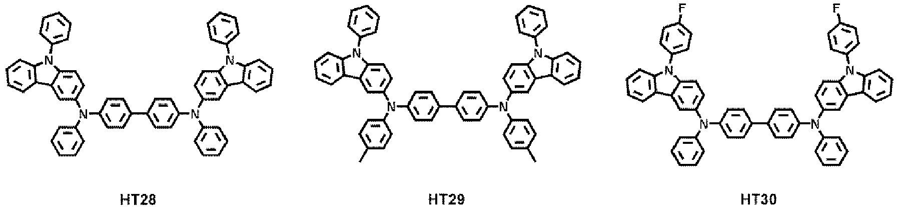

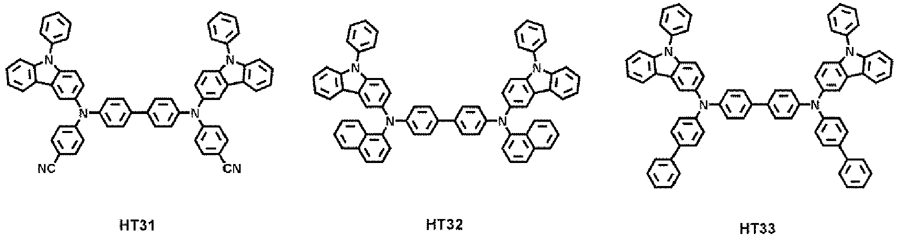

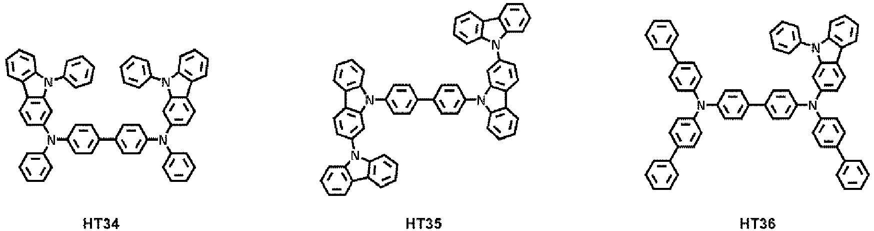

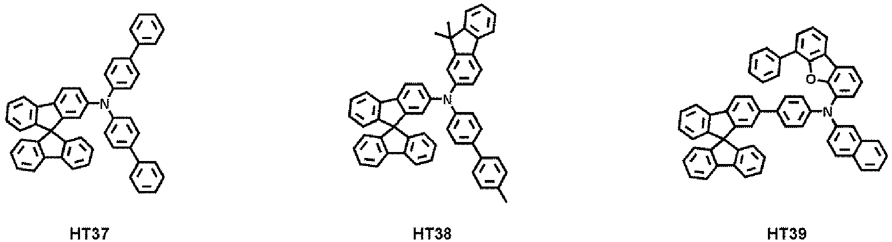

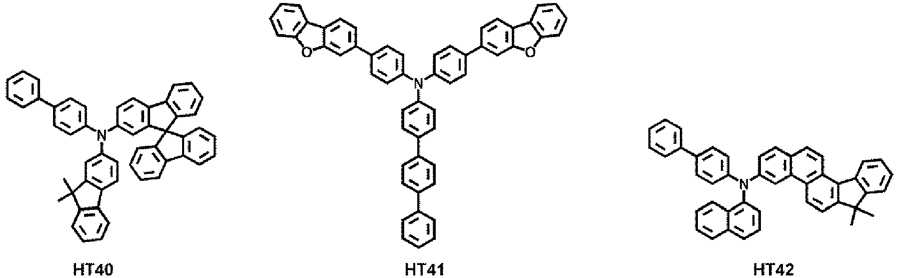

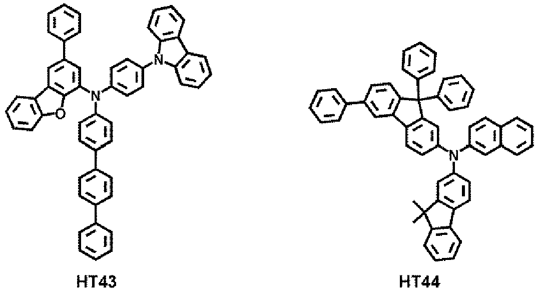

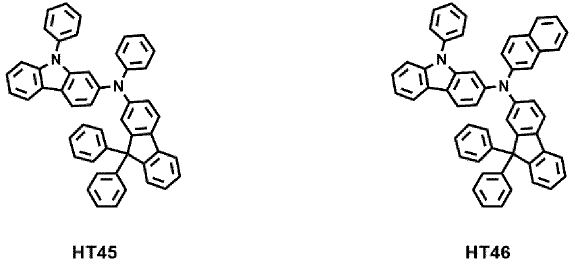

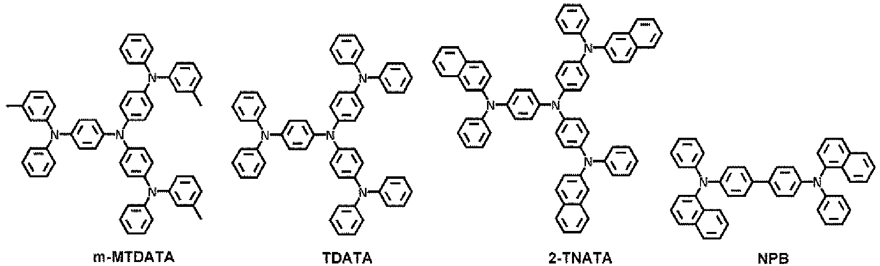

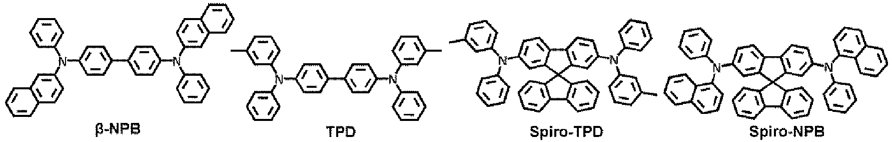

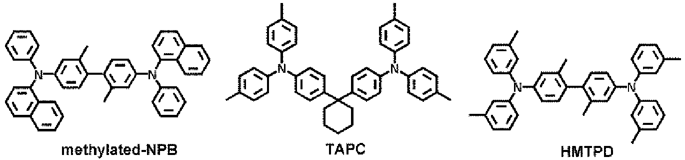

A thickness of the hole transport region may be in a range of about 50 Å to about 10,000 Å. For example, the thickness of the hole transport region may be in a range of about 100 Å to about 4,000 Å. When the hole transport region includes a hole injection layer, a hole transport layer, or any combination thereof, a thickness of the hole injection layer may be in a range of about 100 Å to about 9,000 Å, and a thickness of the hole transport layer may be in a range of about 50 Å to about 2,000 Å. For example, the thickness of the hole injection layer may be in a range of about 100 Å to about 1,000 Å. For example, the thickness of the hole transport layer may be in a range of about 100 Å to about 1,500 Å. When the thicknesses of the hole transport region, the hole injection layer, and the hole transport layer are within the ranges described above, satisfactory hole transporting characteristics may be obtained without a substantial increase in driving voltage.

The emission auxiliary layer may increase light emission efficiency by compensating for an optical resonance distance according to a wavelength of light emitted by the emission layer, and the electron blocking layer may block the leakage of electrons from the emission layer to the hole transport region. Materials that may be included in the hole transport region may be included in the emission auxiliary layer and the electron blocking layer.

### [p-dopant]

The hole transport region may further include, in addition to the materials described above, a charge-generation material for the improvement of conductive properties. The charge-generation material may be uniformly or non-uniformly dispersed in the hole transport region (for example, in the form of a single layer consisting of a charge-generation material).

The charge-generation material may be, for example, a p-dopant.

For example, a lowest unoccupied molecular orbital (LUMO) energy level of the p-dopant may be equal to or less than about -3.5 eV.

In an embodiment, the p-dopant may include a quinone derivative, a cyano group-containing compound, a compound including element EL1 and element EL2, or any combination thereof.

Examples of a quinone derivative may include TCNQ and F4-TCNQ.

Examples of a cyano group-containing compound may include HAT-CN and a compound represented by Formula 221.

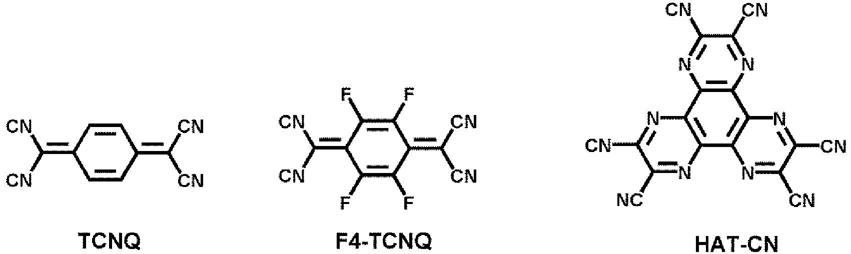

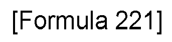

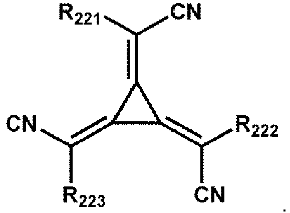

In Formula 221,
R₂₂₁ to R₂₂₃ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ, and
at least one of R₂₂₁ to R₂₂₃ may each independently be a C₃-C₆₀ carbocyclic group or a C₁-C₆₀ heterocyclic group, each substituted with: a cyano group; -F; -Cl; - Br; -I; a C₁-C₂₀ alkyl group substituted with a cyano group, -F, -Cl, -Br, -I, or any combination thereof; or any combination thereof.

In the compound including element EL1 and element EL2, element EL1 may be a metal, a metalloid, or a combination thereof, and element EL2 may be a non-metal, a metalloid, or a combination thereof.

Examples of a metal may include: an alkali metal (for example, lithium (Li), sodium (Na), potassium (K), rubidium (Rb), cesium (Cs), etc.); an alkaline earth metal (for example, beryllium (Be), magnesium (Mg), calcium (Ca), strontium (Sr), barium (Ba), etc.); a transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), etc.); a post-transition metal (for example, zinc (Zn), indium (In), tin (Sn), etc.); and a lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), etc.).

Examples of a metalloid may include silicon (Si), antimony (Sb), and tellurium (Te).

Examples of a non-metal may include oxygen (O) and a halogen (for example, F, Cl, Br, I, etc.).

Examples of a compound including element EL1 and element EL2 may include a metal oxide, a metal halide (for example, a metal fluoride, a metal chloride, a metal bromide, a metal iodide, etc.), a metalloid halide (for example, a metalloid fluoride, a metalloid chloride, a metalloid bromide, a metalloid iodide, etc.), a metal telluride, or any combination thereof.

Examples of a metal oxide may include a tungsten oxide (for example, WO, W₂O₃, WO₂, WO₃, W₂O₅, etc.), a vanadium oxide (for example, VO, V₂O₃, VO₂, V₂O₅, etc.), a molybdenum oxide (MoO, Mo₂O₃, MoO₂, MoO₃, Mo₂O₅, etc.), and a rhenium oxide (for example, ReO₃, etc.).

Examples of a metal halide may include an alkali metal halide, an alkaline earth metal halide, a transition metal halide, a post-transition metal halide, and a lanthanide metal halide.

Examples of an alkali metal halide may include LiF, NaF, KF, RbF, CsF, LiCl, NaCl, KCI, RbCl, CsCl, LiBr, NaBr, KBr, RbBr, CsBr, Lil, Nal, KI, Rbl, and Csl.

Examples of an alkaline earth metal halide may include BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, BeI₂, MgI₂, CaI₂, SrI₂, and BaI₂.

Examples of a transition metal halide may include a titanium halide (for example, TiF₄, TiCl₄, TiBr₄, TiI₄, etc.), a zirconium halide (for example, ZrF₄, ZrCl₄, ZrBr₄, ZrI₄, etc.), a hafnium halide (for example, HfF₄, HfCl₄, HfBr₄, HfI₄, etc.), a vanadium halide (for example, VF₃, VCl₃, VBr₃, VI₃, etc.), a niobium halide (for example, NbF₃, NbCl₃, NbBr₃, NbI₃, etc.), a tantalum halide (for example, TaF₃, TaCl₃, TaBr₃, TaI₃, etc.), a chromium halide (for example, CrF₃, CrCl₃, CrBr₃, CrI₃, etc.), a molybdenum halide (for example, MoF₃, MoCl₃, MoBr₃, MoI₃, etc.), a tungsten halide (for example, WF₃, WCl₃, WBr₃, WI₃, etc.), a manganese halide (for example, MnF₂, MnCl₂, MnBr₂, MnI₂, etc.), a technetium halide (for example, TcF₂, TcCl₂, TcBr₂, TcI₂, etc.), a rhenium halide (for example, ReF₂, ReCl₂, ReBr₂, ReI₂, etc.), an iron halide (for example, FeF₂, FeCl₂, FeBr₂, FeI₂, etc.), a ruthenium halide (for example, RuF₂, RuCl₂, RuBr₂, RuI₂, etc.), an osmium halide (for example, OsF₂, OsCl₂, OsBr₂, OsI₂, etc.), a cobalt halide (for example, CoF₂, CoCl₂, CoBr₂, CoI₂, etc.), a rhodium halide (for example, RhF₂, RhCl₂, RhBr₂, RhI₂, etc.), an iridium halide (for example, IrF₂, IrCl₂, IrBr₂, IrI₂, etc.), a nickel halide (for example, NiF₂, NiCl₂, NiBr₂, NiI₂, etc.), a palladium halide (for example, PdF₂, PdCl₂, PdBr₂, PdI₂, etc.), a platinum halide (for example, PtF₂, PtCl₂, PtBr₂, PtI₂, etc.), a copper halide (for example, CuF, CuCl, CuBr, Cul, etc.), a silver halide (for example, AgF, AgCl, AgBr, Agl, etc.), and a gold halide (for example, AuF, AuCl, AuBr, Aul, etc.).

Examples of a post-transition metal halide may include a zinc halide (for example, ZnF₂, ZnCl₂, ZnBr₂, ZnI₂, etc.), an indium halide (for example, InI₃, etc.), and a tin halide (for example, SnI₂, etc.).

Examples of a lanthanide metal halide may include YbF, YbF₂, YbF₃, SmF₃, YbCl, YbCl₂, YbCl₃, SmCl₃, YbBr, YbBr₂, YbBr₃, SmBr₃, Ybl, YbI₂, YbI₃, SmI₃, and the like.

Examples of a metalloid halide may include an antimony halide (for example, SbCl₅, etc.).

Examples of a metal telluride may include an alkali metal telluride (for example, Li₂Te, Na₂Te, K₂Te, Rb₂Te, Cs₂Te, etc.), an alkaline earth metal telluride (for example, BeTe, MgTe, CaTe, SrTe, BaTe, etc.), a transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, etc.), a post-transition metal telluride (for example, ZnTe, etc.), and a lanthanide metal telluride (for example, LaTe, CeTe, PrTe, NdTe, PmTe, EuTe, GdTe, TbTe, DyTe, HoTe, ErTe, TmTe, YbTe, LuTe, etc.).

### [Emission layer in interlayer 130]

When the light-emitting device 10 is a full-color light-emitting device, the emission layer may be patterned into a red emission layer, a green emission layer, and/or a blue emission layer, according to a subpixel. In an embodiment, the emission layer may have a stacked structure having two or more layers selected from a red emission layer, a green emission layer, and a blue emission layer, in which the two or more layers may contact each other or may be separated from each other, to emit white light. In an embodiment, the emission layer may include two or more materials selected from a red light-emitting material, a green light-emitting material, and a blue light-emitting material, in which the two or more materials may be mixed with each other in a single layer, to emit white light.

In an embodiment, the emission layer may include a host and a dopant (or an emitter). In an embodiment, the emission layer may further include an auxiliary dopant that promotes energy transfer to a dopant (or to an emitter), in addition to the host and the dopant (or emitter). When the emission layer includes the dopant (or emitter) and the auxiliary dopant, the dopant (or emitter) and the auxiliary dopant may be different from each other.

An amount (by weight) of the dopant (or an emitter) in the emission layer may be in a range of about 0.01 parts by weight to about 15 parts by weight, based on 100 parts by weight of the host.

In an embodiment, the emission layer may include a quantum dot.

According to an embodiment, the emission layer may include a delayed fluorescence material. The delayed fluorescence material may serve as a host or a dopant in the emission layer.

A thickness of the emission layer may be in a range of about 100 Å to about 1,000 Å. For example, the thickness of the emission layer may be in a range of about 200 Å to about 600 Å. When the thickness of the emission layer is within any of the ranges described above, excellent luminescence characteristics may be obtained without a substantial increase in driving voltage.

### [Host]

In an embodiment, the host may include a compound represented by Formula 301:

[Formula 301] [Ar₃₀₁]_{xb11}-[(L₃₀₁)_{xb1}-R₃₀₁]_{xb21}

In Formula 301,
Ar₃₀₁ and L₃₀₁ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ alkyl group that is unsubstituted or substituted with at least one R₁₀ₐ, a C₂-C₆₀ alkenyl group that is unsubstituted or substituted with at least one R₁₀ₐ, a C₂-C₆₀ alkynyl group that is unsubstituted or substituted with at least one R₁₀ₐ, a C₁-C₆₀ alkoxy group that is unsubstituted or substituted with at least one R₁₀ₐ, a C₃-C₆₀ carbocyclic group that is unsubstituted or substituted with at least one R₁₀ₐ, a C₁-C₆₀ heterocyclic group that is unsubstituted or substituted with at least one R₁₀ₐ,-Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), or - P(=O)(Q₃₀₁)(Q₃₀₂),
xb21 may be an integer from 1 to 5, and
Q₃₀₁ to Q₃₀₃ may each independently be the same as described in connection with Q₁.

In an embodiment, in Formula 301, when xb11 is 2 or more, two or more of Ar₃₀₁ may be linked to each other via a single bond.

In an embodiment, the host may include a compound represented by Formula 301-1, a compound represented by Formula 301-2, or any combination thereof:

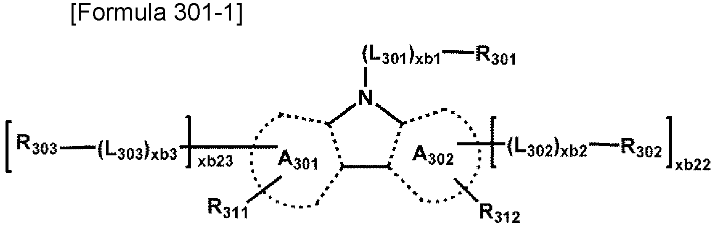

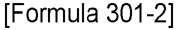

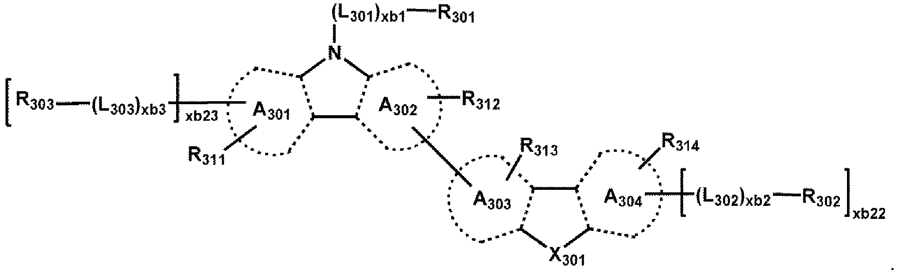

In Formulae 301-1 and 301-2,
ring A₃₀₁ to ring A₃₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
X₃₀₁ may be O, S, N-[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ are each the same as described in the specification,
L₃₀₂ to L₃₀₄ may each independently be the same as described in connection with L₃₀₁,
xb2 to xb4 may each independently be the same as described in connection with xb1, and
R₃₀₂ to R₃₀₅ and R₃₁₁ to R₃₁₄ may each independently be the same as described in connection with R₃₀₁.

In an embodiment, the host may include an alkali earth metal complex, a post-transition metal complex, or any combination thereof. In an embodiment, the host may include a Be complex (for example, Compound H55), an Mg complex, a Zn complex, or any combination thereof.

In an embodiment, the host may include: one of Compounds H1 to H129; 9,10-di(2-naphthyl)anthracene (ADN); 2-methyl-9,10-bis(naphthalen-2-yl)anthracene (MADN); 9,10-di-(2-naphthyl)-2-t-butyl-anthracene (TBADN); 4,4'-bis(N-carbazolyl)-1,1'-biphenyl (CBP); 1,3-di-9-carbazolylbenzene (mCP); 1,3,5-tri(carbazol-9-yl)benzene (TCP); or any combination thereof:

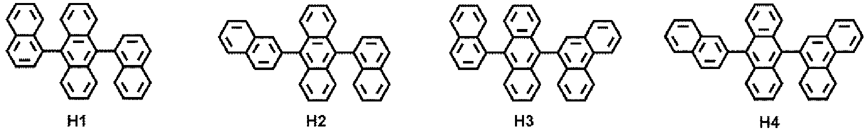

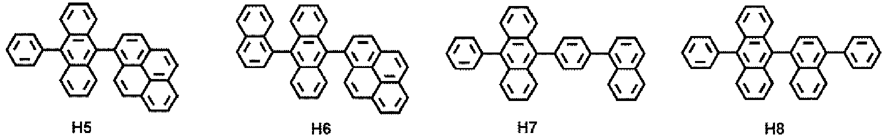

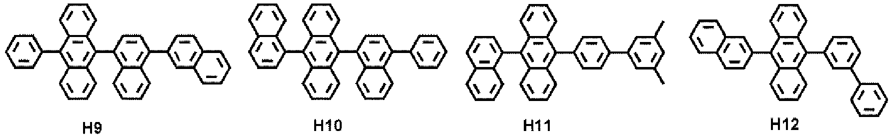

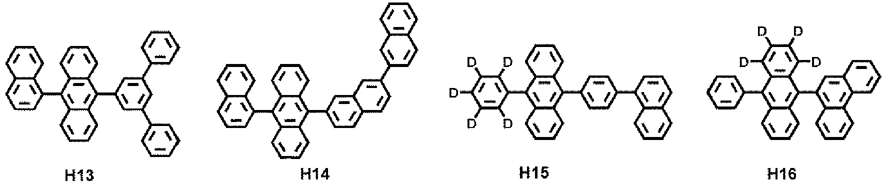

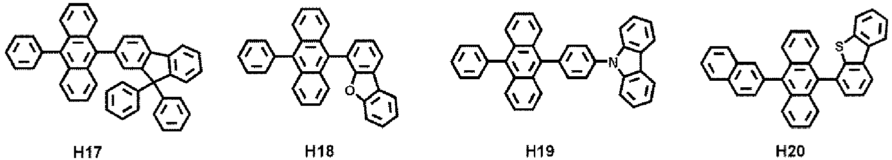

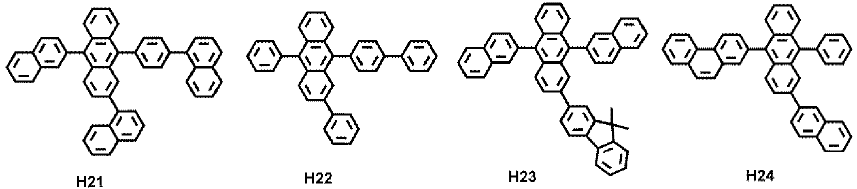

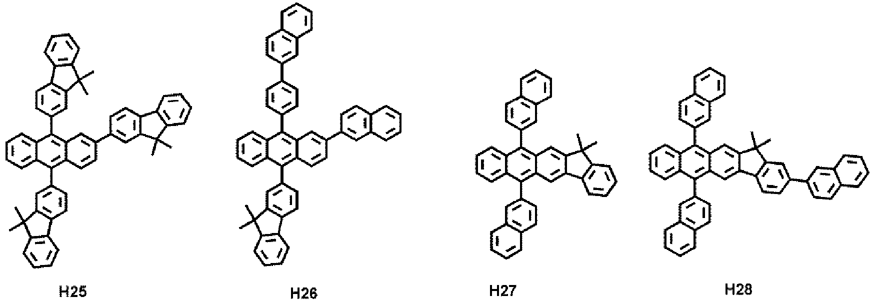

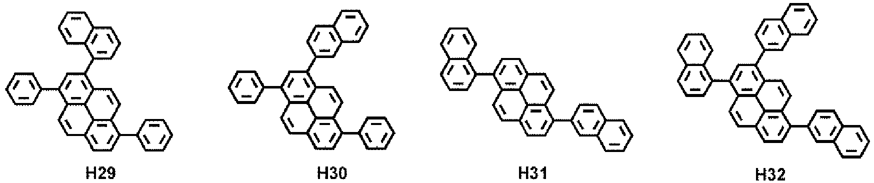

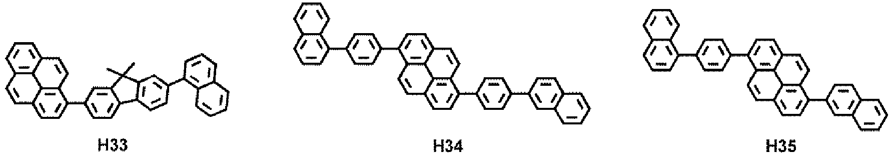

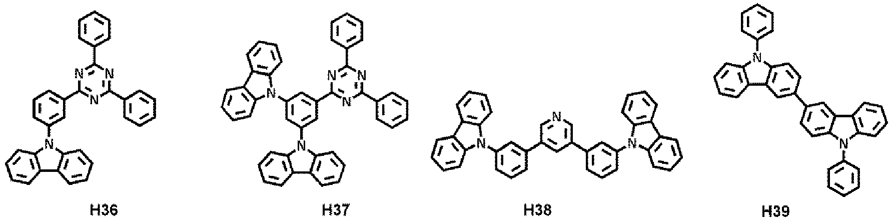

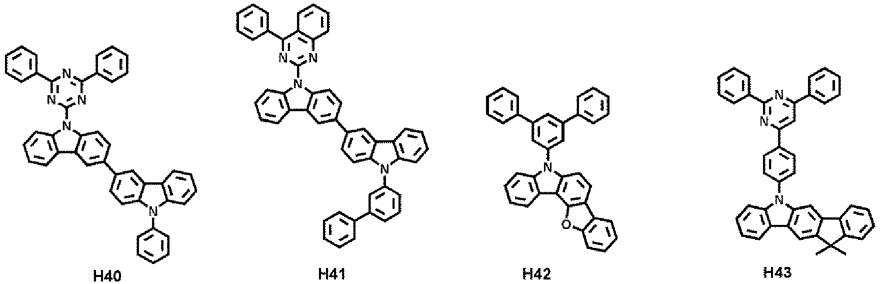

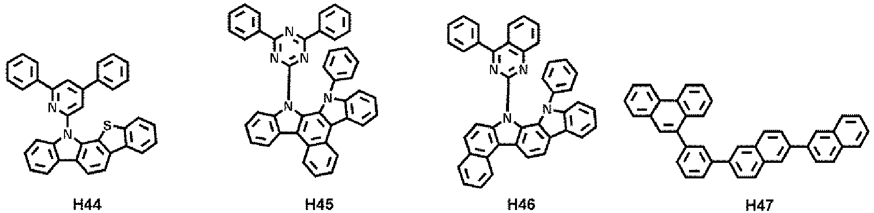

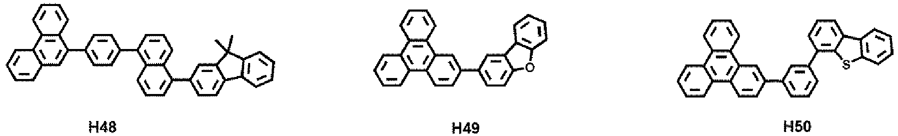

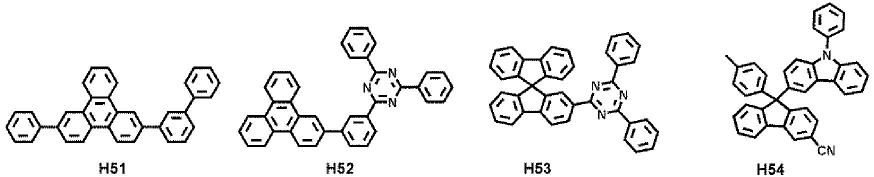

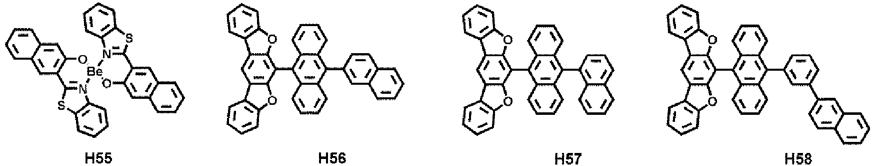

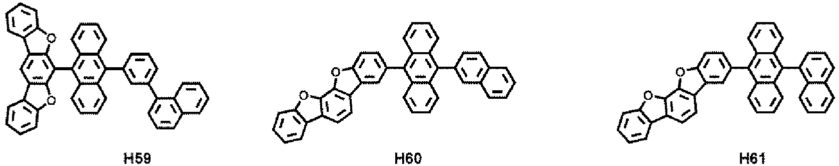

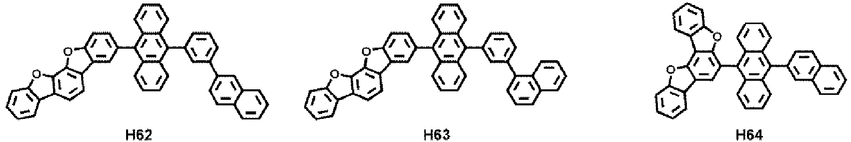

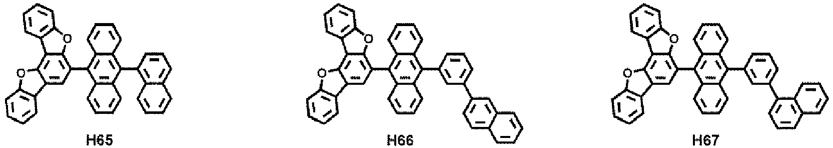

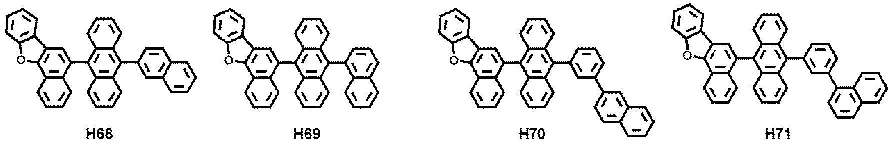

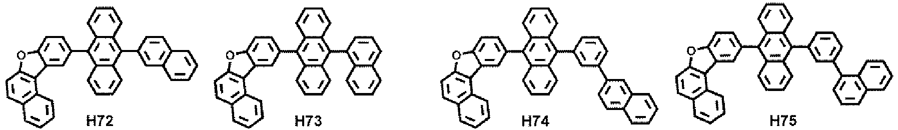

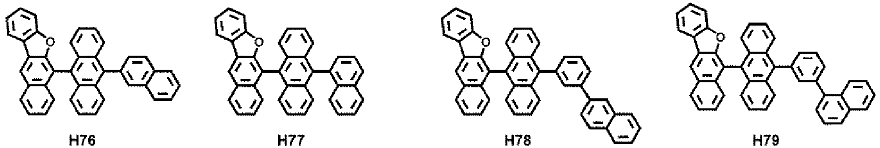

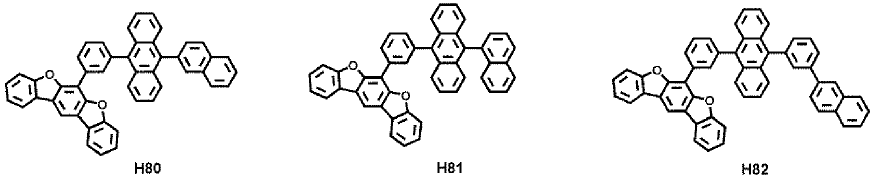

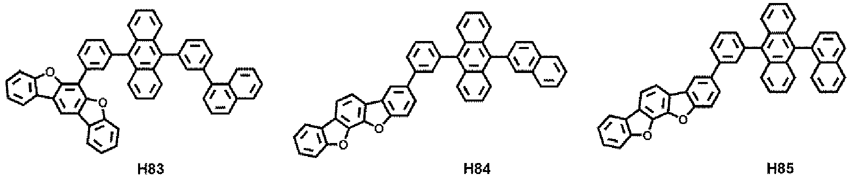

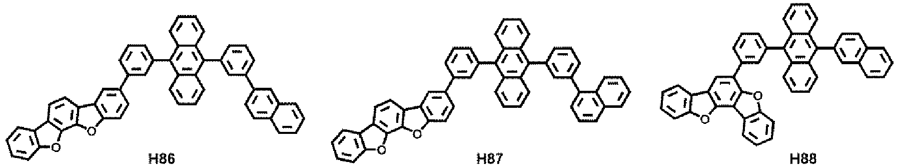

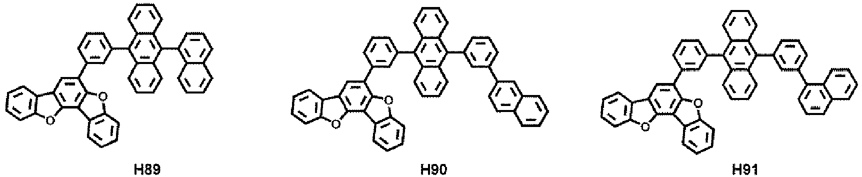

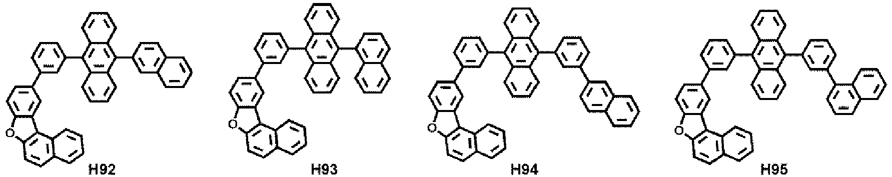

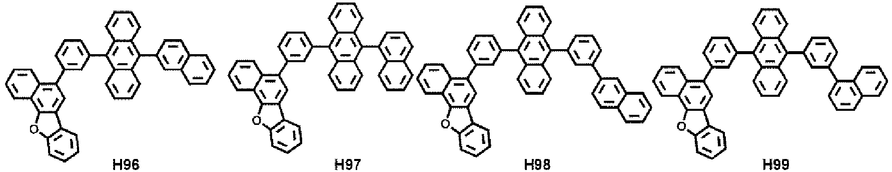

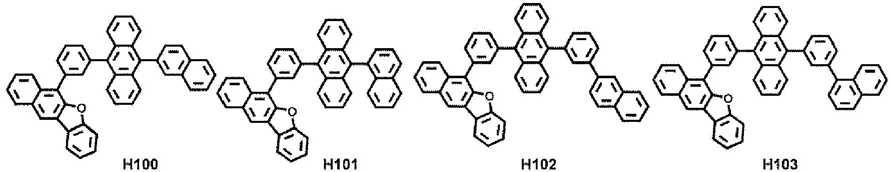

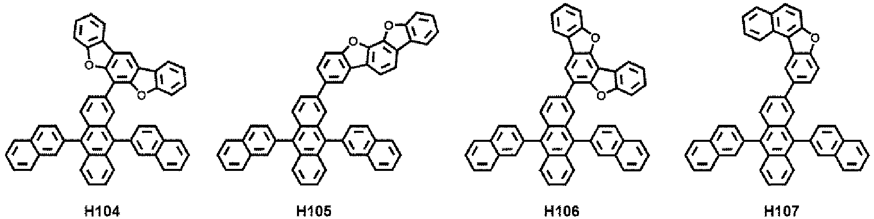

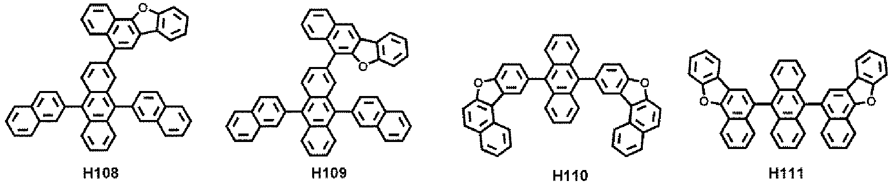

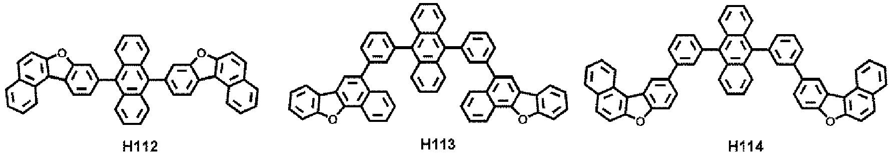

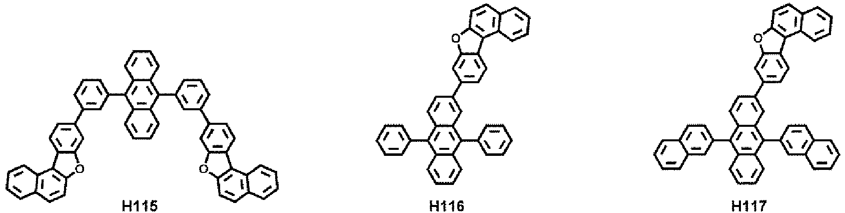

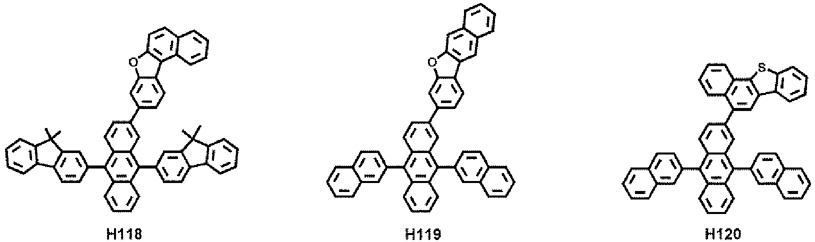

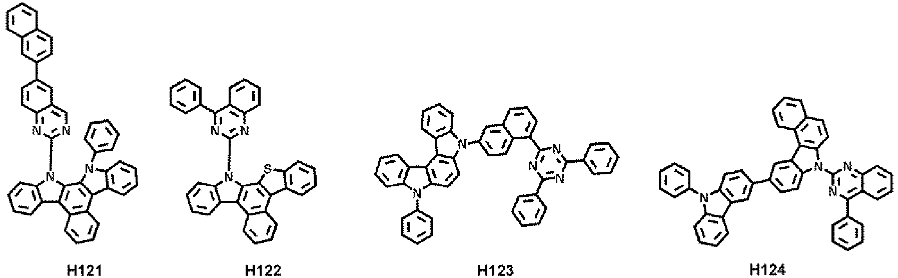

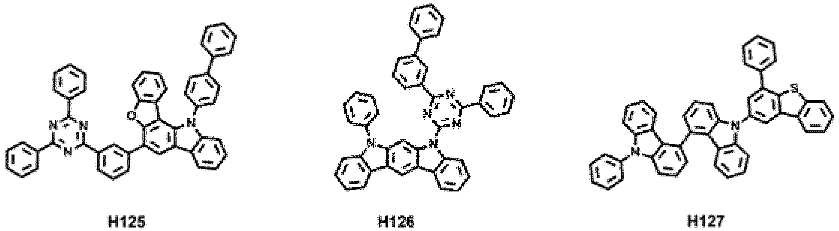

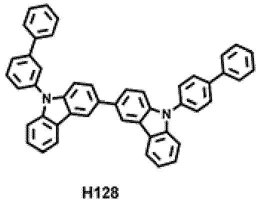

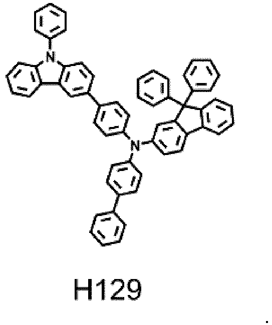

In an embodiment, the host may include a silicon-containing compound, a phosphine oxide-containing compound, or any combination thereof.

The host may have various modifications. For example, the host may include only one kind of compound, or may include two or more kinds of different compounds.

### [Phosphorescent dopant]

In embodiments, the emission layer may include a phosphorescent dopant.

The phosphorescent dopant may include at least one transition metal as a central metal. Therefore, the phosphorescent dopant may correspond to the third compound as described herein.

The phosphorescent dopant may include a monodentate ligand, a bidentate ligand, a tridentate ligand, a tetradentate ligand, a pentadentate ligand, a hexadentate ligand, or any combination thereof.

The phosphorescent dopant may be electrically neutral.

In an embodiment, the phosphorescent dopant may include an organometallic compound represented by Formula 401:

[Formula 401] M(L₄₀₁)_{xc1}(L402)xc2

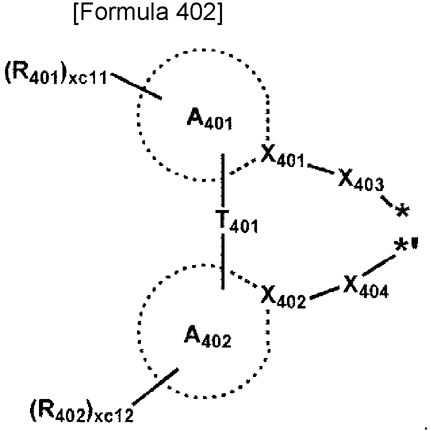

In Formulae 401 and 402,
M may be a transition metal (e.g., Ir, Pt, Pd, Os, Ti, Au, Hf, Eu, Tb, Rh, Re, or Tm),
L₄₀₁ may be a ligand represented by Formula 402, and xc1 may be 1, 2, or 3, wherein when xc1 is 2 or more, two or more of L₄₀₁ may be identical to or different from each other,
L₄₀₂ may be an organic ligand, and xc2 may be 0, 1, 2, 3, or 4, wherein when xc2 is 2 or more, two or more of L₄₀₂ may be identical to or different from each other,
X₄₀₁ and X₄₀₂ may each independently be nitrogen or carbon,
ring A₄₀₁ and ring A₄₀₂ may each independently be a C₃-C₆₀ carbocyclic group or a C₁-C₆₀ heterocyclic group,
T₄₀₁ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*',
X₄₀₃ and X₄₀₄ may each independently be a chemical bond (for example, a covalent bond or a coordinate bond), O, S, N(Q₄₁₃), B(Q₄₁₃), P(Q₄₁₃), C(Q₄₁₃)(Q₄₁₄), or Si(Q₄₁₃)(Q₄₁4),
Q₄₁₁ to Q₄₁₄ may each independently be the same as described in connection with Q₁,
R₄₀₁ and R₄₀₂ may each independently be hydrogen, deuterium, -F, -Cl, -Br, -I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), or -P(=O)(Q₄₀₁)(Q₄₀₂),
Q₄₀₁ to Q₄₀₃ may each independently be the same as described in connection with Q₁,
xc11 and xc12 may each independently be an integer from 0 to 10, and
* and *' in Formula 402 each indicate a binding site to M in Formula 401.

In an embodiment, in Formula 402, X₄₀₁ may be nitrogen and X₄₀₂ may be carbon, or X₄₀₁ and X₄₀₂ may each be nitrogen.

According to an embodiment, in Formula 401, when xc1 is 2 or more, two of ring A₄₀₁ among two or more of L₄₀₁ may be optionally linked together via T₄₀₂, which is a linking group, and two of ring A₄₀₂ may be optionally linked together via T₄₀₃, which is a linking group (see Compounds PD1 to PD4 and PD7). T₄₀₂ and T₄₀₃ may each independently be the same as described in connection with T₄₀₁.

In Formula 401, L₄₀₂ may be an organic ligand. In an embodiment, L₄₀₂ may include a halogen group, a diketone group (for example, an acetylacetonate group), a carboxylic acid group (for example, a picolinate group), -C(=O), an isonitrile group, a - CN group, a phosphorus group (for example, a phosphine group, a phosphite group, etc.), or any combination thereof.

In an embodiment, the phosphorescent dopant may include, for example, one of Compounds PD1 to PD39, or any combination thereof:

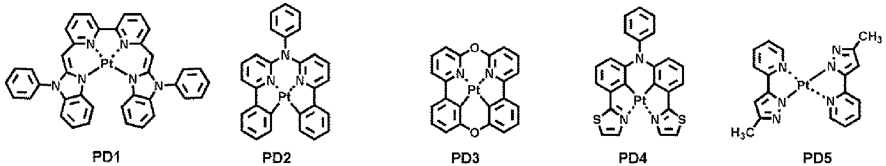

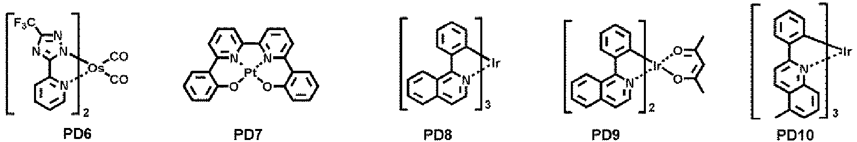

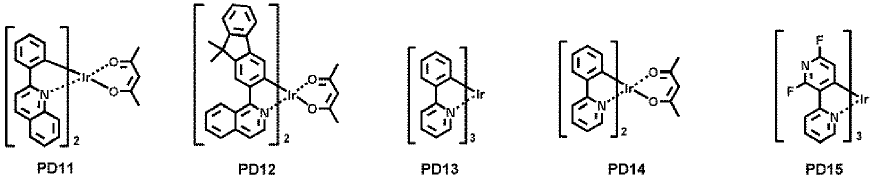

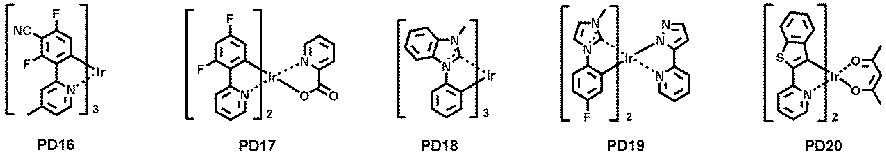

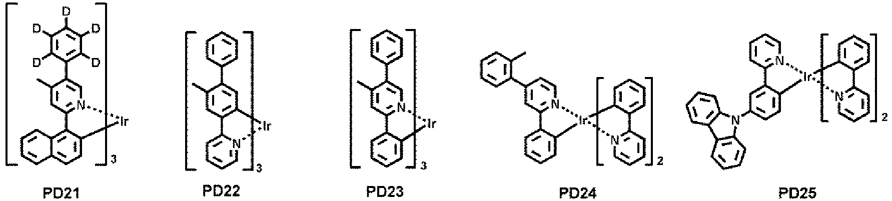

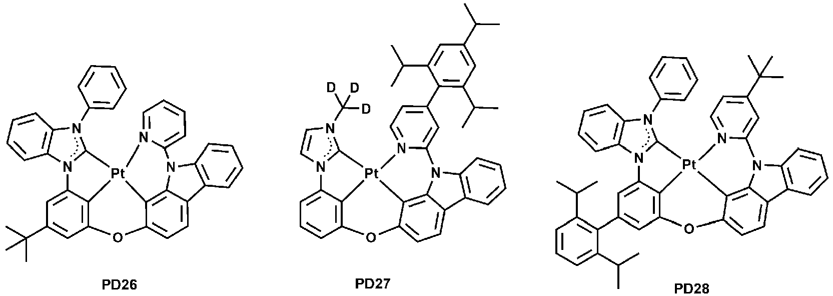

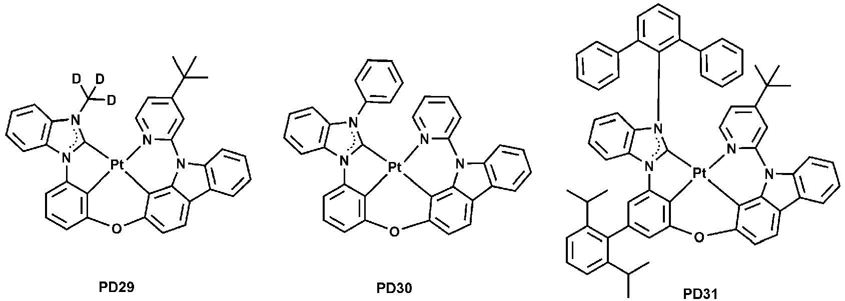

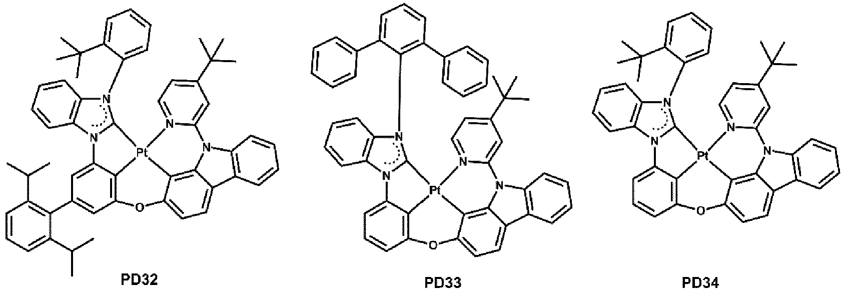

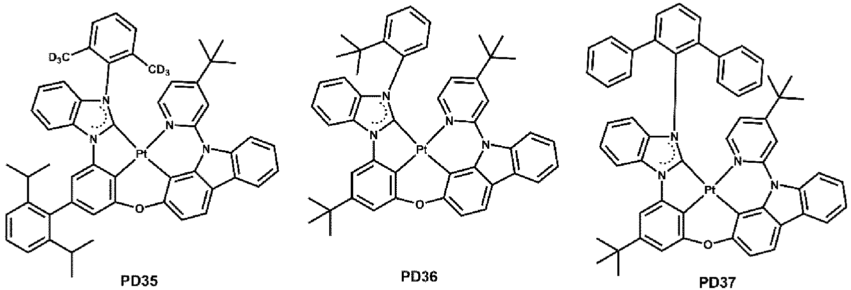

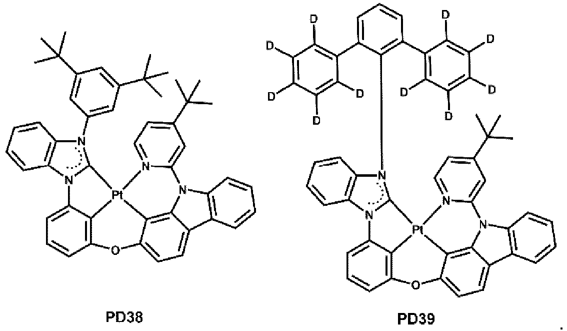

### [Fluorescent dopant]

In embodiments, the emission layer may include a fluorescent dopant and/or an auxiliary dopant.

In an embodiment, the fluorescent dopant and/or the auxiliary dopant may each independently include a compound represented by Formula 501:

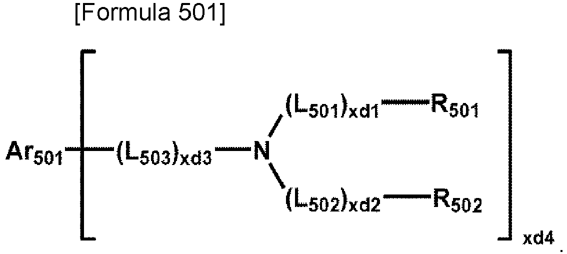

In Formula 501,
Ar₅₀₁, L₅₀₁ to L₅₀₃, R₅₀₁, and R₅₀₂ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xd1 to xd3 may each independently be 0, 1, 2, or 3, and
xd4 may be 1, 2, 3, 4, 5, or 6.

In an embodiment, in Formula 501, Ar₅₀₁ may be a condensed cyclic group (for example, an anthracene group, a chrysene group, a pyrene group, etc.) in which three or more monocyclic groups are condensed together.

In an embodiment, in Formula 501, xd4 may be 2.

In an embodiment, the fluorescent dopant and the auxiliary dopant may each independently include: one of Compounds FD1 to FD37; DPVBi; DPAVB; or any combination thereof:

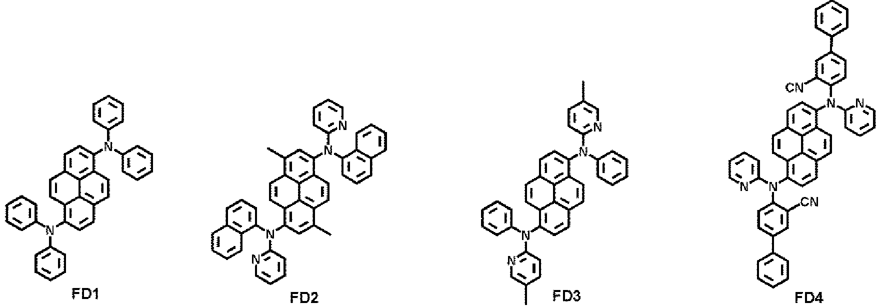

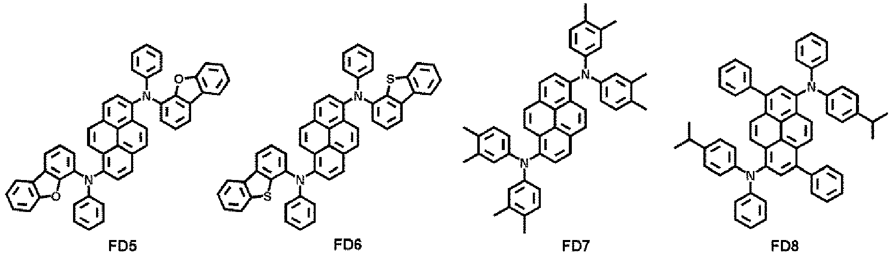

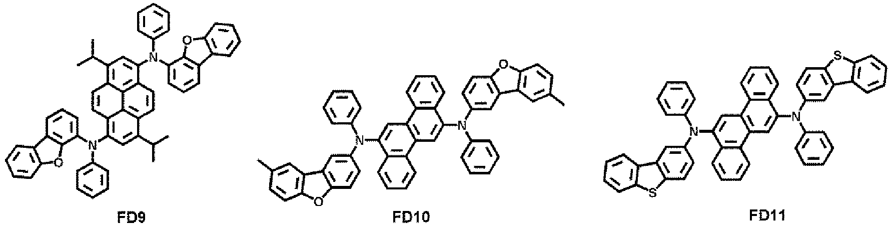

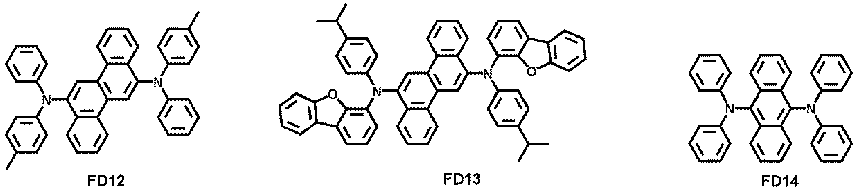

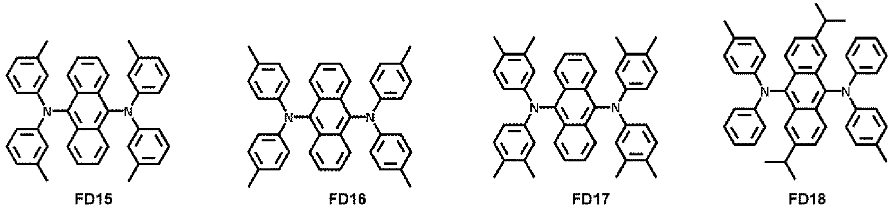

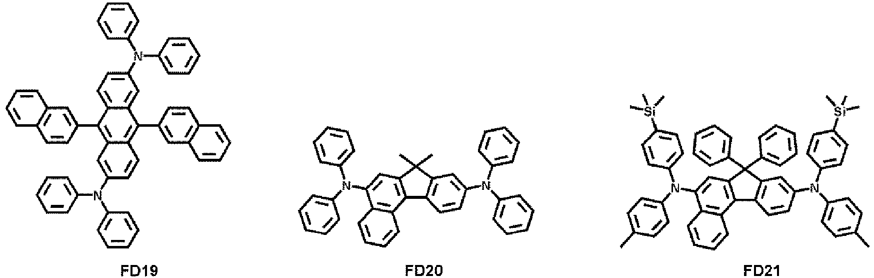

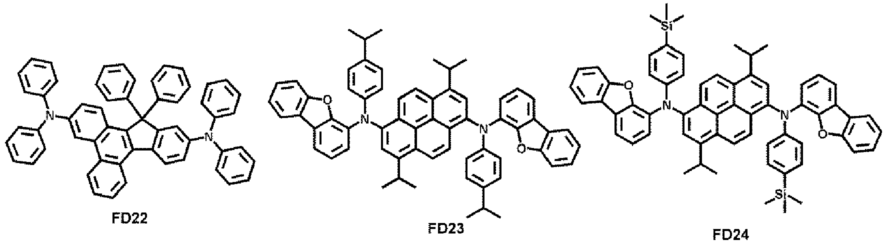

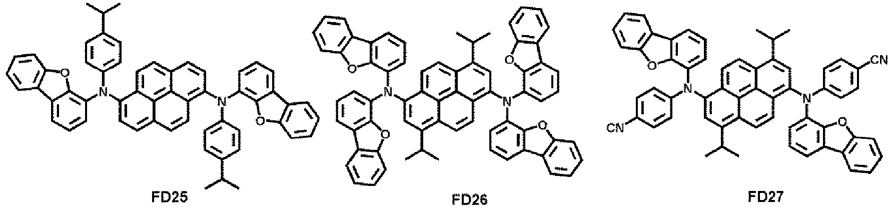

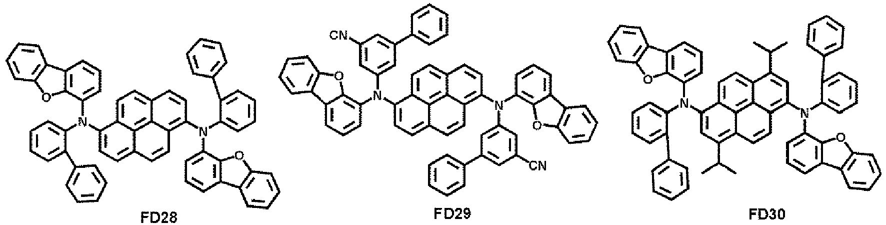

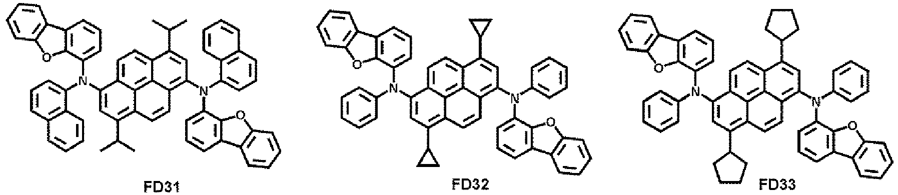

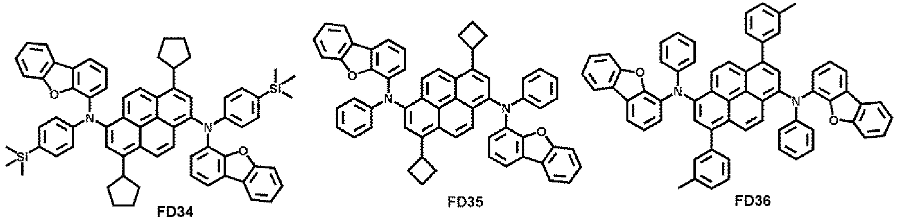

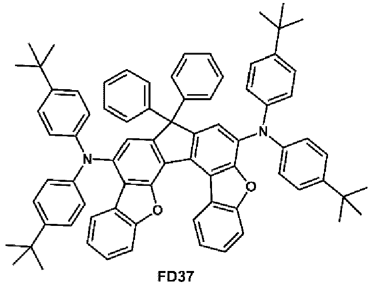

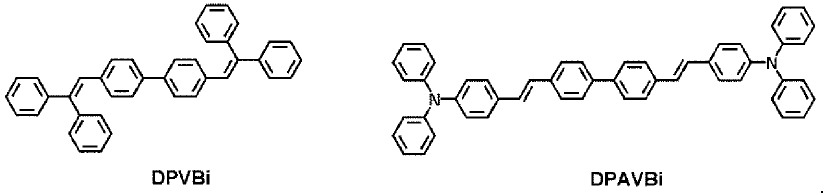

### [Delayed fluorescence material]

In embodiments, the emission layer may include a delayed fluorescence material.

In the specification, a delayed fluorescence material may be selected from any compound that is capable of emitting delayed fluorescence, based on a delayed fluorescence emission mechanism.

The delayed fluorescence material included in the emission layer may serve as a host or as a dopant, depending on the types of other materials included in the emission layer.

In an embodiment, a difference between a triplet energy level (eV) of the delayed fluorescence material and a singlet energy level (eV) of the delayed fluorescence material may be at least about 0 eV and not more than about 0.5 eV. When the difference between the triplet energy level (eV) of the delayed fluorescence material and the singlet energy level (eV) of the delayed fluorescence material satisfies the range described above, up-conversion from a triplet state to a singlet state of the delayed fluorescence materials may effectively occur, and thus, the luminescence efficiency of the light-emitting device 10 may be improved.

In an embodiment, the delayed fluorescence material may include: a material that includes at least one electron donor (for example, a π electron-rich C₃-C₆₀ cyclic group such as a carbazole group) and at least one electron acceptor (for example, a sulfoxide group, a cyano group, a π electron-deficient nitrogen-containing C₁-C₆₀ cyclic group, and the like); or a material that includes a C₈-C₆₀ polycyclic group including at least two cyclic groups that are condensed with each other while sharing boron (B).

In an embodiment, the delayed fluorescence material may include at least one of Compounds DF1 to DF14:

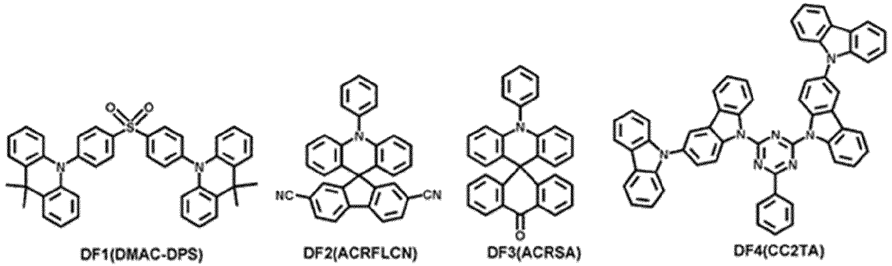

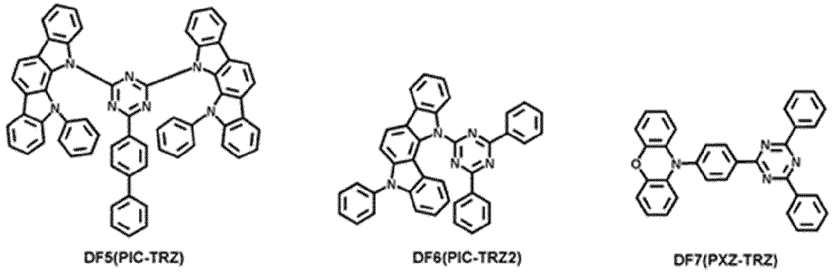

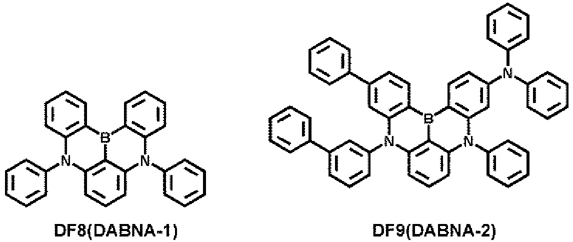

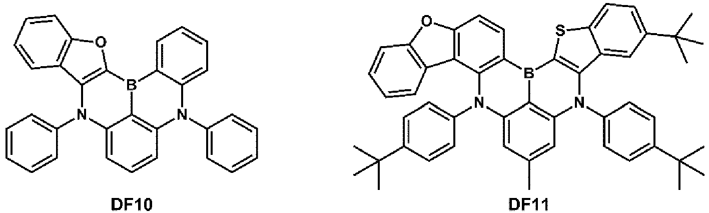

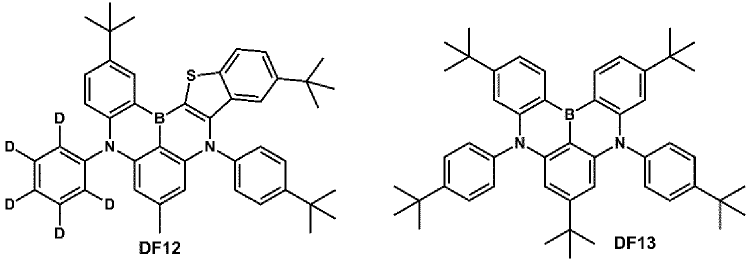

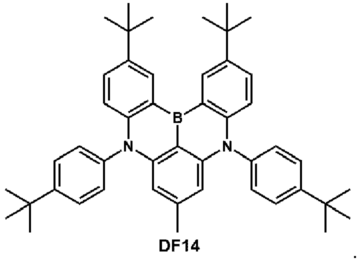

### [Quantum dot]

In embodiments, the emission layer may include a quantum dot.

In the specification, a quantum dot may be a crystal of a semiconductor compound, and may include any material capable of emitting light of various emission wavelengths according to a size of the crystal. In an embodiment, quantum dots may emit light of various emission wavelengths by adjusting an elemental ratio in a quantum dot compound.

A diameter of the quantum dot may be, for example, in a range of about 1 nm to about 10 nm.

The quantum dot may be synthesized by a wet chemical process, a metal organic chemical vapor deposition process, a molecular beam epitaxy process, or any process similar thereto.

The wet chemical process is a method that includes mixing a precursor material with an organic solvent and growing a quantum dot particle crystal. When the crystal grows, the organic solvent naturally serves as a dispersant that is coordinated on the surface of the quantum dot crystal and controls the growth of the crystal. Thus, the growth of quantum dot particles can be controlled through a process which costs less, and may be more readily performed than vapor deposition methods, such as metal organic chemical vapor deposition (MOCVD) or molecular beam epitaxy (MBE).

A quantum dot may include a Group II-VI semiconductor compound, a Group III-V semiconductor compound, a Group III-VI semiconductor compound, a Group I-III-VI semiconductor compound, a Group IV-VI semiconductor compound, a Group IV element or compound, or a combination thereof.

Examples of a Group II-VI semiconductor compound may include: a binary compound, such as CdS, CdSe, CdTe, ZnS, ZnSe, ZnTe, ZnO, HgS, HgSe, HgTe, MgSe, or MgS; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, or MgZnS; a quaternary compound, such as CdZnSeS, CdZnSeTe, CdZnSTe, CdHgSeS, CdHgSeTe, CdHgSTe, HgZnSeS, HgZnSeTe, or HgZnSTe; and any combination thereof.

Examples of a Group III-V semiconductor compound may include: a binary compound, such as GaN, GaP, GaAs, GaSb, AIN, AlP, AlAs, AlSb, InN, InP, InAs, InSb, or the like; a ternary compound, such as GaNP, GaNAs, GaNSb, GaPAs, GaPSb, AINP, AlNAs, AlNSb, AlPAs, AlPSb, InGaP, InNP, InAlP, InNAs, InNSb, InPAs, InPSb, or the like; a quaternary compound, such as GaAlNP, GaAINAs, GaAINSb, GaAlPAs, GaAlPSb, GalnNP, GalnNAs, GaInNSb, GalnPAs, GalnPSb, InAlNP, InAlNAs, InAlNSb, InAlPAs, InAlPSb, or the like; and any combination thereof. In an embodiment, a Group III-V semiconductor compound may further include a Group II element. Examples of a Group III-V semiconductor compound further including a Group II element may include InZnP, InGaZnP, InAlZnP, etc.

Examples of a Group III-VI semiconductor compound may include: a binary compound, such as GaS, GaSe, Ga₂Se₃, GaTe, InS, InSe, In₂S₃, In₂Se₃, or InTe; a ternary compound, such as InGaS₃, or InGaSe₃; and any combination thereof.

Examples of a Group I-III-VI semiconductor compound may include: a ternary compound, such as AgInS, AgInS₂, AgInSe₂, AgGaS, AgGaS₂, AgGaSe₂, CulnS, CuInS₂, CuInSe₂, CuGaS₂, CuGaSe₂, CuGaO₂, AgGaO₂, AgAlO₂, or the like; a quaternary compound, such as AgInGaS₂, AgInGaSe₂, or the like; and any combination thereof.

Examples of a Group IV-VI semiconductor compound may include: a binary compound, such as SnS, SnSe, SnTe, PbS, PbSe, or PbTe; a ternary compound, such as SnSeS, SnSeTe, SnSTe, PbSeS, PbSeTe, PbSTe, SnPbS, SnPbSe, or SnPbTe; a quaternary compound, such as SnPbSSe, SnPbSeTe, or SnPbSTe; and any combination thereof.

Examples of a Group IV element or compound may include: a single element material, such as Si or Ge; a binary compound, such as SiC or SiGe; and any combination thereof.

Each element included in a compound, such as a binary compound, a ternary compound, or a quaternary compound, may be present in a particle at a uniform concentration or at a non-uniform concentration. A formula of a quantum dot compound may refer to the types of elements included in each compound, but an elemental ratio of a compound may vary. For example, AgInGaS₂ may indicate AgInₓGa₁₋ₓS₂ (where x is a real number satisfying 0<x<1).

In embodiments, a quantum dot may have a single structure in which the concentration of each element in the quantum dot is uniform, or a quantum dot may have a core-shell structure. For example, when a quantum dot has a core-shell structure, a material included in the core and a material included in the shell may be different from each other.

The shell of a quantum dot may serve as a protective layer that prevents chemical degeneration of the core to maintain semiconductor characteristics, and/or may serve as a charging layer that imparts electrophoretic characteristics to the quantum dot. The shell may be single-layered or multilayered. An interface between the core and the shell may have a concentration gradient in which the concentration of an element that is present in the shell decreases toward the core.

Examples of a shell of a quantum dot may include a metal oxide, a metalloid oxide, a non-metal oxide, a semiconductor compound, and a combination thereof. Examples of a metal oxide, a metalloid oxide, or a non-metal oxide may include: a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, or NiO; a ternary compound, such as MgAl₂O₄, CoFe₂O₄, NiFe₂O₄, or CoMn₂O₄; and a combination thereof. Examples of a semiconductor compound may include, as described herein: a Group II-VI semiconductor compound; a Group III-V semiconductor compound; a Group III-VI semiconductor compound; a Group I-III-VI semiconductor compound; a Group IV-VI semiconductor compound; and a combination thereof. For example, the semiconductor compound may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, or a combination thereof.

A full width at half maximum (FWHM) of an emission wavelength spectrum of a quantum dot may be equal to or less than about 45 nm. For example, an FWHM of an emission wavelength spectrum of a quantum dot may be equal to or less than about 40 nm. For example, an FWHM of an emission wavelength spectrum of a quantum dot may be equal to or less than about 30 nm. Within any of these ranges, color purity or color reproducibility may be increased. Light emitted through a quantum dot may be emitted in all directions, so that a wide viewing angle may be improved.

In embodiments, a quantum dot may be in the form of a spherical particle, a pyramidal particle, a multi-arm particle, a cubic nanoparticle, a nanotube particle, a nanowire particle, a nanofiber particle, or a nanoplate particle.

Since an energy band gap may be adjusted by controlling a size of the quantum dot, light having various wavelength bands may be obtained from a quantum dot emission layer. Accordingly, by using quantum dots of different sizes, a light-emitting device that emits light of various wavelengths may be implemented. In embodiments, the size of quantum dots or an elemental ratio of quantum dot compounds may be selected so that red light, green light, and/or blue light can be emitted. In an embodiment, quantum dots may be configured to emit white light by a combination of light of various colors.

### [Electron transport region in interlayer 130]

The electron transport region may have a structure consisting of a layer consisting of a single material, a structure consisting of a layer including different materials, or a structure including multiple layers including different materials.

The electron transport region may include a buffer layer, a hole blocking layer, an electron control layer, an electron transport layer, an electron injection layer, or any combination thereof.

In embodiments, the electron transport region may have a multilayered structure, for example, an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein the layers of each structure may be stacked from an emission layer in its respective stated order, but the structure of the electron transport region is not limited thereto.

The electron transport region (e.g., a buffer layer, a hole blocking layer, an electron control layer, or an electron transport layer in the electron transport region) may include a metal-free compound including at least one π electron-deficient nitrogen-containing C₁-C₆₀ heterocyclic group.

In an embodiment, the electron transport region may include a compound represented by Formula 601.

[Formula 601] [Ar₆₀₁]ₓₑ₁₁-[(L₆₀₁)ₓₑ₁-R₆₀₁]ₓₑ₂₁

In Formula 601,
Ar₆₀₁ and L₆₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group that is unsubstituted or substituted with at least one R₁₀ₐ, a C₁-C₆₀ heterocyclic group that is unsubstituted or substituted with at least one R₁₀ₐ, -Si(Q₆₀₁)(Q₆₀₂)(Q₆₀₃), -C(=O)(Q₆₀₁), -S(=O)₂(Q₆₀₁), or - P(=O)(Q₆₀₁)(Q₆₀₂),
Q₆₀₁ to Q₆₀₃ may each independently be the same as described in connection with Q₁,
xe21 may be 1, 2, 3, 4, or 5, and
at least one of Ar₆₀₁, L₆₀₁, and R₆₀₁ may each independently be a π electron-deficient nitrogen-containing C₁-C₆₀ cyclic group that is unsubstituted or substituted with at least one R₁₀ₐ.

In an embodiment, in Formula 601, when xe11 is 2 or more, two or more of Ar₆₀₁ may be linked together via a single bond.

In an embodiment, in Formula 601, Ar₆₀₁ may be a substituted or unsubstituted anthracene group.

In an embodiment, the electron transport region may include a compound represented by Formula 601-1:

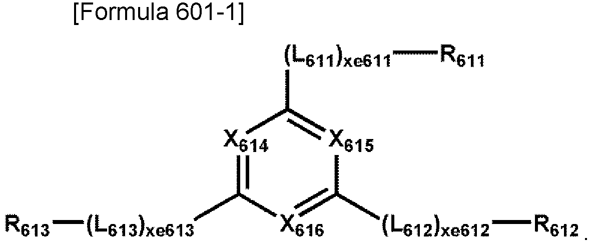

In Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may each be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group that is unsubstituted or substituted with at least one R₁₀ₐ, or a C₁-C₆₀ heterocyclic group that is unsubstituted or substituted with at least one R₁₀ₐ.

In an embodiment, in Formulae 601 and 601-1, xe1 and xe611 to xe613 may each independently be 0, 1, or 2.

In embodiments, the electron transport region may include one of Compounds ET1 to ET45, 2,9-dimethyl-4,7-diphenyl-1,10-phenanthroline (BCP), 4,7-diphenyl-1,10-phenanthroline (Bphen), Alq₃, BAlq, TAZ, NTAZ, or any combination thereof:

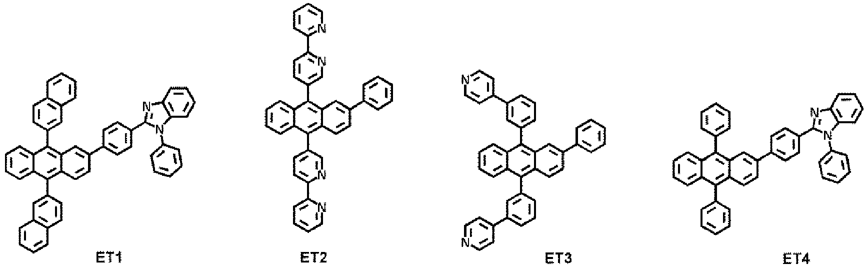

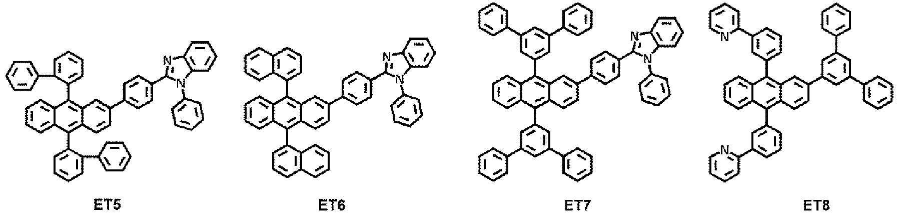

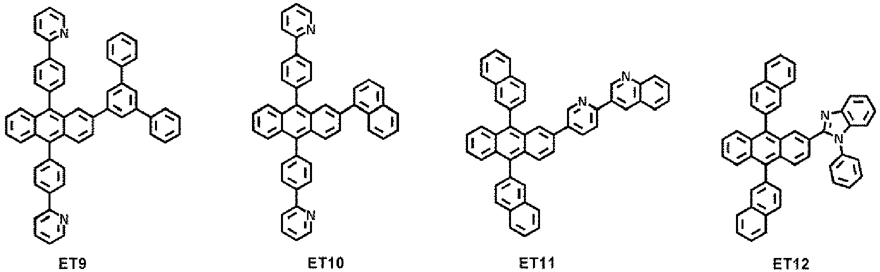

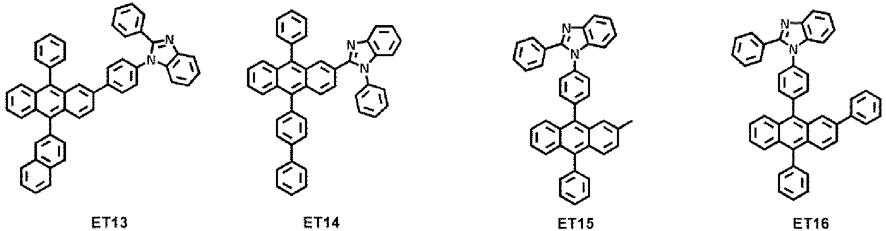

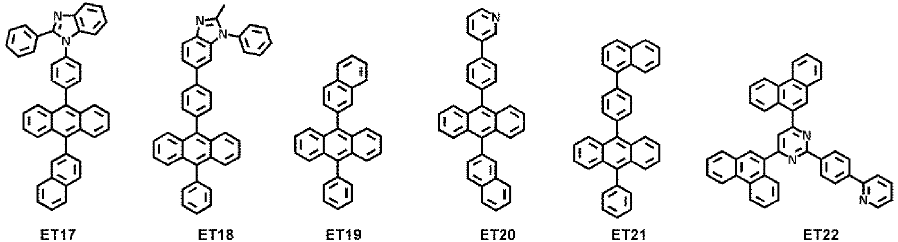

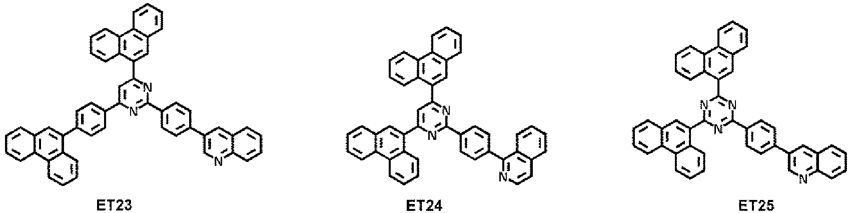

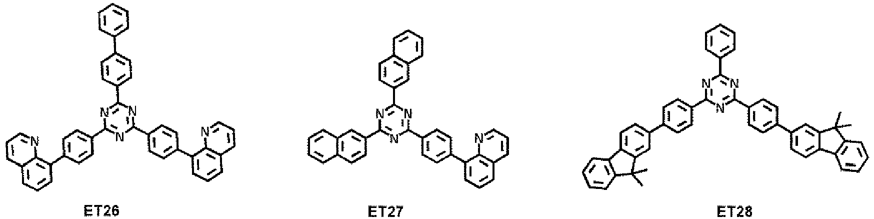

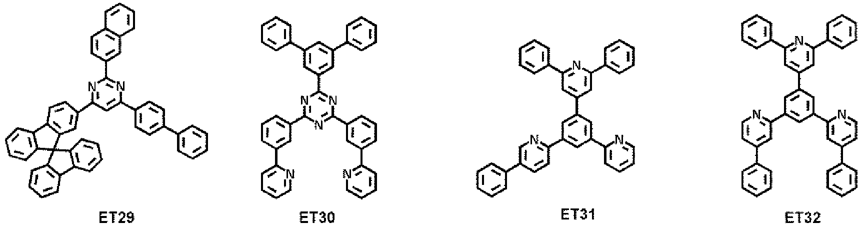

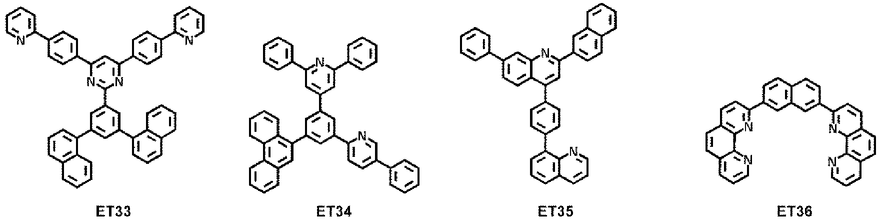

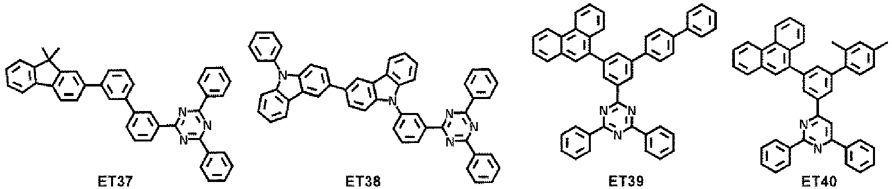

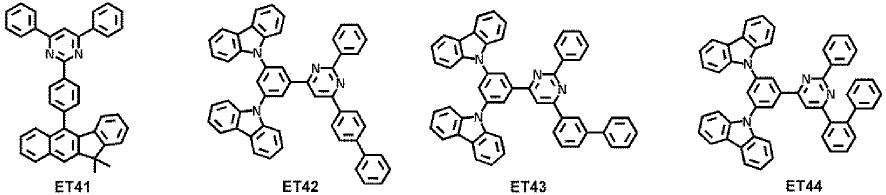

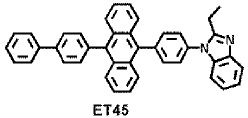

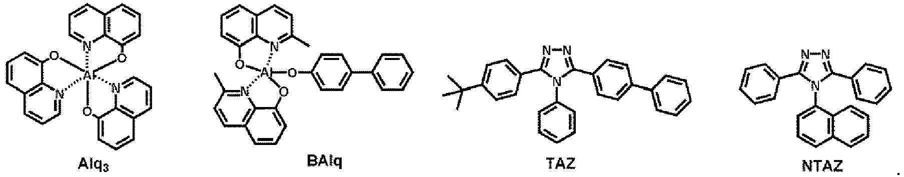

A thickness of the electron transport region may be in a range of about 100 Å to about 5,000 Å. For example, the thickness of the electron transport region may be in a range of about 160 Å to about 4,000 Å. When the electron transport region includes a buffer layer, a hole blocking layer, an electron control layer, an electron transport layer, or any combination thereof, a thickness of the buffer layer, the hole blocking layer, or the electron control layer may each independently be in a range of about 20 Å to about 1,000 Å, and a thickness of the electron transport layer may be in a range of about 100 Å to about 1,000 Å. For example, the thickness of the buffer layer, the hole blocking layer, or the electron control layer may each independently be in a range of about 30 Å to about 300 Å. For example, the thickness of the electron transport layer may be in a range of about 150 Å to about 500 Å. When the thicknesses of the buffer layer, the hole blocking layer, the electron control layer, the electron transport layer, and/or the electron transport region are within the ranges described above, satisfactory electron transporting characteristics may be obtained without a substantial increase in driving voltage.

The electron transport region (for example, an electron transport layer in the electron transport region) may further include, in addition to the materials described above, a metal-containing material.

The metal-containing material may include an alkali metal complex, an alkaline earth metal complex, or any combination thereof. A metal ion of the alkali metal complex may be a Li ion, a Na ion, a K ion, a Rb ion, or a Cs ion, and a metal ion of the alkaline earth metal complex may be a Be ion, a Mg ion, a Ca ion, a Sr ion, or a Ba ion.

A ligand coordinated with a metal ion of an alkali metal complex or with a metal ion of an alkaline earth-metal complex may each independently include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

In an embodiment, the metal-containing material may include a Li complex. The Li complex may include, for example, Compound ET-D1 (LiQ) or Compound ET-D2:

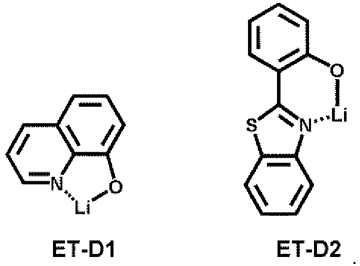

The electron transport region may include an electron injection layer that facilitates the injection of electrons from the second electrode 150. The electron injection layer may directly contact the second electrode 150.

The electron injection layer may have a structure consisting of a layer consisting of a single material, a structure consisting of a layer including different materials, or a structure including multiple layers including different materials.

The electron injection layer may include an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may include oxides, halides (for example, fluorides, chlorides, bromides, iodides, etc.), or tellurides of the alkali metal, the alkaline earth metal, and the rare earth metal, or any combination thereof.

The alkali metal-containing compound may include: an alkali metal oxide, such as Li₂O, Cs₂O, K₂O, etc.; an alkali metal halide, such as LiF, NaF, CsF, KF, Lil, Nal, Csl, KI, etc.; or any combination thereof. The alkaline earth metal-containing compound may include an alkaline earth metal oxide, such as BaO, SrO, CaO, BaₓSr₁₋ₓO (wherein x is a real number satisfying 0<x<1), or BaₓCa₁₋ₓO (wherein x is a real number satisfying 0<x<1). The rare earth metal-containing compound may include YbF₃, ScF₃, Sc₂O₃, Y₂O₃, Ce₂O₃, GdF₃, TbF₃, YbI₃, ScI₃, TbI₃, or any combination thereof. In an embodiment, the rare earth metal-containing compound may include a lanthanide metal telluride. Examples of a lanthanide metal telluride may include LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, and Lu₂Te₃.

The alkali metal complex, the alkaline earth-metal complex, and the rare earth metal complex may include: an alkali metal ion, an alkaline earth metal ion, or a rare earth metal ion; and a ligand bonded to the metal ion (for example, a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenyl benzimidazole, a hydroxyphenylbenzothiazole, a bipyridine, a phenanthroline, a cyclopentadiene, or any combination thereof).

In an embodiment, the electron injection layer may consist of an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. According to an embodiment, the electron injection layer may further include an organic material (for example, a compound represented by Formula 601).

In an embodiment, the electron injection layer may consist of an alkali metal-containing compound (for example, alkali metal halide); or the electron injection layer may consist of an alkali metal-containing compound (for example, alkali metal halide), and an alkali metal, an alkaline earth metal, a rare earth metal, or any combination thereof. According to an embodiment, the electron injection layer may be a Kl:Yb co-deposited layer, an Rbl:Yb co-deposited layer, a LiF:Yb co-deposited layer, or the like.

When the electron injection layer further includes an organic material, an alkali metal, an alkaline earth metal, a rare earth metal, an alkali metal-containing compound, an alkaline earth metal-containing compound, a rare earth metal-containing compound, an alkali metal complex, an alkaline earth-metal complex, a rare earth metal complex, or any combination thereof may be uniformly or non-uniformly dispersed in a matrix including the organic material.

A thickness of the electron injection layer may be in a range of about 1 Å to about 100 Å. For example, the thickness of the electron injection layer may be in a range of about 3 Å to about 90 Å. When the thickness of the electron injection layer is within any of the ranges described above, satisfactory electron injection characteristics may be obtained without a substantial increase in driving voltage.

### [Second electrode 150]

The second electrode 150 is arranged on the interlayer 130. The second electrode 150 may be a cathode, which is an electron injection electrode. When the second electrode 150 is a cathode, the second electrode 150 may include a material having a low-work function, such as a metal, an alloy, an electrically conductive compound, or any combination thereof.

The second electrode 150 may include lithium (Li), silver (Ag), magnesium (Mg), aluminium (Al), aluminium-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a transflective electrode, or a reflective electrode.

The second electrode 150 may have a single-layered structure or a multilayered structure.

### [Capping layer]

The light-emitting device 10 may include a first capping layer arranged outside the first electrode 110, and/or a second capping layer arranged outside the second electrode 150. In embodiments, the light-emitting device 10 may have a structure in which the first capping layer, the first electrode 110, the interlayer 130, and the second electrode 150 are stacked in this stated order, a structure in which the first electrode 110, the interlayer 130, the second electrode 150, and the second capping layer are stacked in this stated order, or a structure in which the first capping layer, the first electrode 110, the interlayer 130, the second electrode 150, and the second capping layer are stacked in this stated order.

Light generated in an emission layer of the interlayer 130 of the light-emitting device 10 may be extracted through the first electrode 110, which may be a transflective electrode or a transmissive electrode, and through the first capping layer to the outside. Light generated in an emission layer of the interlayer 130 of the light-emitting device 10 may be extracted through the second electrode 150, which may be a transflective electrode or a transmissive electrode, and through the second capping layer to the outside.

The first capping layer and the second capping layer may each increase external emission efficiency according to the principle of constructive interference. Accordingly, light extraction efficiency of the light-emitting device 10 is increased, such that the luminescence efficiency of the light-emitting device 10 may be increased.

The first capping layer and the second capping layer may each include a material having a refractive index equal to or greater than about 1.6 (with respect to a wavelength of about 589 nm).

The first capping layer and the second capping layer may each independently be an organic capping layer including an organic material, an inorganic capping layer including an inorganic material, or an organic-inorganic composite capping layer including an organic material and an inorganic material.

At least one of the first capping layer and the second capping layer may each independently include a carbocyclic compound, a heterocyclic compound, an amine group-containing compound, a porphine derivative, a phthalocyanine derivative, a naphthalocyanine derivative, an alkali metal complex, an alkaline earth metal complex, or any combination thereof. The carbocyclic compound, the heterocyclic compound, and the amine group-containing compound may optionally be substituted with a substituent including O, N, S, Se, Si, F, Cl, Br, I, or any combination thereof. In an embodiment, at least one of the first capping layer and the second capping layer may each independently include an amine group-containing compound.

In an embodiment, at least one of the first capping layer and the second capping layer may each independently include a compound represented by Formula 201, a compound represented by Formula 202, or any combination thereof.

In an embodiment, at least one of the first capping layer and the second capping layer may each independently include one of Compounds HT28 to HT33, one of Compounds CP1 to CP6, β-NPB, or any combination thereof:

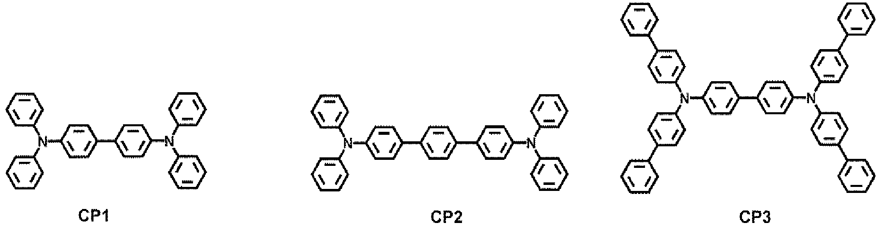

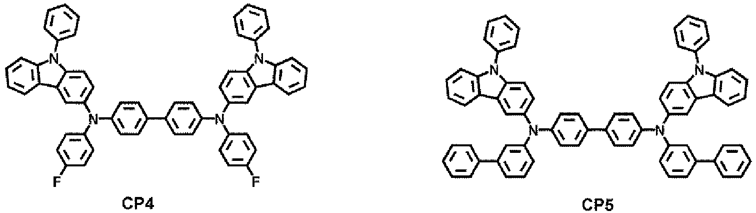

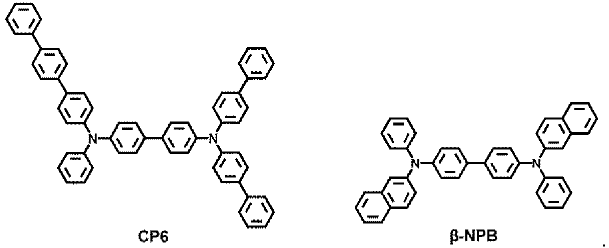

### [Film]

The heterocyclic compound represented by Formula 1 may be included in various films. Thus, according to embodiments, a film may include the heterocyclic compound represented by Formula 1. The film may be, for example, an optical member (or a light control means) (e.g., a color filter, a color conversion member, a capping layer, a light extraction efficiency enhancement layer, a selective light absorbing layer, a polarizing layer, a quantum dot-containing layer, etc.), a light blocking member (e.g., a light reflective layer, a light absorbing layer, etc.), a protective member (e.g., an insulating layer, a dielectric layer, etc.), or the like.

### [Electronic apparatus]

The light-emitting device may be included in various electronic apparatuses. For example, an electronic apparatus including the light-emitting device may be a light-emitting apparatus, an authentication apparatus, or the like.

The electronic apparatus (for example, a light-emitting apparatus) may further include, in addition to the light-emitting device, a color filter, a color conversion layer, or a color filter and a color conversion layer. The color filter and/or the color conversion layer may be arranged in at least one traveling direction of light emitted from the light-emitting device. For example, light emitted from the light-emitting device may be blue light, green light, or white light. Further details on the light-emitting device may be the same as described herein. In an embodiment, the color conversion layer may include quantum dots.

The electronic apparatus may include a substrate. The substrate may include subpixels, the color filter may include color filter areas that respectively correspond to the subpixels, and the color conversion layer may include color conversion areas that respectively correspond to the subpixels.

A pixel-defining film may be arranged between the subpixels to define each subpixel.

The color filter may further include color filter areas and light-shielding patterns arranged between the color filter areas, and the color conversion layer may further include color conversion areas and light-shielding patterns arranged between the color conversion areas.

The color filter areas (or the color conversion areas) may include a first area emitting first color light, a second area emitting second color light, and/or a third area emitting third color light, wherein the first color light, the second color light, and/or the third color light may have different maximum emission wavelengths. In an embodiment, the first color light may be red light, the second color light may be green light, and the third color light may be blue light. In an embodiment, the color filter areas (or the color conversion areas) may include quantum dots. For example, the first area may include red quantum dots, the second area may include green quantum dots, and the third area may not include quantum dots. Further details on the quantum dots may be the same as described herein. The first area, the second area, and/or the third area may each further include a scatterer.

According to an embodiment, the light-emitting device may emit first light, the first area may absorb the first light to emit first-first color light, the second area may absorb the first light to emit second-first color light, and the third area may absorb the first light to emit third-first color light. The first-first color light, the second-first color light, and the third-first color light may have different maximum emission wavelengths. For example, the first light may be blue light, the first-first color light may be red light, the second-first color light may be green light, and the third-first color light may be blue light.

The electronic apparatus may further include a thin-film transistor, in addition to the light-emitting device as described above. The thin-film transistor may include a source electrode, a drain electrode, and an active layer, wherein any one of the source electrode and the drain electrode may be electrically connected to any one of the first electrode and the second electrode of the light-emitting device.

The thin-film transistor may further include a gate electrode, a gate insulating film, or the like.

The active layer may include crystalline silicon, amorphous silicon, an organic semiconductor, an oxide semiconductor, or the like.

The electronic apparatus may further include a sealing portion for sealing the light-emitting device. The sealing portion may be arranged between the color filter and/or the color conversion layer and the light-emitting device. The sealing portion allows light from the light-emitting device to be extracted to the outside, and simultaneously prevents ambient air and moisture from penetrating into the light-emitting device. The sealing portion may be a sealing substrate that includes a transparent glass substrate or a plastic substrate. The sealing portion may be a thin-film encapsulation layer that includes at least one of an organic layer and/or an inorganic layer. When the sealing portion is a thin film encapsulation layer, the electronic apparatus may be flexible.

Various functional layers may be further included on the sealing portion, in addition to the color filter and/or the color conversion layer, according to a use of the electronic apparatus. Examples of a functional layer may include a touch screen layer and a polarizing layer. The touch screen layer may be a pressure-sensitive touch screen layer, a capacitive touch screen layer, or an infrared touch screen layer. The authentication apparatus may be, for example, a biometric authentication apparatus that authenticates an individual by using biometric information of a living body (for example, fingertips, pupils, etc.).

The authentication apparatus may further include, in addition to the light-emitting device as described above, a biometric information collector.

The electronic apparatus may be applied to various displays, light sources, lighting, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, various measuring instruments, meters (for example, meters for a vehicle, an aircraft, and a vessel), projectors, and the like.

### [Electronic device]

The light-emitting device may be included in various electronic devices.

In an embodiment, an electronic device including the light-emitting device may be a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater screen, a stadium screen, a phototherapy device, or a signboard.

Since the light-emitting device has excellent effects in terms of luminescence efficiency and long lifespan, an electronic device that includes the light-emitting device may have characteristics such as high luminance, high resolution, and low power consumption.

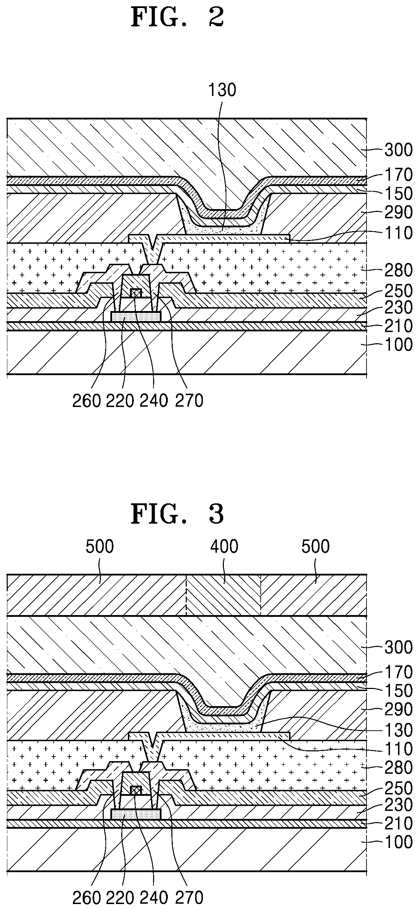

### [Description of FIGS. 2 and 3]

FIG. 2 is a schematic cross-sectional view of an electronic apparatus according to an embodiment.

The electronic apparatus (for example, a light-emitting apparatus) of FIG. 2 includes a substrate 100, a thin-film transistor (TFT), a light-emitting device, and an encapsulation portion 300 that seals the light-emitting device.

The substrate 100 may be a flexible substrate, a glass substrate, or a metal substrate. A buffer layer 210 may be arranged on the substrate 100. The buffer layer 210 may prevent the penetration of impurities through the substrate 100 and may provide a flat surface on the substrate 100.

A TFT may be arranged on the buffer layer 210. The TFT may include an active layer 220, a gate electrode 240, a source electrode 260, and a drain electrode 270.

The active layer 220 may include an inorganic semiconductor, such as silicon or polysilicon, an organic semiconductor, or an oxide semiconductor, and the active layer 220 may include a source region, a drain region, and a channel region.

A gate insulating film 230 for insulating the active layer 220 from the gate electrode 240 may be arranged on the active layer 220, and the gate electrode 240 may be arranged on the gate insulating film 230.

An interlayer insulating film 250 may be arranged on the gate electrode 240. The interlayer insulating film 250 may be arranged between the gate electrode 240 and the source electrode 260 and between the gate electrode 240 and the drain electrode 270 to insulate the gate electrode 240, the source electrode 260, and the drain electrode 270 from one another.

The source electrode 260 and the drain electrode 270 may be arranged on the interlayer insulating film 250. The interlayer insulating film 250 and the gate insulating film 230 may be formed to expose a source region and a drain region of the active layer 220, and the source electrode 260 and the drain electrode 270 may respectively contact the exposed portions of the source region and the drain region of the active layer 220.

The TFT may be electrically connected to a light-emitting device to drive the light-emitting device, and may be covered and protected by a passivation layer 280. The passivation layer 280 may include an inorganic insulating film, an organic insulating film, or any combination thereof. A light-emitting device may be provided on the passivation layer 280. The light-emitting device may include the first electrode 110, the interlayer 130, and the second electrode 150.

The first electrode 110 may be arranged on the passivation layer 280. The passivation layer 280 may not completely cover the drain electrode 270 and may expose a portion of the drain electrode 270. The first electrode 110 may be connected (for example, electrically connected) to the exposed portion of the drain electrode 270.

A pixel-defining film 290 including an insulating material may be arranged on the first electrode 110. The pixel-defining film 290 may expose a region of the first electrode 110, and the interlayer 130 may be formed on the exposed region of the first electrode 110. The pixel-defining film 290 may be a polyimide-based organic film or a polyacrylic organic film. Although not shown in FIG. 2, at least some layers of the interlayer 130 may extend beyond the upper portion of the pixel-defining film 290 to be provided in the form of a common layer.

The second electrode 150 may be arranged on the interlayer 130, and a capping layer 170 may be further included on the second electrode 150. The capping layer 170 may be formed to cover the second electrode 150.

The encapsulation portion 300 may be arranged on the capping layer 170. The encapsulation portion 300 may be disposed on a light-emitting device to protect the light-emitting device from moisture and/or oxygen. The encapsulation portion 300 may include: an inorganic film including silicon nitride (SiNₓ), silicon oxide (SiOₓ), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, or the like), an epoxy-based resin (for example, aliphatic glycidyl ether (AGE), or the like), or any combination thereof; or a combination of the inorganic film and the organic film.

FIG. 3 is a schematic cross-sectional view of an electronic apparatus according to another embodiment.

The electronic apparatus (for example, a light-emitting apparatus) of FIG. 3 may differ from the electronic apparatus of FIG. 2, at least in that a light-shielding pattern 500 and a functional region 400 are further included on the encapsulation portion 300. The functional region 400 may be a color filter area, a color conversion area, or a combination of the color filter area and the color conversion area. In an embodiment, a light-emitting device included in the electronic apparatus of FIG. 3 may be a tandem light-emitting device.

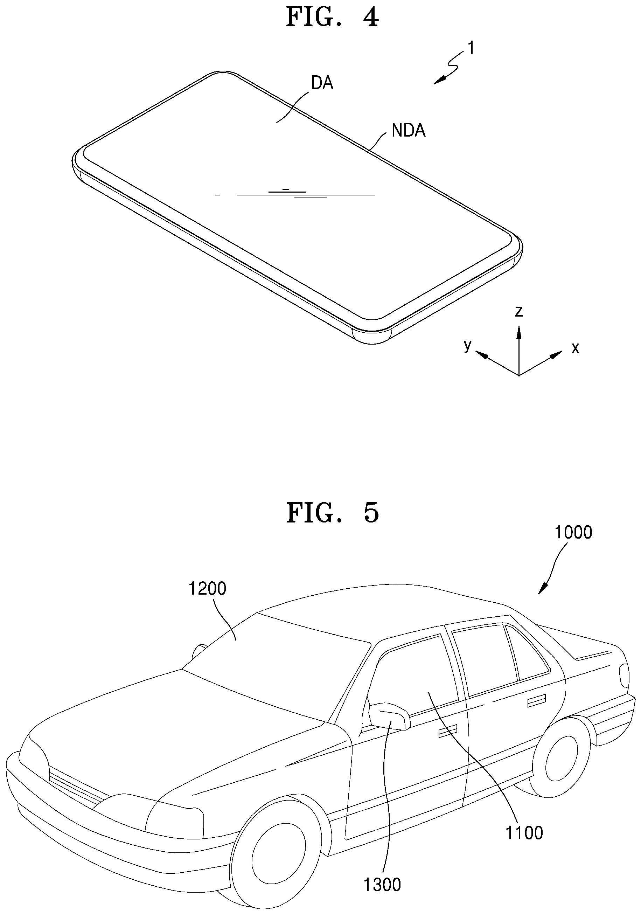

### [Description of FIG. 4]

FIG. 4 is a schematic perspective view of an electronic device including a light-emitting device according to an embodiment.

The electronic device 1, which may be an apparatus that displays a moving image or a still image, may be not only a portable electronic device, such as a mobile phone, a smartphone, a tablet computer, a mobile communication terminal, an electronic notebook, an electronic book, a portable multimedia player (PMP), a navigation device, or an ultra-mobile personal computer (UMPC), but may also be various products, such as a television, a laptop computer, a monitor, a billboard, or an Internet of things (IoT) device. The electronic device 1 may be any such product as described above or a part thereof.

In an embodiment, the electronic device 1 may be a wearable device, such as a smart watch, a watch phone, a glasses-type display, or a head mounted display (HMD), or a part of the wearable device. However, embodiments are not limited thereto.

In embodiments, examples of the electronic device 1 may include a dashboard of a vehicle, a center information display (CID) arranged on a center fascia or dashboard of a vehicle, a room mirror display that replaces a side-view mirror of a vehicle, an entertainment display for a rear seat of a vehicle, a display arranged on the back of a front seat of a vehicle, a head up display (HUD) installed at the front of a vehicle or projected on a front window glass, or a computer generated hologram augmented reality head up display (CGH AR HUD). FIG. 4 illustrates an embodiment in which the electronic device 1 is a smartphone, for convenience of explanation.

The electronic device 1 may include a display area DA, and a non-display area NDA outside the display area DA. A display apparatus may implement an image through a two-dimensional array of pixels that are arranged in the display area DA.

The non-display area NDA is an area that does not display an image, and may surround (for example, entirely surround) the display area DA. A driver which provides electrical signals or power to display devices arranged on the display area DA, may be arranged in the non-display area NDA. A pad, which is an area to which an electronic element or a printed circuit board may be electrically connected, may be arranged in the non-display area NDA.

In the electronic device 1, a length in an x-axis direction and a length in a y-axis direction may be different from each other. In an embodiment as shown in FIG. 4, the length in the x-axis direction may be less than the length in the y-axis direction. In an embodiment, the length in the x-axis direction may be the same as the length in the y-axis direction. In an embodiment, the length in the x-axis direction may be greater than the length in the y-axis direction.

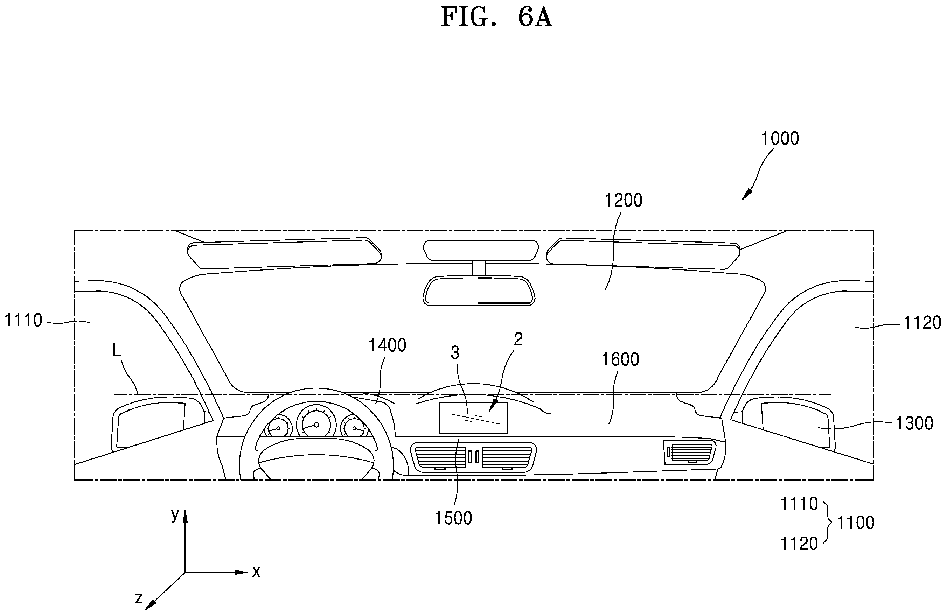

### [Descriptions of FIGS. 5 and 6A to 6C]

FIG. 5 is a schematic perspective view of an exterior of a vehicle 1000 as an electronic device including a light-emitting device, according to an embodiment. FIGS. 6A to 6C are each a schematic diagram of an interior of a vehicle 1000 according to embodiments.

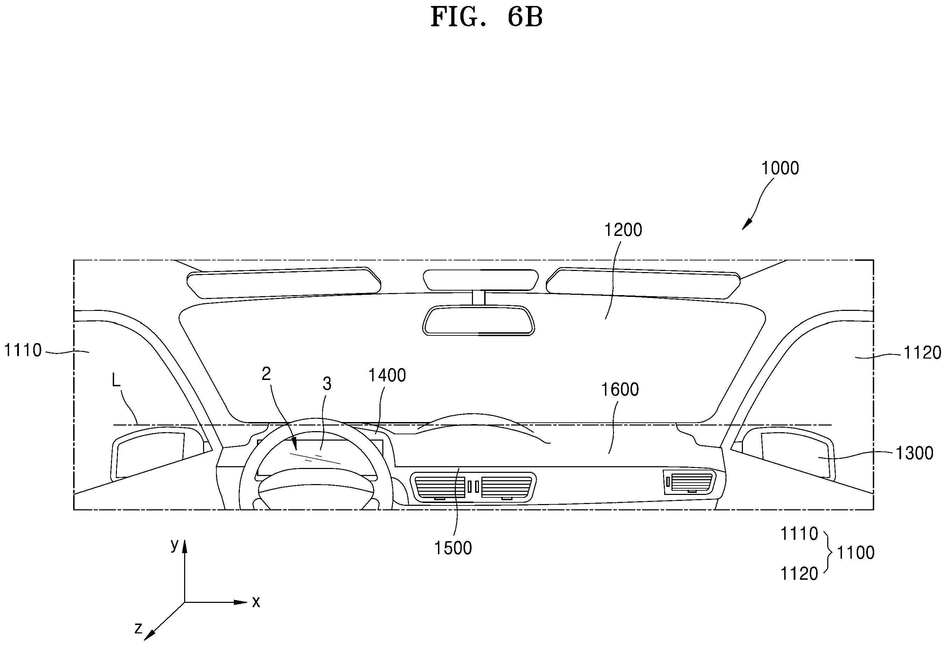

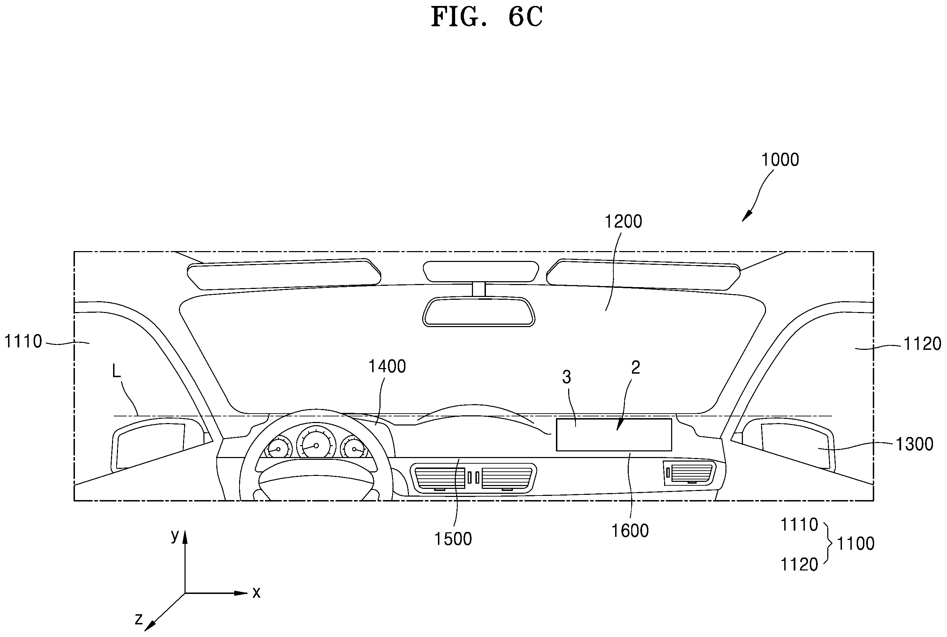

Referring to FIGS. 5, 6A, 6B, and 6C, embodiments of a vehicle 1000 may include various apparatuses for moving a subject to be transported, such as a person, an object, or an animal, from a departure point to a destination. Examples of a vehicle 1000 may include a vehicle traveling on a road or a track, a vessel moving over a sea or a river, an airplane flying in the sky using the action of air, and the like.

The vehicle 1000 may travel on a road or a track. The vehicle 1000 may move in a selected or given direction according to the rotation of at least one wheel. In an embodiment, examples of a vehicle 1000 may include a three-wheeled or four-wheeled vehicle, a construction machine, a two-wheeled vehicle, a prime mover device, a bicycle, and a train running on a track.

The vehicle 1000 may include a vehicle body having an interior and an exterior, and a chassis that is a portion excluding the vehicle body in which mechanical apparatuses necessary for driving are installed. The exterior of the vehicle body may include a front panel, a bonnet, a roof panel, a rear panel, a trunk, a pillar provided at a boundary between doors, and the like. The chassis may include a power generating device, a power transmitting device, a driving device, a steering device, a braking device, a suspension device, a transmission device, a fuel device, front and rear wheels, left and right wheels, and the like.

The vehicle 1000 may include a side window glass 1100, a front window glass 1200, a side-view mirror 1300, a cluster 1400, a center fascia 1500, a passenger seat dashboard 1600, and a display apparatus 2.

The side window glass 1100 and the front window glass 1200 may be partitioned by a pillar arranged between the side window glass 1100 and the front window glass 1200.

The side window glass 1100 may be installed on a side of the vehicle 1000. In an embodiment, the side window glass 1100 may be installed on a door of the vehicle 1000. Multiple side window glasses 1100 may be provided and may face each other. In an embodiment, the side window glass 1100 may include a first side window glass 1110 and a second side window glass 1120. In an embodiment, the first side window glass 1110 may be arranged adjacent to the cluster 1400, and the second side window glass 1120 may be arranged adjacent to the passenger seat dashboard 1600.

In an embodiment, the side window glasses 1100 may be spaced apart from each other in an x direction or a -x direction. In an embodiment, the first side window glass 1110 and the second side window glass 1120 may be spaced apart from each other in the x direction or the -x direction. For example, a virtual straight line L connecting the side window glasses 1100 may extend in the x direction or the -x direction. In an embodiment, a virtual straight line L connecting the first side window glass 1110 and the second side window glass 1120 to each other may extend in the x direction or the -x direction.

The front window glass 1200 may be installed in the front of the vehicle 1000. The front window glass 1200 may be arranged between the side window glasses 1100 facing each other.

The side-view mirror 1300 may provide a rear view of the vehicle 1000. The side-view mirror 1300 may be installed on the exterior of the vehicle body. In an embodiment, multiple side-view mirrors 1300 may be provided. For example, one of the side-view mirrors 1300 may be arranged outside the first side window glass 1110, and another of the side-view mirrors 1300 may be arranged outside the second side window glass 1120.

The cluster 1400 may be arranged in front of a steering wheel. The cluster 1400 may include a tachometer, a speedometer, a coolant thermometer, a fuel gauge, a turn signal indicator, a high beam indicator, a warning light, a seat belt warning light, an odometer, a tachograph, an automatic shift selector indicator, a door open warning light, an engine oil warning light, and/or a low fuel warning light.

The center fascia 1500 may include a control panel on which buttons for adjusting an audio device, an air conditioning device, and a seat heater are disposed. The center fascia 1500 may be arranged on a side of the cluster 1400.

The passenger seat dashboard 1600 may be spaced apart from the cluster 1400, and the center fascia 1500 may be arranged between the cluster 1400 and the passenger seat dashboard 1600. In an embodiment, the cluster 1400 may be arranged to correspond to a driver seat (not shown), and the passenger seat dashboard 1600 may be arranged to correspond to a passenger seat (not shown). In an embodiment, the cluster 1400 may be adjacent to the first side window glass 1110, and the passenger seat dashboard 1600 may be adjacent to the second side window glass 1120.

In an embodiment, the display apparatus 2 may include a display panel 3, and the display panel 3 may display an image. The display apparatus 2 may be arranged inside the vehicle 1000. In an embodiment, the display apparatus 2 may be arranged between the side window glasses 1100 facing each other. The display apparatus 2 may be arranged on at least one of the cluster 1400, the center fascia 1500, and the passenger seat dashboard 1600.

The display apparatus 2 may include an organic light-emitting display, an inorganic electroluminescent display, a quantum dot display, or the like. Hereinafter, an organic light-emitting display apparatus including the light-emitting device according to an embodiment will be described as an example of the display apparatus 2. However, various types of display apparatuses as described above may be used in embodiments.

Referring to FIG. 6A, the display apparatus 2 may be arranged on the center fascia 1500. In an embodiment, the display apparatus 2 may display navigation information. In an embodiment, the display apparatus 2 may display information regarding audio settings, video settings, or vehicle settings.

Referring to FIG. 6B, the display apparatus 2 may be arranged on the cluster 1400. The cluster 1400 may display driving information and the like through the display apparatus 2. For example, the cluster 1400 may digitally implement driving information and the life. The cluster 1400 may digitally implement vehicle information and driving information as images. In an embodiment, a needle and a gauge of a tachometer and various warning lights or icons may be displayed by a digital signal.

Referring to FIG. 6C, the display apparatus 2 may be arranged on the passenger seat dashboard 1600. The display apparatus 2 may be embedded in the passenger seat dashboard 1600 or arranged on the passenger seat dashboard 1600. In an embodiment, the display apparatus 2 arranged on the passenger seat dashboard 1600 may display an image that is related to information displayed on the cluster 1400 and/or information displayed on the center fascia 1500. In an embodiment, the display apparatus 2 arranged on the passenger seat dashboard 1600 may display information that is different from information displayed on the cluster 1400 and/or information displayed on the center fascia 1500.

### [Manufacturing method]

Layers constituting the hole transport region, the emission layer, and the layers constituting the electron transport region may be formed in a selected region by using various methods such as vacuum deposition, spin coating, casting, Langmuir-Blodgett (LB) deposition, ink-jet printing, laser-printing, laser-induced thermal imaging, and the like.

When the layers constituting the hole transport region, the emission layer, and the layers constituting the electron transport region are formed by vacuum deposition, the deposition may be performed at a deposition temperature in a range of about 100 °C to about 500 °C, at a vacuum degree in a range of about 10⁻⁸ torr to about 10⁻³ torr, and at a deposition speed in a range of about 0.01 Å/sec to about 100 Å/sec, depending on a material to be included in a layer to be formed and the structure of a layer to be formed.

### [Definitions of terms]

The term "C₃-C₆₀ carbocyclic group" as used herein may be a cyclic group consisting of carbon atoms as the only ring-forming atoms and having three to sixty carbon atoms. The term "C₁-C₆₀ heterocyclic group" as used herein may be a cyclic group that has one to sixty carbon atoms and further includes, in addition to the carbon atoms, at least one heteroatom as a ring-forming atom. The C₃-C₆₀ carbocyclic group and the C₁-C₆₀ heterocyclic group may each be a monocyclic group consisting of one ring or a polycyclic group in which two or more rings are condensed with each other. In an embodiment, the number of ring-forming atoms in a C₁-C₆₀ heterocyclic group may be from 3 to 61.

The term "cyclic group" as used herein may be a C₃-C₆₀ carbocyclic group or a C₁-C₆₀ heterocyclic group.

The term "π electron-rich C₃-C₆₀ cyclic group" as used herein may be a cyclic group that has 3 to 60 carbon atoms and may not include *-N=*' as a ring-forming moiety. The term "π electron-deficient nitrogen-containing C₁-C₆₀ heterocyclic group" as used herein may be a heterocyclic group that has 1 to 60 carbon atoms and may include *-N=*' as a ring-forming moiety.

### In embodiments,

a C₃-C₆₀ carbocyclic group may be a T1 group or a group in which two or more T1 groups are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),

a C₁-C₆₀ heterocyclic group may be a T2 group, a group in which two or more T2 groups are condensed with each other, or a group in which at least one T2 group and at least one T1 group are condensed with each other (e.g., a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, etc.),

a π electron-rich C₃-C₆₀ cyclic group may be a T1 group, a group in which two or more T1 groups are condensed with each other, a T3 group, a group in which two or more T3 groups are condensed with each other, or a group in which at least one T3 group and at least one T1 group are condensed with each other (for example, a C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, or the like), and

a π electron-deficient nitrogen-containing C₁-C₆₀ heterocyclic group may be a T4 group, a group in which two or more T4 groups are condensed with each other, a group in which at least one T4 group and at least one T1 group are condensed with each other, a group in which at least one T4 group and at least one T3 group are condensed with each other, or a group in which at least one T4 group, at least one T1 group, and at least one T3 group are condensed with one another (e.g., a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, etc.), wherein

a T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or bicyclo[2.2.1]heptane) group, a norbornene group, a bicyclo[1.1.1]pentane group, a bicyclo[2.1.1]hexane group, a bicyclo[2.2.2]octane group, or a benzene group,

a T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,

a T3 group may be a furan group, a thiophene group, a 1H-pyrrole group, a silole group, or a borole group, and

a T4 group may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "cyclic group," "C₃-C₆₀ carbocyclic group," "C₁-C₆₀ heterocyclic group," "π electron-rich C₃-C₆₀ cyclic group," and "π electron-deficient nitrogen-containing C₁-C₆₀ heterocyclic group" as used herein may each be a group condensed to any cyclic group, a monovalent group, or a polyvalent group (e.g., a divalent group, a trivalent group, a tetravalent group, etc.) according to the structure of a formula for which the corresponding term is used. For example, a "benzene group" may be a benzo group, a phenyl group, a phenylene group, or the like, which may be readily understood by those of ordinary skill in the art according to the structure of a formula including the "benzene group."

Examples of a monovalent C₃-C₆₀ carbocyclic group or a monovalent C₁-C₆₀ heterocyclic group may include a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group. Examples of a divalent C₃-C₆₀ carbocyclic group or a divalent C₁-C₆₀ heterocyclic group may include a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group.

The term "C₁-C₆₀ alkyl group" as used herein may be a linear or branched monovalent aliphatic hydrocarbon group that has one to sixty carbon atoms, and examples thereof may include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, and a tert-decyl group. The term "C₁-C₆₀ alkylene group" as used herein may be a divalent group having a same structure as the C₁-C₆₀ alkyl group.

The term "C₂-C₆₀ alkenyl group" as used herein may be a monovalent hydrocarbon group having at least one carbon-carbon double bond in the middle or at a terminus of a C₂-C₆₀ alkyl group, and examples thereof may include an ethenyl group, a propenyl group, and a butenyl group. The term "C₂-C₆₀ alkenylene group" as used herein may be a divalent group having a same structure as the C₂-C₆₀ alkenyl group.

The term "C₂-C₆₀ alkynyl group" as used herein may be a monovalent hydrocarbon group having at least one carbon-carbon triple bond in the middle or at a terminus of a C₂-C₆₀ alkyl group, and examples thereof may include an ethynyl group and a propynyl group. The term "C₂-C₆₀ alkynylene group" as used herein may be a divalent group having a same structure as the C₂-C₆₀ alkynyl group.

The term "C₁-C₆₀ alkoxy group" as used herein may be a monovalent group represented by -O(A₁₀₁) (wherein A₁₀₁ may be a C₁-C₆₀ alkyl group), and examples thereof may include a methoxy group, an ethoxy group, and an isopropyloxy group.

The term "C₃-C₁₀ cycloalkyl group" as used herein may be a monovalent saturated hydrocarbon cyclic group having 3 to 10 carbon atoms, and examples thereof may include a cyclopropyl group, a cyclobutyl group, a cyclopentyl group, a cyclohexyl group, a cycloheptyl group, a cyclooctyl group, an adamantanyl group, a norbornanyl group (or bicyclo[2.2.1]heptyl group), a bicyclo[1.1.1]pentyl group, a bicyclo[2.1.1]hexyl group, a bicyclo[2.2.2]octyl group, and the like. The term "C₃-C₁₀ cycloalkylene group" as used herein may be a divalent group having a same structure as the C₃-C₁₀ cycloalkyl group.

The term "C₁-C₁₀ heterocycloalkyl group" as used herein may be a monovalent cyclic group that has one to ten carbon atoms and further includes, in addition to the carbon atoms, at least one heteroatom as a ring-forming atom, and examples thereof may include a 1,2,3,4-oxatriazolidinyl group, a tetrahydrofuranyl group, and a tetrahydrothiophenyl group. The term "C₁-C₁₀ heterocycloalkylene group" as used herein may be a divalent group having a same structure as the C₁-C₁₀ heterocycloalkyl group.

The term "C₃-C₁₀ cycloalkenyl group" as used herein may be a monovalent cyclic group that has three to ten carbon atoms and at least one carbon-carbon double bond in the cyclic structure thereof and no aromaticity, and examples thereof may include a cyclopentenyl group, a cyclohexenyl group, and a cycloheptenyl group. The term "C₃-C₁₀ cycloalkenylene group" as used herein may be a divalent group having a same structure as the C₃-C₁₀ cycloalkenyl group.

The term "C₁-C₁₀ heterocycloalkenyl group" as used herein may be a monovalent cyclic group that has one to ten carbon atoms that further includes, in addition to the carbon atoms, at least one heteroatom as a ring-forming atom, and has at least one double bond in the cyclic structure thereof. Examples of a C₁-C₁₀ heterocycloalkenyl group may include a 4,5-dihydro-1,2,3,4-oxatriazolyl group, a 2,3-dihydrofuranyl group, and a 2,3-dihydrothiophenyl group. The term "C₁-C₁₀ heterocycloalkenylene group" as used herein may be a divalent group having a same structure as the C₁-C₁₀ heterocycloalkenyl group.

The term "C₆-C₆₀ aryl group" as used herein may be a monovalent group having a carbocyclic aromatic system of six to sixty carbon atoms, and the term "C₆-C₆₀ arylene group" as used herein may be a divalent group having a carbocyclic aromatic system of six to sixty carbon atoms. Examples of a C₆-C₆₀ aryl group may include a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, and an ovalenyl group. When the C₆-C₆₀ aryl group and the C₆-C₆₀ arylene group each include two or more rings, the respective two or more rings may be condensed with each other.

The term "C₁-C₆₀ heteroaryl group" as used herein may be a monovalent group having a heterocyclic aromatic system that has one to sixty carbon atoms and further includes, in addition to the carbon atoms, at least one heteroatom as a ring-forming atom. The term "C₁-C₆₀ heteroarylene group" as used herein may be a divalent group having a heterocyclic aromatic system that has one to sixty carbon atoms and further includes, in addition to the carbon atoms, at least one heteroatom as a ring-forming atom. Examples of a C₁-C₆₀ heteroaryl group may include a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, and a naphthyridinyl group. When the C₁-C₆₀ heteroaryl group and the C₁-C₆₀ heteroarylene group each include two or more rings, the respective two or more rings may be condensed with each other.

The term "monovalent non-aromatic condensed polycyclic group" as used herein may be a monovalent group having two or more rings condensed with each other, only carbon atoms (for example, eight to sixty carbon atoms) as ring-forming atoms, and no aromaticity in its molecular structure when considered as a whole. Examples of a monovalent non-aromatic condensed polycyclic group may include an indenyl group, a fluorenyl group, a spiro-bifluorenyl group, a benzofluorenyl group, an indenophenanthrenyl group, and an indeno anthracenyl group. The term "divalent non-aromatic condensed polycyclic group" as used herein may be a divalent group having a same structure as the monovalent non-aromatic condensed polycyclic group.

The term "monovalent non-aromatic condensed heteropolycyclic group" as used herein may be a monovalent group that has two or more rings condensed with each other that further includes, in addition to carbon atoms (for example, one to sixty carbon atoms), at least one heteroatom as a ring-forming atom, and has no aromaticity in its molecular structure when considered as a whole. Examples of a monovalent non-aromatic condensed heteropolycyclic group may include a pyrrolyl group, a thiophenyl group, a furanyl group, an indolyl group, a benzoindolyl group, a naphtho indolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indenocarbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, a benzothienodibenzothiophenyl group, and the like. The term "divalent non-aromatic condensed heteropolycyclic group" as used herein may be a divalent group having a same structure as the monovalent non-aromatic condensed heteropolycyclic group.

The term "C₆-C₆₀ aryloxy group" as used herein may be a group represented by -O(A₁₀₂) (wherein A₁₀₂ may be a C₆-C₆₀ aryl group), the term "C₆-C₆₀ arylthio group" as used herein may be a group represented by -S(A₁₀₃) (wherein A₁₀₃ may be a C₆-C₆₀ aryl group), and the term "C₆-C₆₀ arylseleno group" as used herein may be a group represented by -Se(A₁₀₈) (wherein A₁₀₈ may be a C₆-C₆₀ aryl group).

The term "C₇-C₆₀ arylalkyl group" as used herein may be a group represented by -(A₁₀₄)(A₁₀₅) (wherein A₁₀₄ may be a C₁-C₅₄ alkylene group, and A₁₀₅ may be a C₆-C₅₉ aryl group), and the term "C₂-C₆₀ heteroarylalkyl group" as used herein may be a group represented by -(A₁₀₆)(A₁₀₇) (wherein A₁₀₆ may be a C₁-C₅₉ alkylene group, and A₁₀₇ may be a C₁-C₅₉ heteroaryl group).

In the specification, the group "R₁₀ₐ" may be:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂).

In the specification, Q₁ to Q₃, Q₁₁ to Q₁₃, Q₂₁ to Q₂₃, and Q₃₁ to Q₃₃ may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, -F, a cyano group, a C₁-C₆₀ alkyl group, a C₁-C₆₀ alkoxy group, a phenyl group, a biphenyl group, or any combination thereof.

The term "heteroatom" as used herein may be any atom other than a carbon atom or a hydrogen atom. Examples of a heteroatom may include O, S, N, P, Si, B, Ge, Se, and any combination thereof.

In the specification, examples of a "third-row transition metal" may include hafnium (Hf), tantalum (Ta), tungsten (W), rhenium (Re), osmium (Os), iridium (Ir), platinum (Pt), gold (Au), and the like.

In the specification, the term "Ph" refers to a phenyl group, the term "Me" refers to a methyl group, the term "Et" refers to an ethyl group, the terms "tert-Bu" and "Bu^{t}" each refers to a tert-butyl group, and the term "OMe" refers to a methoxy group.

The term "biphenyl group" as used herein may be a "phenyl group that is substituted with a phenyl group." For example, a "biphenyl group" may be a substituted phenyl group having a C₆-C₆₀ aryl group as a substituent.

The term "terphenyl group" as used herein may be a "phenyl group substituted with a biphenyl group." For example, a "terphenyl group" may be a substituted phenyl group having, as a substituent, a C₆-C₆₀ aryl group substituted with a C₆-C₆₀ aryl group.

In the specification, the symbols * and *', unless defined otherwise, each represent a binding site to a neighboring atom in a corresponding formula or moiety.

In the specification, the terms "x-axis," "y-axis," and "z-axis" are not limited to three axes in an orthogonal coordinate system (for example, a Cartesian coordinate system), and may be interpreted in a broader sense than the aforementioned three axes in an orthogonal coordinate system. For example, the x-axis, y-axis, and z-axis may describe axes that are orthogonal to each other, or may describe axes that are in different directions that are not orthogonal to each other.

As used herein, the term "C₃-C₆₀ carbocyclic group" includes a C₃-C₅₀ carbocyclic group, a C₃-C₄₀ carbocyclic group, a C₃-C₃₀ carbocyclic group, a C₃-C₂₀ carbocyclic group, or a C₃-C₁₀ carbocyclic group;
The term "C₁-C₆₀ heterocyclic group" includes a C₁-C₅₀ heterocyclic group, a C₁-C₄₀ heterocyclic group, a C₁-C₃₀ heterocyclic group, a C₁-C₂₀ heterocyclic group, or a C₁-C₁₀ heterocyclic group;
The term "C₁-C₆₀ alkyl group" includes a C₁-C₅₀ alkyl group, a C₁-C₃₀ alkyl group, a C₁-C₂₀ alkyl group, or a C₁-C₁₀ alkyl group;
The term "C₂-C₆₀ alkenyl group" includes a C₂-C₃₀ alkenyl group, a C₂-C₂₀ alkenyl group, or a C₂-C₁₀ alkenyl group;
The term "C₂-C₆₀ alkynyl group" includes a C₂-C₃₀ alkynyl group, a C₂-C₂₀ alkynyl group, or a C₂-C₁₀ alkynyl group;
The term "C₁-C₆₀ alkoxy group" includes a C₁-C₃₀ alkoxy group, a C₁-C₂₀ alkoxy group, or a C₁-C₁₀ alkoxy group;
The term "C₆-C₆₀ aryl group" includes a C₆-C₅₀ aryl group, a C₆-C₄₀ aryl group, a C₆-C₃₀ aryl group, a C₆-C₂₀ aryl group, or a C₆-C₁₅ aryl group;
The term "C₁-C₆₀ heteroaryl group" includes a C₁-C₅₀ heteroaryl group, a C₁-C₄₀ heteroaryl group, a C₁-C₃₀ heteroaryl group, a C₁-C₂₀ heteroaryl group, or a C₁-C₁₀ heteroaryl group;
The term "monovalent non-aromatic condensed polycyclic group" includes a C₈-C₆₀ monovalent non-aromatic condensed polycyclic group, a C₈-C₅₀ monovalent non-aromatic condensed polycyclic group, a C₈-C₄₀ monovalent non-aromatic condensed polycyclic group, a C₈-C₃₀ monovalent non-aromatic condensed polycyclic group, or a C₈-C₂₀ monovalent non-aromatic condensed polycyclic group;
The term "monovalent non-aromatic condensed heteropolycyclic group" includes a C₁-C₆₀ monovalent non-aromatic condensed heteropolycyclic group, a C₁-C₅₀ monovalent non-aromatic condensed heteropolycyclic group, a C₁-C₄₀ monovalent non-aromatic condensed heteropolycyclic group, a C₁-C₃₀ monovalent non-aromatic condensed heteropolycyclic group, or a C₁-C₂₀ monovalent non-aromatic condensed heteropolycyclic group;
The term "C₆-C₆₀ aryloxy group" includes a C₆-C₅₀ aryloxy group, a C₆-C₄₀ aryloxy group, a C₆-C₃₀ aryloxy group, a C₆-C₂₀ aryloxy group, or a C₆-C₁₅ aryloxy group;
The term "C₆-C₆₀ arylthio group" includes a C₆-C₅₀ arylthio group, a C₆-C₄₀ arylthio group, a C₆-C₃₀ arylthio group, a C₆-C₂₀ arylthio group, or a C₆-C₁₅ arylthio group;
The term "C₆-C₆₀ arylseleno group" includes a C₆-C₅₀ arylseleno group, a C₆-C₄₀ arylseleno group, a C₆-C₃₀ arylseleno group, a C₆-C₂₀ arylseleno group, or a C₆-C₁₅ arylseleno group;
The term "C₇-C₆₀ arylalkyl group" includes a C₇-C₅₀ arylalkyl group, a C₇-C₄₀ arylalkyl group, a C₇-C₃₀ arylalkyl group, a C₇-C₂₀ arylalkyl group, or a C₇-C₁₅ arylalkyl group; and
The term "C₂-C₆₀ heteroarylalkyl group" includes a C₂-C₅₀ heteroarylalkyl group, a C₂-C₄₀ heteroarylalkyl group, a C₂-C₃₀ heteroarylalkyl group, a C₂-C₂₀ heteroarylalkyl group, or a C₂-C₁₅ heteroarylalkyl group.

In the present invention, "an integer selected from 0 to 20" refers to an integer selected from 0, 1, 2, 3, 4, 5, 6, 7, 8, 9, 10, 11, 12, 13, 14, 15, 16, 17, 18, 19, and 20. The above description of numerical ranges is also applicable for any other numerical range that appears in the present specification, for example, an integer selected from 0 and 1, an integer selected from 0 to 2, an integer selected from 0 to 3, an integer selected from 0 to 4, an integer selected from 0 to 5, an integer selected from 0 to 6, an integer selected from 0 to 7, an integer selected from 0 to 8, an integer selected from 0 to 9, an integer selected from 0 to 10, an integer selected from 0 to 11, an integer selected from 0 to 12, an integer selected from 0 to 13, an integer selected from 0 to 14, an integer selected from 0 to 15, an integer selected from 0 to 16, an integer selected from 0 to 17, an integer selected from 0 to 18, an integer selected from 0 to 19, and the like.

Hereinafter, compounds according to embodiments and light-emitting devices according to embodiments will be described in detail with reference to the Synthesis Examples and the Examples. The wording "B was used instead of A" used in describing Synthesis Examples means that an identical molar equivalent of B was used in place of A.

### [Synthesis Examples]

### [Synthesis Example 1: Synthesis of Compound 15]

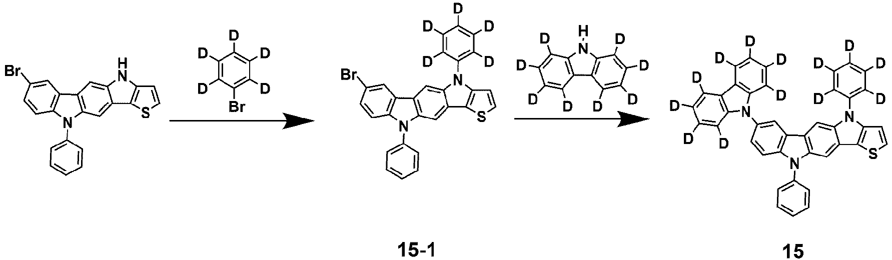

### 1) Synthesis of Intermediate 15-1

Pd(OAc)₂ (0.1 eq), [(t-Bu)₃PH]BF₄ (0.15 eq), K₂CO₃ (5 eq), and toluene (based on 0.1 M 1 eq reagent) were added to a flask containing 7-bromo-10-phenyl-4,10-dihydrothieno[2',3':4,5]pyrrolo[3,2-b]carbazole (1 eq) and 1-bromobenzene-2,3,4,5,6-d5 (1.2 eq), and the mixture was stirred under reflux for 24 hours. The reaction mixture was cooled to room temperature, an extraction process was performed thereon by using methylene chloride (MC), and the extracted material was washed with distilled water. After drying with MgSO₄ and distillation under reduced pressure, the residue was separated through a column to synthesize Intermediate 15-1 (yield of 59.9 %). Intermediate 15-1 was confirmed by LC-MS.

### (C₂₈H₁₂D₅BrN₂S : [M]+497.05)

### 2) Synthesis of Compound 15

Pd(dba)₃ (0.06 eq), (t-Bu)3P(tri-tert-butylphosphine, 0.09 eq), t-BuONa(4.4 eq), and toluene (based on 0.1 M 1 eq reagent) were added to a flask containing Intermediate 15-1 (1eq) and 9H-carbazole-1,2,3,4,5,6,7,8-d8 (1.2 eq), and the mixture was stirred under reflux for 24 hours. The reaction mixture was cooled to room temperature, an extraction process was performed thereon by using MC, and the extracted material was washed with distilled water. After drying with MgSO₄ and distillation under reduced pressure, the residue was separated through a column to obtain Compound 15 (yield of 90.5 %). Compound 15 was confirmed by LC-MS.

### [Synthesis Example 2: Synthesis of Compound 16]

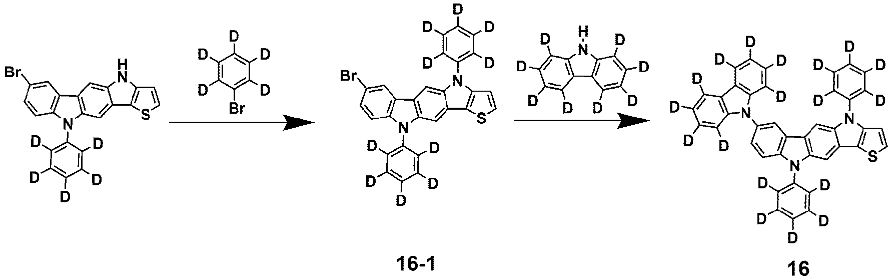

### 1) Synthesis of Intermediate 16-1

Pd(OAc)₂ (0.1 eq), [(t-Bu)₃PH]BF₄ (0.15 eq), K₂CO₃ (5 eq), and toluene (based on 0.1 M 1 eq reagent) were added to a flask containing 7-bromo-10-(phenyl-d5)-4,10-dihydrothieno[2',3':4,5]pyrrolo[3,2-b]carbazole (1 eq) and 1-bromobenzene-2,3,4,5,6-d5 (1.2 eq), and the mixture was stirred under reflux for 27 hours. The reaction mixture was cooled to room temperature, an extraction process was performed thereon by using MC, and the extracted material was washed with distilled water. After drying with MgSO₄ and distillation under reduced pressure, the residue was separated through a column to synthesize Intermediate 16-1 (yield of 48.7 %). Intermediate 16-1 was confirmed by LC-MS.

### (C₂₈H₇D₁₀BrN₂S : [M]+502.05)

### 2) Synthesis of Compound 16

Pd(dba)₃ (0.06 eq), (t-Bu)3P (0.09 eq), t-BuONa (4.4 eq), and toluene (based on 0.1 M 1 eq reagent) were added to a flask containing Intermediate 16-1 (1 eq) and 9H-carbazole-1,2,3,4,5,6,7,8-d8 (1.2 eq), and the mixture was stirred under reflux for 30 hours. The reaction mixture was cooled to room temperature, an extraction process was performed thereon by using MC, and the extracted material was washed with distilled water. After drying with MgSO₄ and distillation under reduced pressure, the residue was separated through a column to obtain Compound 16 (yield of 85.7 %). Compound 16 was confirmed by LC-MS.

### [Synthesis Example 3: Synthesis of Compound 19]

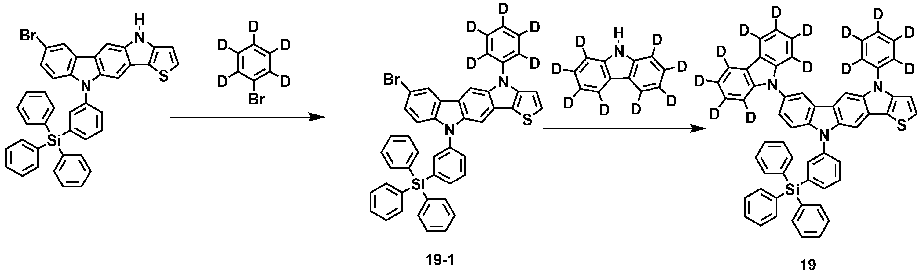

### 1) Synthesis of Intermediate 19-1

Pd(OAc)₂ (0.1 eq), [(t-Bu)₃PH]BF₄ (0.15 eq), K₂CO₃ (5 eq), and toluene (based on 0.1 M 1 eq reagent) were added to a flask containing 7-bromo-10-(3-(triphenylsilyl)phenyl)-4,10-dihydrothieno[2',3':4,5]pyrrolo[3,2-b]carbazole (1 eq) and 1-bromobenzene-2,3,4,5,6-d5 (1.2 eq), and the mixture was stirred under reflux for 27 hours. The reaction mixture was cooled to room temperature, an extraction process was performed thereon by using MC, and the extracted material was washed with distilled water. After drying with MgSO₄ and distillation under reduced pressure, the residue was separated through a column to synthesize Intermediate 19-1 (yield of 50.2 %). Intermediate 19-1 was confirmed by LC-MS.

### (C₄₆H₂₆D₅BrN₂SSi : [M]+755.50)2) Synthesis of Compound 19

Pd(dba)₃ (0.06 eq), (t-Bu)3P (0.09 eq), t-BuONa (4.4 eq), and toluene (based on 0.1 M 1 eq reagent) were added to a flask containing Intermediate 19-1 (1 eq) and 9H-carbazole-1,2,3,4,5,6,7,8-d8 (1.2 eq), and the mixture was stirred under reflux for 30 hours. The reaction mixture was cooled to room temperature, an extraction process was performed thereon by using MC, and the extracted material was washed with distilled water. After drying with MgSO₄ and distillation under reduced pressure, the residue was separated through a column to obtain Compound 19 (yield of 79.2 %). Compound 19 was confirmed by LC-MS.

### [Synthesis Example 4: Synthesis of Compound 20]

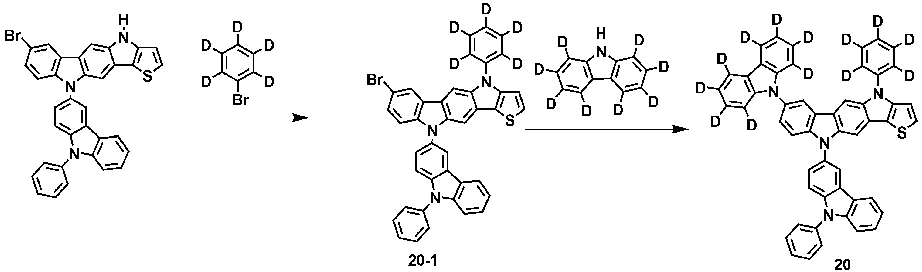

### 1) Synthesis of Intermediate 20-1

Pd(OAc)₂ (0.1 eq), [(t-Bu)₃PH]BF₄ (0.15 eq), K₂CO₃ (5 eq), and toluene (based on 0.1 M 1 eq reagent) were added to a flask containing 7-bromo-10-(9-phenyl-9H-carbazol-3-yl)-4,10-dihydrothieno[2',3':4,5]pyrrolo[3,2-b]carbazole (1 eq) and 1-bromobenzene-2,3,4,5,6-d5 (1.2 eq), and the mixture was stirred under reflux for 27 hours. The reaction mixture was cooled to room temperature, an extraction process was performed thereon by using MC, and the extracted material was washed with distilled water. After drying with MgSO₄ and distillation under reduced pressure, the residue was separated through a column to synthesize Intermediate 20-1 (yield of 49.3 %). Intermediate 20-1 was confirmed by LC-MS.

### (C₄₀H₁₉D₅BrN₃S: [M]+677.6)2) Synthesis of Compound 20

Pd(dba)₃ (0.06 eq), (t-Bu)3P (0.09 eq), t-BuONa (4.4 eq), and toluene (based on 0.1 M 1 eq reagent) were added to a flask containing Intermediate 20-1 (1 eq) and 9H-carbazole-1,2,3,4,5,6,7,8-d8 (1.2 eq), and the mixture was stirred under reflux for 30 hours. The reaction mixture was cooled to room temperature, an extraction process was performed thereon by using MC, and the extracted material was washed with distilled water. After drying with MgSO₄ and distillation under reduced pressure, the residue was separated through a column to obtain Compound 20 (yield of 70.6 %). Compound 20 was confirmed by LC-MS.

### [Synthesis Example 5: Synthesis of Compound 21]

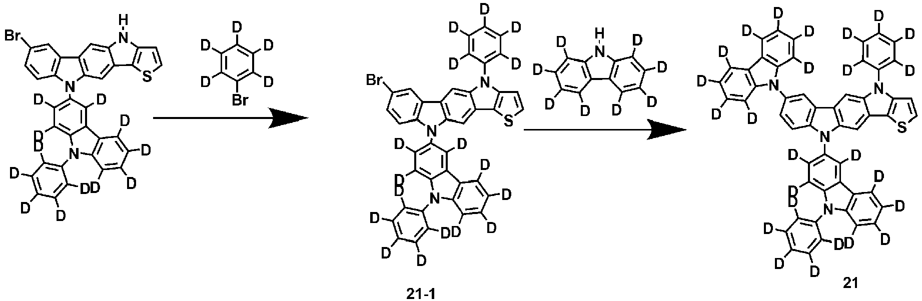

### 1) Synthesis of Intermediate 21-1

Pd(OAc)₂ (0.1 eq), [(t-Bu)₃PH]BF₄ (0.15 eq), K₂CO₃ (5 eq), and toluene (based on 0.1 M 1 eq reagent) were added to a flask containing 7-bromo-10-(9-(phenyl-d5)-9H-carbazol-3-yl-1,2,4,5,6,7,8-d7)-4,10-dihydrothieno[2',3':4,5]pyrrolo[3,2-b]carbazole (1 eq) and 1-bromobenzene-2,3,4,5,6-d5 (1.2 eq), and the mixture was stirred under reflux for 27 hours. The reaction mixture was cooled to room temperature, an extraction process was performed thereon by using MC, and the extracted material was washed with distilled water. After drying with MgSO₄ and distillation under reduced pressure, the residue was separated through a column to synthesize Intermediate 21-1 (yield of 47.3 %). Intermediate 21-1 was confirmed by LC-MS.

### (C₄₀H₇D₁₇BrN₃S: [M]+675.71)2) Synthesis of Compound 21

Pd(dba)₃ (0.06 eq), (t-Bu)3P (0.09 eq), t-BuONa (4.4 eq), and toluene (based on 0.1 M 1 eq reagent) were added to a flask containing Intermediate 21-1 (1 eq) and 9H-carbazole-1,2,3,4,5,6,7,8-d8 (1.2 eq), and the mixture was stirred under reflux for 30 hours. The reaction mixture was cooled to room temperature, an extraction process was performed thereon by using MC, and the extracted material was washed with distilled water. After drying with MgSO₄ and distillation under reduced pressure, the residue was separated through a column to obtain Compound 21 (yield of 69.5 %).

Compound 21 was confirmed by LC-MS.

**[Table 1]**

| Compound No. | ¹H-NMR(CDCl₃) | MS/FAB | |
|---|---|---|---|
| | | found | calc. |
| 15 | 7.12(d, 1H), 7.30(d, 1H), 7.40(s, 1H), 7.4-7.75 (m, 8H), 8.09(d, 1H) | 591.79 | 592.80 |
| 16 | 7.12(d, 1H), 7.30(d, 1H), 7.40(s, 1H), 7.4-7.75 (m, 3H), 8.09(d, 1H) | 596.77 | 597.83 |
| 19 | 7.12(d, 1H), 7.30(d, 1H), 7.40(s, 1H), 7.40-7.50 (m, 17H), 7.59-7.70(m, 4H), 7.74(d,1H) 8.07(d, 1H) | 850.22 | 851.20 |
| 20 | 7.12(d, 1H), 7.20(t, 1H), 7.31(d, 1H), 7.40-7.50 (m, 13H), 8.06-8.08(m, 2H), 8.19(d,1H) | 757.09 | 758.00 |
| 21 | 7.12(d, 1H), 7.31(d, 1H), 7.41(s,1H), 7.56(s, 1H), 7.67-7.74 (m, 2H), 8.08(d, 1H), | 769.01 | 770.07 |

### Evaluation Example 1: Identifying properties of compounds

The HOMO energy level, LUMO energy level, and T₁ energy level of the compounds synthesized by Synthesis Examples 1 to 5 were measured. The results thereof are shown in Table 3.

The HOMO, LUMO, and T₁ energy levels were evaluated according to the method shown in Table 2 below.

**[Table 2]**

| | |
|---|---|
| HOMO energy level evaluation method | By using cyclic voltammetry (CV) (electrolyte: 0.1 M Bu₄NPF₆ / solvent: dimethylforamide (DMF) / electrode: 3-electrode system (working electrode: GC, reference electrode: Ag/AgCl, and auxiliary electrode: Pt)), the potential (V)-current (A) graph of each compound was obtained, and from the oxidation onset of the graph, the HOMO energy level of each compound was calculated. |
| LUMO energy level evaluation | By using cyclic voltammetry (CV) (electrolyte: 0.1 M Bu₄NPF₆ / solvent: dimethylforamide (DMF) / electrode: 3-electrode system (working electrode: |
| method | GC, reference electrode: Ag/AgCl, and auxiliary electrode: Pt)), the potential (V)-current (A) graph of each compound was obtained, and from the reduction onset of the graph, the LUMO energy level of each compound was calculated. |
| T₁ energy level evaluation method | "Triplet energy at onset wavelength" refers to the triplet energy at the beginning of the low-temperature photoluminescence spectroscopy (PL) spectrum, and is calculated from the triplet energy at the point (i.e., the x-intercept) where the PL spectrum intersects the wavelength axis of the function obtained by plotting the PL spectrum as a quadratic function. In this regard, low-temperature PL spectra were obtained by dissolving each compound in tetrahydrofuran (THF) at a concentration of 1 X 10⁻⁵ M and measuring at low temperature (77 K). Triplet energy levels were derived from the analysis of only the peaks observed only at low temperatures compared to the room temperature PL spectrum. |

**[Table 3]**

| | HOMO (eV) | LUMO (eV) | T₁ (eV)_onset |
|---|---|---|---|
| Compound 15 | -5.55 | -1.89 | 2.96 |
| Compound 16 | -5.57 | -1.86 | 3.01 |
| Compound 19 | -5.54 | -1.83 | 2.98 |
| Compound 20 | -5.55 | -1.87 | 2.94 |
| Compound 21 | -5.53 | -1.82 | 2.99 |

### [Examples]

### [Example 1]

An ITO glass substrate was cut to a size of 50 mm x 50 mm x 0.5 mm, ultrasonically cleaned with isopropyl alcohol and pure water each for 10 minutes, and cleaned by irradiation of ultraviolet rays and exposure to ozone for 10 minutes. The ITO glass substrate was loaded onto a vacuum deposition apparatus. m-MTDATA was vacuum-deposited on the ITO glass substrate to form a hole injection layer having a thickness of 40 Å, and NPB, which is a hole transport material, was vacuum-deposited on the hole injection layer to form a hole transport layer having a thickness of 10 Å. Compound 15 and Compound ETH66 (weight ratio of 5:5) as hosts and Compound DF15 (2 wt% doping) as a dopant were co-deposited on the hole transport layer to form an emission layer having a thickness of 300 Å. Compound ET1 was deposited on the emission layer to form an electron transport layer having a thickness of 300 Å, and Al (anode) was vacuum-deposited to form an Al electrode, thereby completing the manufacture of a light-emitting device.

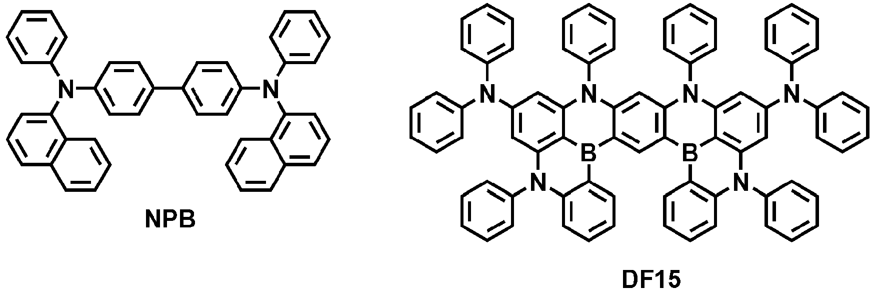

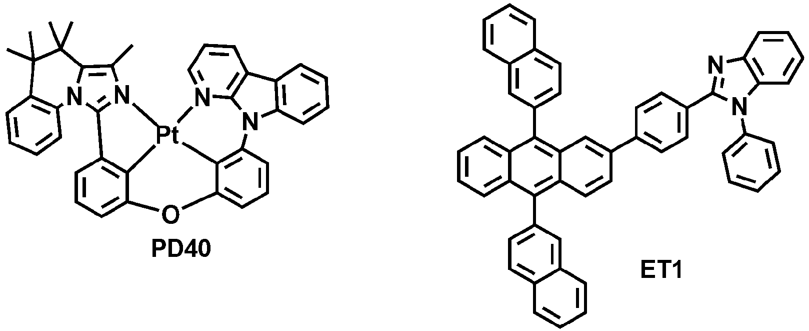

### [Examples 2 to 15 and Comparative Examples 1 to 8]

Light-emitting devices were manufactured in the same manner as in Example 1, except that the compounds listed in Table 4 below were used as the host and the dopant (Compound DF15 (2 wt% doping) or Compound PD40 (15 wt% doping)) when forming the emission layer.

### Evaluation Example 2: Characterization of light-emitting devices

The driving voltage, device efficiency, and device lifespan of the light-emitting devices of Examples 1 to 15 and Comparative Examples 1 to 8 were evaluated. The results thereof are shown in Table 4. In order to evaluate the characteristics of the manufactured light-emitting devices, the driving voltage and efficiency (cd/A) at a current density of 10 mA/cm² were measured using the V7000 OLED IVL test system, (Polaronix). The time from the initial value to 90 % luminance degradation when continuously driven at a current density of 10 mA/cm² was measured for evaluation.

**[Table 4]**

| | Dopant | Hole transporting host | Electron transporting host | Driving voltage (V) | Efficiency (cd/A) | Lifespan (T₉₀, hr) |
|---|---|---|---|---|---|---|
| Example 1 | DF15 | 15 | ETH66 | 4.35 | 23.0 | 60.1 |
| Example 2 | DF15 | 16 | ETH66 | 4.33 | 24.0 | 72.1 |
| Example 3 | DF15 | 19 | ETH66 | 4.47 | 23.1 | 68.2 |
| Example 4 | DF15 | 19 | ETH2 | 4.15 | 25.8 | 73.1 |
| Example 5 | DF15 | 20 | ETH2 | 4.09 | 24.9 | 70.2 |
| Example 6 | DF15 | 16 | ETH2 | 4.12 | 25.7 | 66.1 |
| Example 7 | DF15 | 21 | ETH90 | 4.07 | 23.9 | 62.5 |
| Example 8 | PD40 | 15 | ETH66 | 4.51 | 22.9 | 61.7 |
| Example 9 | PD40 | 16 | ETH66 | 4.45 | 23.5 | 63.4 |
| Example 10 | PD40 | 19 | ETH66 | 4.56 | 25.3 | 75.2 |
| Example 11 | PD40 | 19 | ETH2 | 4.21 | 24.9 | 70.3 |
| Example 12 | PD40 | 20 | ETH2 | 4.15 | 24.3 | 68.1 |
| Example 13 | PD40 | 16 | ETH2 | 4.13 | 25.1 | 72.9 |
| Example 14 | DF15 | 15 | - | - | 19.9 | - |
| Example 15 | PD40 | 15 | - | - | 20.1 | - |
| Comparative Example 1 | DF15 | A | - | 5.56 | 16.5 | 12.0 |
| Comparative | DF15 | - | ETH66 | 5.88 | 12.3 | 12.3 |
| Example 2 | | | | | | |
| Comparative Example 3 | PD40 | A | - | 5.64 | - | 18.1 |
| Comparative Example 4 | PD40 | - | ETH66 | 5.65 | 13.2 | 13.1 |
| Comparative Example 5 | DF15 | B | - | 4.78 | 15.1 | 22.1 |
| Comparative Example 6 | PD40 | B | - | 4.81 | 16.2 | 24.5 |
| Comparative Example 7 | DF15 | B | ETH66 | - | 14.8 | 20.1 |
| Comparative Example 8 | PD40 | B | ETH66 | - | 15.1 | 24.1 |

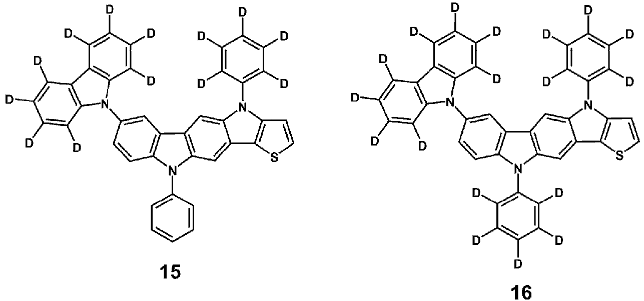

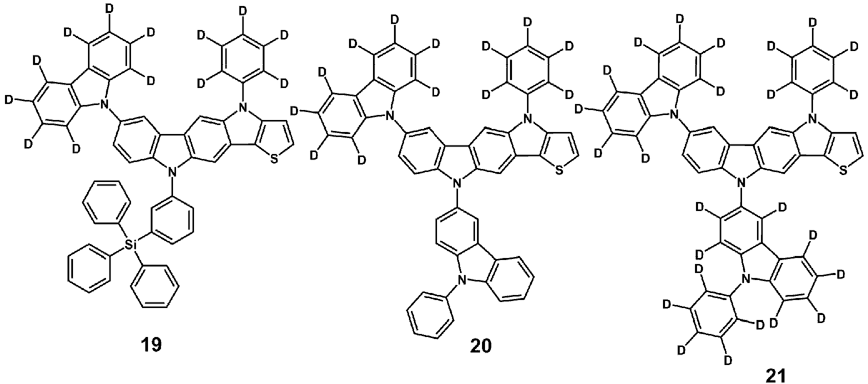

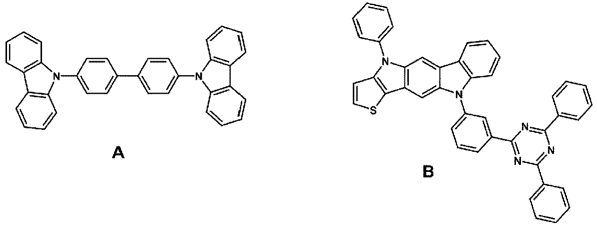

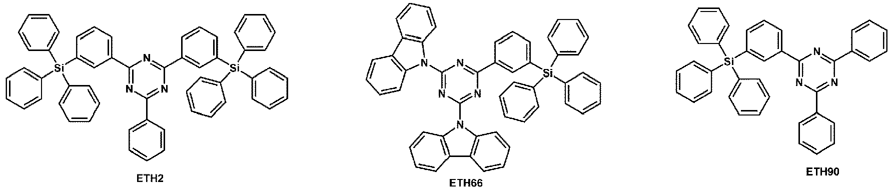

From Table 4, it can be confirmed that the light-emitting devices according to Examples 1 to 15 have lower driving voltage, higher efficiency, longer lifespan, and better color purity characteristics, as compared to the light-emitting devices according to Comparative Examples 1 to 8.

Due to the inclusion of the heterocyclic compound represented by Formula 1, the light-emitting device can have low driving voltage, high efficiency, long lifespan, and excellent color purity characteristics, and high-quality electronic apparatuses and electronic devices can be manufactured by using the same.

Embodiments have been disclosed herein, and although terms are employed, they are used and are to be interpreted in a generic and descriptive sense only and not for the purposes of limitation. In some instances, as would be apparent by one of ordinary skill in the art, features, characteristics, and/or elements described in connection with an embodiment may be used singly or in combination with features, characteristics, and/or elements described in connection with other embodiments unless otherwise specifically indicated. Accordingly, it will be understood by those of ordinary skill in the art that various changes in form and details may be made without departing from the scope of the disclosure as set forth in the claims.

## Claims

1. A light-emitting device (10) comprising:
a first electrode (110);
a second electrode (150) facing the first electrode (110);
an interlayer (130) between the first electrode (110) and the second electrode (150) and including an emission layer; and
a heterocyclic compound represented by Formula 1: 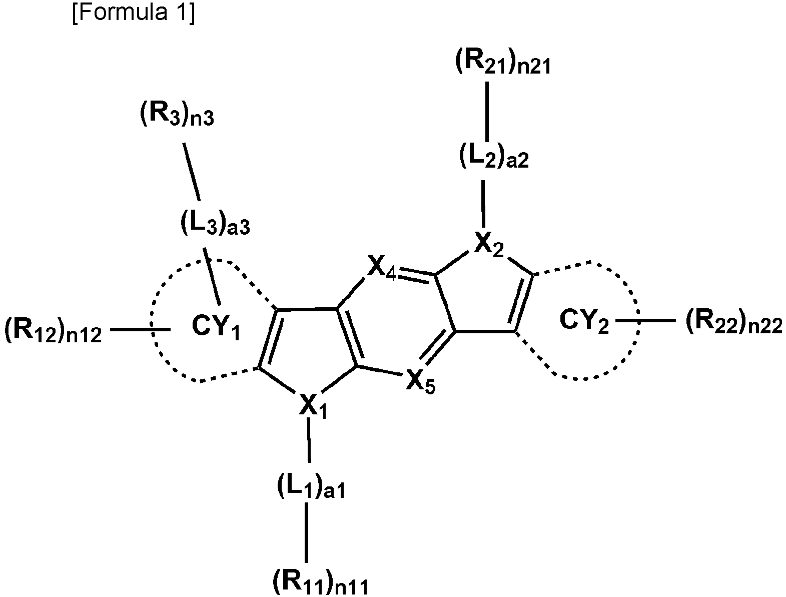 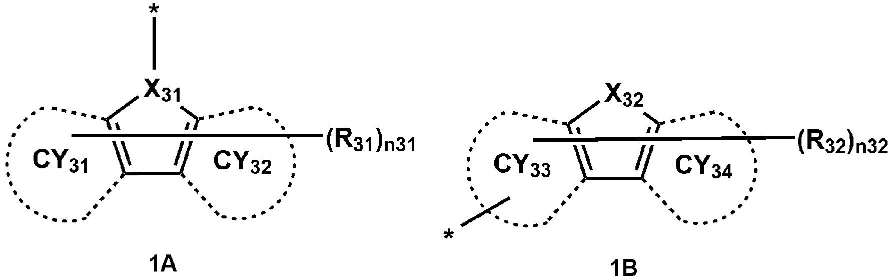 wherein in Formulae 1, 1A, and 1B,
X₁ is C(R₁) or N,
X₂ is C(R₂) or N,
X₃₁ is C(R₃ₐ) or N,
X₃₂ is N(R₃ₐ) or C(R₃ₐ)(R_{3b}),
X₄ is C(R₄) or N,
X₅ is C(R₅) or N,
L₁ to L₃ are each independently a single bond, a C₅-C₆₀ carbocyclic group unsubstituted or substituted with at least one R₁₀ₐ, or a C₁-C₆₀ heterocyclic group unsubstituted or substituted with at least one R₁₀ₐ,
a1 to a3 are each independently an integer from 1 to 3,
ring CY₁, ring CY₂, and ring CY₃₁ to ring CY₃₄ are each independently a C₅-C₆₀ carbocyclic group or a C₁-C₆₀ heterocyclic group,
R₁, R₂, R₃ₐ, R_{3b}, R₄, R₅, R₁₁, R₁₂, R₂₁, R₂₂, R₃, R₃₁, and R₃₂ are each independently a group represented by Formula 1A, a group represented by Formula 1B, hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, a C₆-C₆₀ arylseleno group unsubstituted or substituted with at least one R₁₀ₐ, a C₇-C₆₀ aryl alkyl group unsubstituted or substituted with at least one R₁₀ₐ, a C₂-C₆₀ heteroaryl alkyl group unsubstituted or substituted with at least one R₁₀ₐ, - Si(Q₁)(Q₂)(Q₃), -Ge(Q₁)(Q₂)(Q₃), -N(Q₁)(Q₂), -B(Q₁)(Q₂), -C(=O)(Q₁), -S(=O)₂(Q₁), or - P(=O)(Q₁)(Q₂),
at least one of R₃ in the number of n3 is a group represented by Formula 1A or Formula 1B,
n11 is an integer from 0 to 20,
n12 is an integer from 0 to 30,
n21 is an integer from 0 to 20,
n22 is an integer from 0 to 30,
n3 is an integer from 0 to 30,
n31 is an integer from 0 to 20,
n32 is an integer from 0 to 20,
* indicates a binding site to a neighboring atom,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or a combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or a combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, -F, a cyano group, a C₁-C₆₀ alkyl group, a C₁-C₆₀ alkoxy group, a phenyl group, a biphenyl group, a C₃-C₆₀ carbocyclic group, a C₁-C₆₀ heterocyclic group, or a combination thereof.

2. The light-emitting device (10) of claim 1, wherein:
(i) the emission layer comprises:
the heterocyclic compound; and
a third compound that includes a transition metal, a fourth compound that includes a cyclic group including boron (B) and nitrogen (N) as ring-forming atoms, or a combination thereof, and
the heterocyclic compound, the third compound, and the fourth compound are different from each other; or
(ii) the emission layer comprises:
the heterocyclic compound; and
a second compound that includes at least one π electron-deficient nitrogen-containing C₁-C₆₀ heterocyclic group, and
the second compound is different from the heterocyclic compound.

3. The light-emitting device (10) of claim 1 or claim 2, wherein
the emission layer comprises a host and a dopant, and
the host includes the heterocyclic compound.

4. The light-emitting device (10) of any one of claims 1 to 3, wherein the emission layer emits blue light.

5. An electronic apparatus comprising the light-emitting device (10) of any one of claims 1 to 4.

6. The electronic apparatus of claim 5, further comprising:
a thin-film transistor, wherein
the thin-film transistor includes a source electrode and a drain electrode, and
the first electrode of the light-emitting device is electrically connected to at least one of the source electrode and the drain electrode, optionally further comprising:
a color filter, a color conversion layer, a touch screen layer, a polarizing layer, or a combination thereof, optionally wherein the color conversion layer includes quantum dots.

7. An electronic device (1) comprising the light-emitting device (10) of any one of claims 1 to 5.

8. A heterocyclic compound represented by Formula 1: 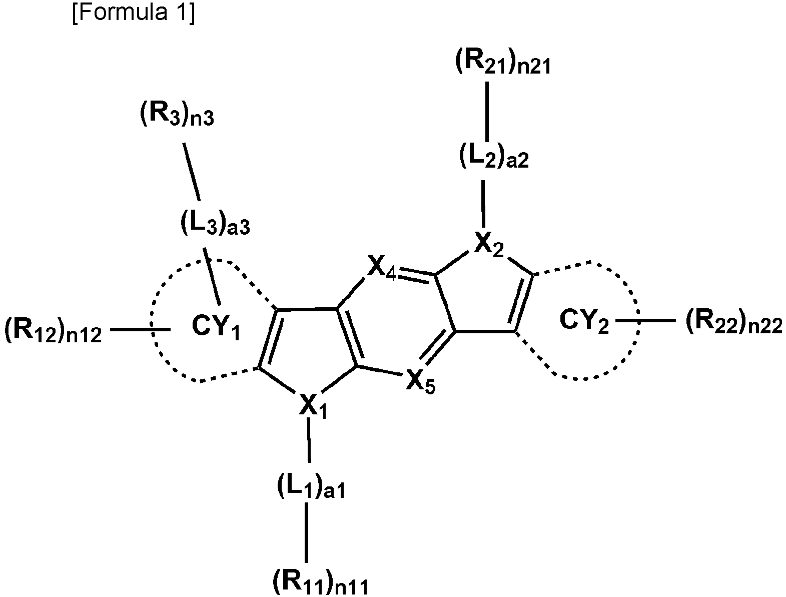 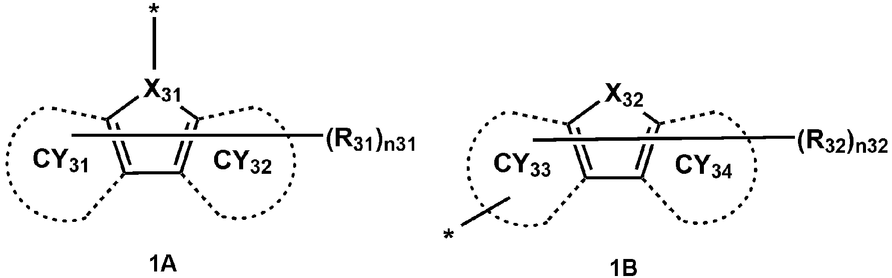 wherein in Formulae 1, 1A, and 1B,
X₁ is C(R₁) or N,
X₂ is C(R₂) or N,
X₃₁ is C(R₃ₐ) or N,
X₃₂ is N(R₃ₐ) or C(R₃ₐ)(R_{3b}),
X₄ is C(R₄) or N,
X₅ is C(R₅) or N,
L₁ to L₃ are each independently a single bond, a C₅-C₆₀ carbocyclic group unsubstituted or substituted with at least one R₁₀ₐ, or a C₁-C₆₀ heterocyclic group unsubstituted or substituted with at least one R₁₀ₐ,
a1 to a3 are each independently an integer from 1 to 3,
ring CY₁, ring CY₂, and ring CY₃₁ to ring CY₃₄ are each independently a C₅-C₆₀ carbocyclic group or a C₁-C₆₀ heterocyclic group,
R₁, R₂, R₃ₐ, R_{3b}, R₄, R₅, R₁₁, R₁₂, R₂₁, R₂₂, R₃, R₃₁, and R₃₂ are each independently a group represented by Formula 1A, a group represented by Formula 1B, hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, a C₆-C₆₀ arylseleno group unsubstituted or substituted with at least one R₁₀ₐ, a C₇-C₆₀ aryl alkyl group unsubstituted or substituted with at least one R₁₀ₐ, a C₂-C₆₀ heteroaryl alkyl group unsubstituted or substituted with at least one R₁₀ₐ, - Si(Q₁)(Q₂)(Q₃), -Ge(Q₁)(Q₂)(Q₃), -N(Q₁)(Q₂), -B(Q₁)(Q₂), -C(=O)(Q₁), -S(=O)₂(Q₁), or - P(=O)(Q₁)(Q₂),
at least one of R₃ in the number of n3 is a group represented by Formula 1A or Formula 1B,
n11 is an integer from 0 to 20,
n12 is an integer from 0 to 30,
n21 is an integer from 0 to 20,
n22 is an integer from 0 to 30,
n3 is an integer from 0 to 30,
n31 is an integer from 0 to 20,
n32 is an integer from 0 to 20,
* indicates a binding site to a neighboring atom,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or a combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or a combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, -F, a cyano group, a C₁-C₆₀ alkyl group, a C₁-C₆₀ alkoxy group, a phenyl group, a biphenyl group, a C₃-C₆₀ carbocyclic group, a C₁-C₆₀ heterocyclic group, or a combination thereof.

9. The heterocyclic compound of claim 8, wherein:
(i) ring CY₁, ring CY₂, and ring CY₃₁ to ring CY₃₄ are each independently a benzene group, a naphthalene group, an anthracene group, a phenanthrene group, a triphenylene group, a pyrene group, a chrysene group, a cyclopentadiene group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an azafluorene group, an azacarbazole group, an azadibenzofuran group, an azadibenzothiophene group, an azadibenzosilole group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a dibenzoxasiline group, a dibenzothiasiline group, a dibenzodihydroazasiline group, a dibenzodihydrodisiline group, a dibenzodihydrosiline group, a dibenzodioxine group, a dibenzoxathiine group, a dibenzoxazine group, a dibenzopyran group, a dibenzodithiine group, a dibenzothiazine group, a dibenzothiopyran group, a dibenzocyclohexadiene group, a dibenzodihydropyridine group, or a dibenzodihydropyrazine group; and/or
(ii) ring CY₁, and ring CY₃₁ to ring CY₃₄ are each a benzene group, and ring CY₂ is a furan group or a thiophene group.

10. The heterocyclic compound of claim 8 or claim 9, wherein:
(i) L₁ to L₃ are each independently:
a single bond; or
a benzene group, a naphthalene group, a phenanthrene group, a fluorene group, a carbazole group, a dibenzofuran group, a dibenzothiophene group, an azacarbazole group, an azadibenzofuran group, or an azadibenzothiophene group, each unsubstituted or substituted with at least one R₁₀ₐ; or
(ii) L₁ to L₃ are each independently a single bond, or a group represented by one of Formula 2-1 to Formula 2-5: 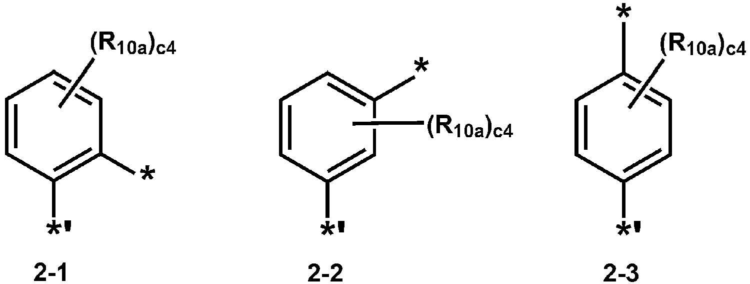 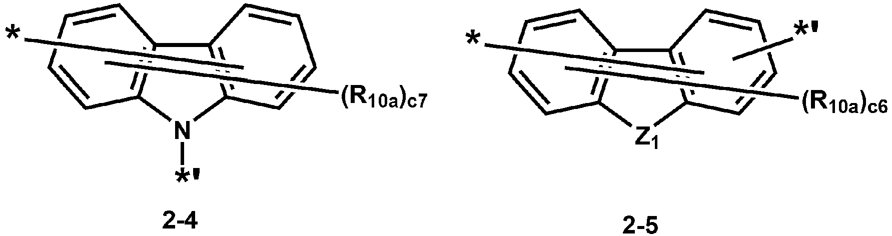 wherein in Formulae 2-1 to 2-5,
Z₁ is O, S, N(R₁'), or C(R₁')(R₂'),
R₁' and R₂' are each independently the same as described in connection with R₁ in Formula 1,
R₁₀ₐ is the same as described in Formula 1,
c4 is an integer from 0 to 4,
c6 is an integer from 0 to 6,
c7 is an integer from 0 to 7, and
* and *' each indicate a binding site to a neighboring atom.

11. The heterocyclic compound of any one of claims 8 to 10, wherein R₁, R₂, R₃ₐ, R_{3b}, R₄, R₅, R₁₁, R₁₂, R₂₁, R₂₂, R₃, R₃₁, and R₃₂ are each independently:
a group represented by Formula 1A, a group represented by Formula 1B, hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a phenyl group, a biphenyl group, or a terphenyl group;
a C₁-C₆₀ alkyl group, a C₃-C₆₀ cycloalkyl group, a C₁-C₆₀ heterocycloalkyl group, a phenyl group, a biphenyl group, a terphenyl group, a naphthyl group, an anthracenyl group, a phenanthrenyl group, a phenalenyl group, a triphenylenyl group, a pyrenyl group, a chrysenyl group, a pyrrolyl group, a furanyl group, a thiophenyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a phenanthrolinyl group, a benzoimidazolyl group, a benzofuranyl group, a dibenzofuranyl group, a benzothiophenyl group, a dibenzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a carbazolyl group, a benzocarbazolyl group, a fluorenyl group, a benzoisothiazolyl group, a benzoxazolyl group, a benzoisoxazolyl group, a phenoxazinyl group, an acridinyl group, or a xanthenyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a phenyl group, a biphenyl group, a terphenyl group, or a combination thereof; or
-Si(Q₁)(Q₂)(Q₃), -Ge(Q₁)(Q₂)(Q₃), or -N(Q₁)(Q₂).

12. The heterocyclic compound of any one of claims 8 to 11, wherein the heterocyclic compound comprises:
i) at least one carbazole group; or
ii) at least one deuterium; or
iii) at least one -Si(Q₁)(Q₂)(Q₃); or
iv) at least one cyano group; or
v) a combination selected from i) to iv).

13. The heterocyclic compound of any one of claims 8 to 12, wherein
the group represented by Formula 1A is represented by one of Formulae 1A-1 to 1A-6, and
the group represented by Formula 1B is represented by one of Formulae 1B-1 to 1B-12: 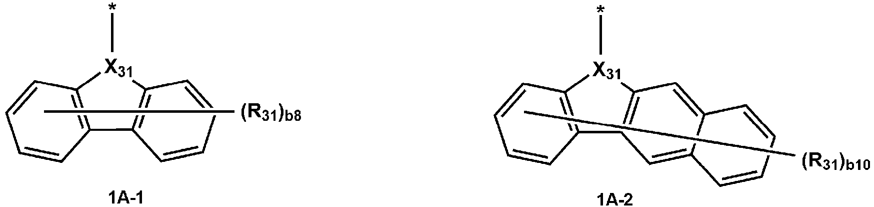 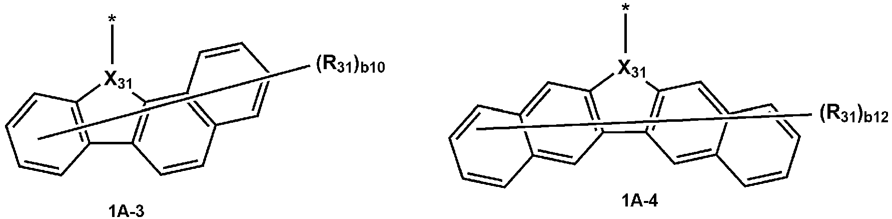 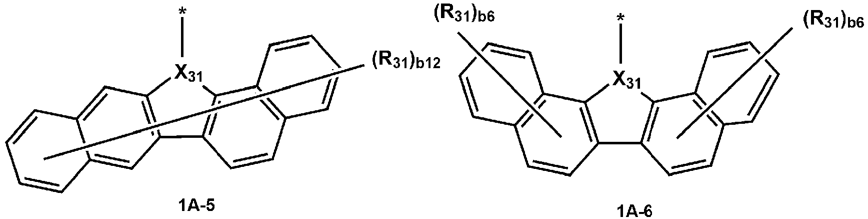 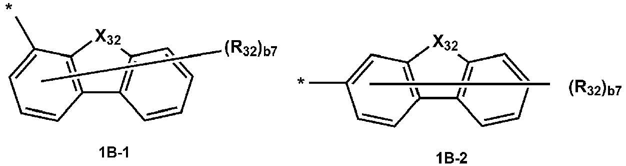 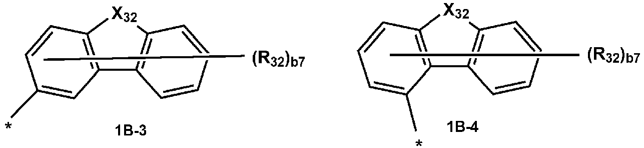 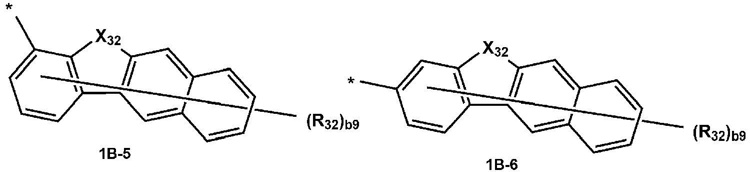 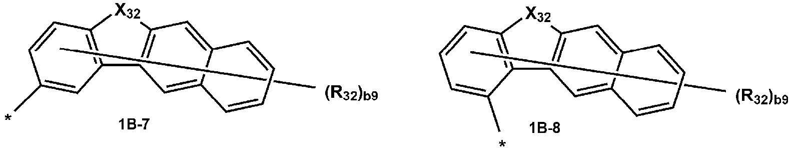 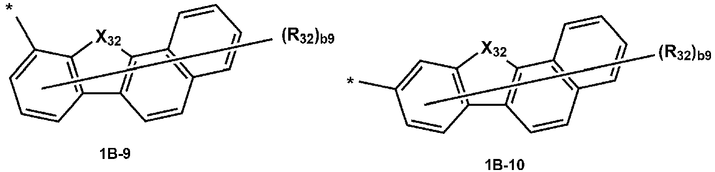 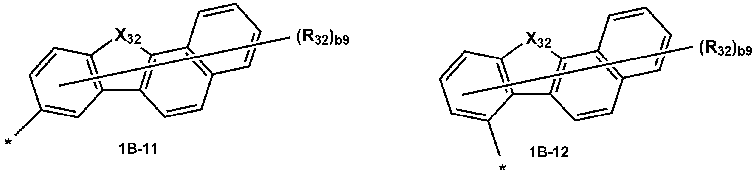 wherein in Formulae 1A-1 to 1A-6 and 1B-1 to 1B-12,
X₃₁, X₃₂, R₃₁, and R₃₂ are the same as described in Formulae 1A and 1B,
b6 is an integer from 0 to 6,
b7 is an integer from 0 to 7,
b8 is an integer from 0 to 8,
b9 is an integer from 0 to 9,
b10 is an integer from 0 to 10,
b12 is an integer from 0 to 12, and
* indicates a binding site to a neighboring atom.

14. The heterocyclic compound of any one of claims 8 to 13, wherein the heterocyclic compound is represented by one of Formula 1-1 to Formula 1-4: 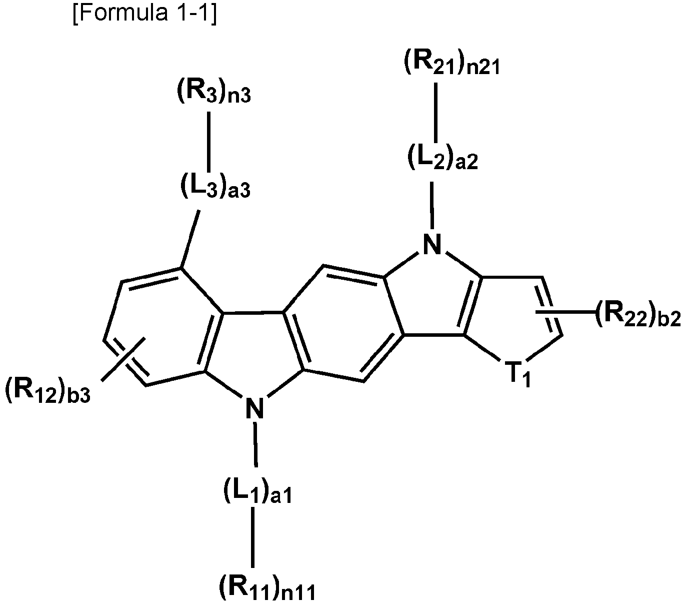 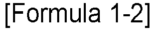 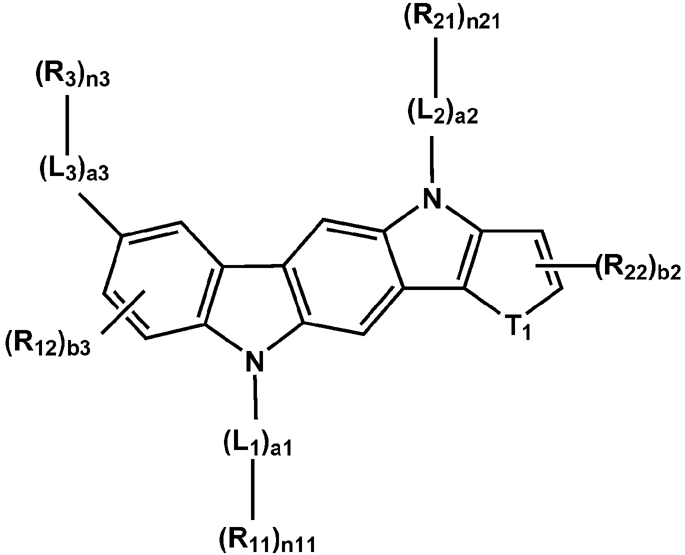 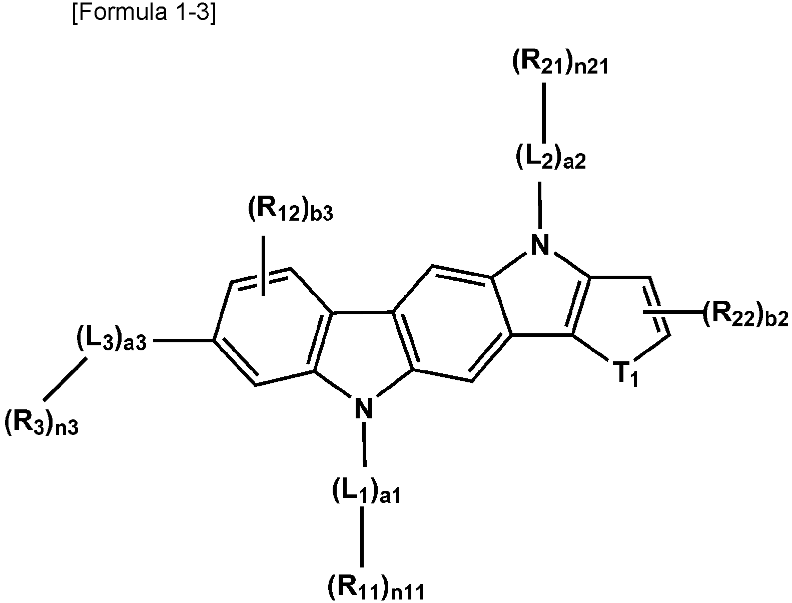 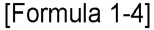 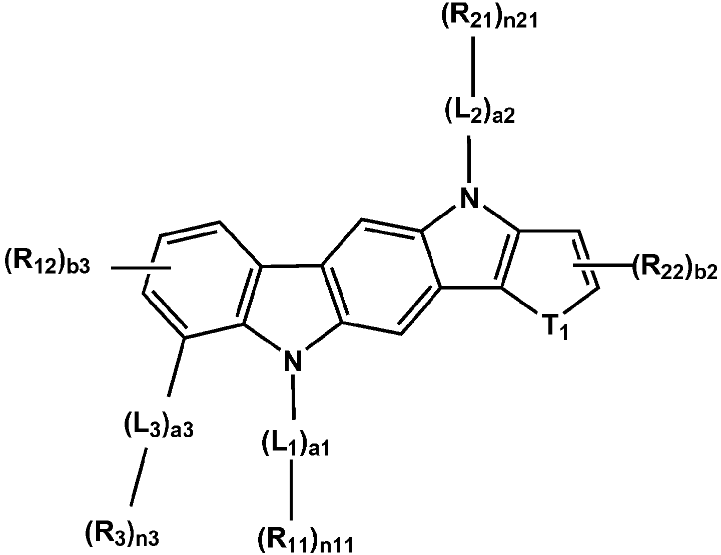 wherein in Formulae 1-1 to 1-4,
L₁ to L₃, a1 to a3, R₁₁, R₁₂, R₂₁, R₂₂, R₃, n11, n21, and n3 are the same as described in Formula 1,
T₁ is O or S,
b2 is an integer from 0 to 2, and
b3 is an integer from 0 to 3.

15. The heterocyclic compound of claim 8, wherein the heterocyclic compound is one of Compounds 1 to 21:
